# EUROPEAN PATENT SPECIFICATION

(11) **EP 3 568 411 B1**
(45) Date of publication and mention of the grant of the patent: **06.03.2024**
(21) Application number: 18704108.2
(22) Date of filing: 12.01.2018
(51) Int. Cl.: C07K 14/575

(54) **METHODS AND COMPOSITIONS FOR TREATING HPA HYPERACTIVITY**
VERFAHREN UND ZUSAMMENSETZUNGEN ZUR BEHANDLUNG VON HPA-HYPERAKTIVITÄT
PROCÉDÉS ET COMPOSITIONS POUR LE TRAITEMENT DE L'HYPERACTIVITÉ HPA

(30) Priority: 13.01.2017 US 201762446294 P
(43) Date of publication of application: 20.11.2019
(73) Proprietor: Sanna, Pietro P., San Diego, California 92106-1614 (US); Lloyd, Linda S., San Diego, California 92106-1614 (US)
(72) Inventor: Sanna, Pietro P., San Diego, California 92106-1614 (US); Lloyd, Linda S., San Diego, California 92106-1614 (US)
(74) Representative: FRKelly
(86) International application number: PCT/US2018/013665
(87) International publication number: WO 2018/132768

(56) References cited:
- WO-A1-92/13074
- WO-A2-2006/086402
- ZOBEL A W ET AL: "EFFECTS OF THE HIGH-AFFINITY CORTICOTROPIN-RELEASING HORMONE RECEPTOR 1 ANTAGONIST R121919 IN MAJOR DEPRESSION: THE FIRST 20 PATIENTS TREATED", JOURNAL OF PSYCHIATRIC RESEARCH, ELSEVIER LTD, GB, vol. 34, no. 3, 1 May 2000 (2000-05-01), pages 171-181, XP001104029, ISSN: 0022-3956, DOI: 10.1016/S0022-3956(00)00016-9

## Description

### BACKGROUND OF THE INVENTION

### 1. Field of the Invention.

This invention relates to treatment methods and compositions for ameliorating hypothalamic pituitary adrenal (HPA) axis hyperactivity. More specifically, this invention relates to engineered corticotropin-releasing factor (CRF) binding agents, and pharmaceutical compositions comprising same, said binding agents and compositions being useful for reducing HPA axis hyperactivity by reducing corticotropin releasing factor (CRF) excess release and peak bursts.

### 2. Related Art.

The following description in this Background section includes information that may be useful in understanding the present invention. It is not an admission that any such information is prior art, or relevant, to the presently claimed inventions, or that any publication specifically or implicitly referenced is prior art. In this specification, a number of documents including patent applications are cited. The disclosures of these documents, while not considered relevant for the patentability of this invention, are referred to herein. In this Background section, and throughout this Description, parenthetical citations to reference documents, numbered 1-167, refer to the numbered documents listed in the reference list immediately after Example 3 herein.

The stress response, though essential for survival, can become dysregulated and result in disease. Here, a novel strategy is described that is aimed at normalizing hypothalamic pituitary adrenal (HPA) axis hyperactivity, which has been implicated in variety of disease states and conditions.

In response to stressors, defined as perceived threats to the physiological or psychological integrity of an organism, corticotropin-releasing factor (CRF), also known as corticotropin-releasing hormone (CRH), is released into the pituitary portal system by parvocellular neuroendocrine neurons of the paraventricular nucleus (PVN) of the hypothalamus (1, 2). CRF elicits the release into systemic circulation of adrenocorticotropin hormone (ACTH) by corticotrope cells in the anterior pituitary (1, 2). In turn, ACTH stimulates glucocorticoid secretion from the adrenal cortex (1, 2). Glucocorticoids exert feedback control on the corticotropes of the pituitary, hypothalamic, and suprahypothalamic levels (1, 2). CRF binding protein (CRF-BP) in plasma contributes to removing CRF from the general circulation (3). Glucocorticoids are remarkably pleiotropic in their effects (4), and HPA axis dysregulation has detrimental effects on almost every organ system (5, 6, 7, 8, 9, 10, 11, 12).

Hyperactivity of the HPA axis predisposes subjects to, and is a component of, a variety of illnesses, including anxiety, depression, Alzheimer's and Parkinson's diseases, obesity, metabolic syndrome, osteoporosis, cardiovascular disease, alcohol and drug abuse, inflammatory bowel disease (IBD) and irritable bowel syndrome (IBS), among others. HPA hyperactivity is characterized by higher production of CRF and glucocorticoids. Prolonged exposure to elevated glucocorticoids has been proposed to deleteriously act on the central nervous system, causing hippocampal and prefrontal cortex functional impairments. Reduced hippocampal inhibition of the hypothalamus further promotes HPA axis hyperactivity. Normalization of HPA hyperactivity is beneficial for the management of multiple conditions characterized by increased HPA activation, including anxiety and depression, Alzheimer's and Parkinson's diseases, obesity, metabolic syndrome, osteoporosis, cardiovascular disease, alcohol and drug abuse, inflammatory bowel disease and irritable bowel syndrome, among others.

Two CRF receptors are known: CRF1 and CRF2. CRF1 is activated by CRF as well as by the related peptide urocortin 1 (Ucn1), which has a threefold higher binding affinity (13) than CRF. CRF1 is the receptor responsible for ACTH release in the pituitary (13, 14), as well as the emerging pro-inflammatory actions of CRF and Ucn1 (15, 16, 17). Two other related peptides, Ucn2 and Ucn3, are selective CRF2 agonists and have much higher binding affinity to CRF2 than CRF or Ucn1 (18). Importantly, neither Ucn2 nor Ucn3 exhibits appreciable affinity for CRF-BP (13). Thus, the inventive strategy described herein to increase CRF-BP binding capacity in plasma will reduce activation of CRF1 while switching the balance of CRF1/CRF2 receptors toward activation of CRF2. This will be beneficial since CRF2 has generally opposite actions as CRF1 and thus may be beneficial in the periphery, for instance, in the cardiovascular system (18). In fact, CRF2-mediated beneficial actions on the cardiovascular system include vasodilation, increases in cardiac output, myocardial contractility, coronary blood flow, and cardioprotection in ischemialreperfusion (18, 19, 20, 21).

In addition to the hypothalamic CRF-expressing neurons in the PVN that control the HPA, extrahypothalamic CRF-expressing neurons are present in the extended amygdala, and in particular in the central nucleus of the amygdala (CeA) and bed nucleus of the stria terminalis (BNST), the neocortex, medial septum, thalamus, cerebellum, and autonomic midbrain and hindbrain nuclei, including the ventral tegmental area (VTA) (22, 23). Evidence indicates that the peripheral (HPA) and central (extrahypothalamic CRF) stress systems exert reciprocal regulations, which are prone to feed-forward potentiation of their activation states (23, 24, 25, 26, 27, 28, 29, 30). Elevated glucocorticoids provide negative feedback to the PVN, reducing CRF production in the PVN neurons that regulate the HPA. Conversely, chronically elevated glucocorticoids increase CRF production in PVN neurons with descending projections, magnocellular neurosecretory neurons, and extended amygdala neurons (23, 25, 26, 27). Increased CRF in the extended amygdala is believed to be key to pathologic fear and anxiety and to clinical syndromes such as melancholic depression, posttraumatic stress disorder (PTSD), and drug and alcohol abuse (31, 32, 33). Increased CRF production in hypothalamic and extrahypothalamic neurons in depression and dysthymia patients results in hyperactivity of the HPA and elevated cortisol plasma concentrations (34, 35, 36, 37). Increased CRF production is implicated in symptoms of depression and aging such as sleep and appetite disturbances, and reduced libido (34). HPA hyperactivity is a marker of depression that normalizes following successful antidepressant treatment (34). Importantly, prolonged exposure to excessive glucocorticoid levels induce prefrontal cortex and hippocampal functional impairment accompanied by dendritic alterations (4, 38, 39, 40, 41, 42, 43, 44, 45, 46). In turn, hippocampal damage can result in reduced hippocampus-mediated inhibition of the HPA axis, sustaining HPA hyperactivation and leading to further central dysfunction (28, 29).

Depression with melancholic and psychotic depressive features as well as borderline personality disorder (BPD) are characterized by enhanced cortisol release and reduced feedback sensitivity of the HPA axis (47, 48, 49), which is interpreted as due to exaggerated CRF drive (50) and/or reduced glucocorticoid receptor function (51). Cortisol findings in PTSD suggest reduced rather than enhanced basal cortisol concentrations (52). However, the evidence is complex and may be confounded by co-morbid major depressive disorder (53). Interestingly, cortisol impairs memory retrieval in normal and depressed patients, but enhances memory - in particular autobiographical memory - in PTSD and BPD patients (48). The glucocorticoid synthesis inhibitor metyrapone showed promise as adjunctive therapy to serotonergic antidepressants (54). The first CRF1 antagonist evaluated in humans, R121919, showed promise in depression (55) with a reduction in depression and anxiety scores allegedly being observed using both patient and clinician ratings; however, development was terminated due to safety issues. Other CRF1 antagonists were not effective in clinical trials of stress-related psychiatric disorders (56, 57), possibly due to shorter dissociation half-times of the latter compounds (58, 59, 60, 61). It is worth noting that current CRF1 antagonists at their therapeutic doses do not reduce peripheral glucocorticoids and ACTH levels (62, 63). The glucocorticoid receptor (GR) blocker mifepristone (RU486) also showed promise in patients suffering from psychotic major depression (64). Cushing's disease is typically due to pituitary tumors producing excessive ACTH (65). However, the instant invention will have applications in the medical treatment of Cushing's syndrome due to hypothalamic or ectopic CRF production (65). Excessive glucocorticoid levels have also been implicated in the reinforcing actions of alcohol and drugs of abuse (66, 67, 68, 69, 70) and recently, mifepristone proved beneficial in alcoholdependent humans (68).

Most conditions characterized by chronic hypercortisolemia are associated with cognitive deficits and, in particular, memory impairments (48, 71, 72). Elevated cortisol levels are seen in Alzheimer's (AD), Parkinson's (PD) and Huntington's (HD) diseases, suggesting a potentially general role for HPA axis hyperactivity in neurodegeneration (24, 73, 74, 75, 76). In particular, risk of Alzheimer's disease is increased by mutations that increase cortisol production (77) and decreases by GR variants conferring glucocorticoid resistance (78). Both clinical evidence (79) and studies with transgenic animal models of AD (80, 81, 82, 83, 84) indicate that HPA dysregulation is likely a key factor in AD progression and cognitive decline. ApoE^{-/-} mice, a model for the human ApoE-4 genotoype (85), have increased circadian and stress-induced corticosterone secretion (86, 87), agedependent cognitive impairments (87), and neuropathological changes in the cortex and hippocampus secondary to HPA axis hyperactivity (86). Both hypothalamic and extrahypothalamic CRF have been implicated in Alzheimer's disease progression (76, 88, 89). Since elevated peripheral glucocorticoids promote neurodegeneration (4, 28, 29, 38, 39, 40, 41, 42, 43, 44, 45, 46) and increase extrahypothalamic CRF production (23, 25, 26, 27), normalization of HPA function will benefit AD patients. CRF1 antagonism has been proposed for the therapy of Alzheimer's disease (88, 89). PTSD also increases the likelihood of suffering from dementia (90, 91). HPA axis regulation is increasingly recognized as a potential therapeutic target for stress-induced obesity, metabolic syndrome and type II diabetes (5, 7, 8). In experimental animals, adrenalectomy reduces food intake and body weight in a glucocorticoid-reversible manner (5) and elevated glucocorticoids may promote palatable food intake (92). Additionally, many of the genetic rodent obesities are accompanied by chronically elevated glucocorticoid concentrations (93) and adrenalectomy without glucocorticoid replacement, blocks both genetically-induced and neuropeptide Y (NPY)-induced obesities (5). Mifepristone was recently approved by the Food and Drug Administration (FDA) for use in patients with Cushing's syndrome with associated diabetes or glucose intolerance (94). Abnormal levels of glucocorticoids negatively impact the cardiovascular system (95, 96) and altered cardiovascular homeostasis and atherosclerosis are seen in mice with mutations affecting the stress responses (21, 97). Modulation of glucocorticoid release also showed benefits in osteoporosis associated with depression and HPA hyperactivity (9, 10, 11). CRF signaling promotes inflammatory and immune responses inducing inflammatory cytokines, such as TNF-α, IL-1, and IL-6, and macrophage activation, *etc.* (15). Expression in the gastrointestinal tract of CRF and Ucn1, which is also bound by CRF-BP at high affinity, has been implicated in the pathogenesis of inflammatory bowel disease (IBD) (15, 16, 17, 98, 99, 100). CRF and Ucn1 also stimulate colonic motility, an effect reversed by CRF antagonists that poorly penetrate the blood brain barrier, suggesting a potential therapy for irritable bowel syndrome (IBS) (101, 102).

Chronic sustained levels of glucocorticoids reduce CRF in the hypothalamus but increase CRF expression in the extended amygdala, which contributes to perpetuating anxiety and depression (31). Prolonged exposure to excessive glucocorticoid levels also induces prefrontal cortex and hippocampal functional and structural impairments (4, 38, 39, 40, 41, 42, 43, 44, 45, 46), which may contribute to cognitive impairment in neurodegenerative conditions and reduce hippocampus-mediated HPA axis inhibition, leading to further HPA activation and CNS dysfunction (28, 29).

As indicated above, HPA hyperactivity and the consequent excessive levels of circulating glucocorticoids and pathologically large bursts of cortisol secretion have detrimental actions on the central nervous system and peripheral organs (47, 75, 104, 105, 106, 107).

Attempts to use glucocorticoid receptor (GR) antagonists for depression and other conditions characterized by increased activity of the HPA axis have shown some promise (64). However, chronically blocking GR-mediated effects may be counterproductive as, for example, it interferes with glucocorticoid negative feedback leading to increased cortisol levels and mineralocorticoid receptor activation (59, 60, 94). CRF receptor type 1 (CRF1) antagonists have proven somewhat disappointing, possibly because of the pharmacodynamic properties, e.g., fast off-rate, of available compounds (58, 59, 60, 61). Importantly, therapeutic doses of CRF1 antagonists currently being investigated do not appear to reduce peripheral glucocorticoids and adrenocorticotropic hormone (ACTH) levels (62, 63). These considerations indicate that new approaches are needed to modulate the HPA axis.

CRF receptor type 1 (CRF1) antagonists have also been extensively explored, but so far have proven disappointing, possibly because of the pharmacodynamic properties of available compounds. Therefore, the identification of novel therapeutics that normalize hyperactivity of the HPA axis (i.e., return HPA axis activity to a "normal", i.e., non-disease associated level) represents an area of significant unmet medical need.

Effective HPA axis modulation remains an unmet medical need. The present invention addresses this need.

### SUMMARY OF THE INVENTION

In one aspect the present invention provides an engineered corticotropin-releasing factor (CRF) binding agent as set out in the claims. In another aspect the present invention provides a pharmaceutical composition comprising an engineered CRF binding agent, as set out in the claims. In a further aspect the present invention provides an engineered nucleic acid molecule as set out in the claims, and in a still further aspect of the invention a recombinant host cell comprising the engineered nucleic acid molecule as set out in the claims is provided. Some preferred embodiments are described in the dependent claims.

This invention concerns normalizing HPA axis hyperactivity by reducing CRF peak bursts. A very high affinity CRF binding protein (CRF-BP) is present in plasma and contributes to removing CRF from the general circulation (3). However, due to its low plasma concentration, CRF-BP does not prevent CRF bursts from activating the pituitary (113). The present invention provides first-in-class therapeutics based on a new therapeutic concept: increasing CRFBP binding capacity as a new approach to counter HPA axis hyperactivity and elevated circulating glucocorticoid levels and their detrimental effects by providing for more natural modulation of HPA hyperactivity through the reduction of pathologically large bursts of cortisol secretion. Administration of CRF-BP in amounts sufficient to neutralize excess CRF activation mediated by CRF peak bursts can address such pituitary activation.

Thus, in one aspect the disclosure relates to an engineered corticotropin-releasing factor (CRF) antagonist agent, comprising a polypeptide antagonist having CRF antagonist activity under physiological conditions, coupled to one or more half-life-extending moieties, or a pharmaceutically acceptable salt of the corticotropin-releasing factor antagonist agent as set out in the claims.

In another aspect, the engineered corticotropin-releasing factor (CRF) antagonist agent of the disclosure involves an engineered corticotropin-releasing factor (CRF) binding agent, as well as pharmaceutically acceptable salts thereof as set out in the claims. Such CRF binding agents can be used to neutralize excess CRF *in vivo.* The CRF binding agents of the invention comprise a polypeptide having CRF-specific binding activity under physiological conditions coupled (or conjugated) to one or more half-life-extending moieties which comprises an Fc forming portion of a mammalian immunoglobulin heavy chain, or an Fc region of an antibody (optionally an Fc region of a human antibody). Other half-life extending moieties include albumin, transferrin, transthyretin, or polyethylene glycol (PEG), as well as one or more glycosylating moieties, e.g., an N-linked glycan or an O-linked glycan, engineered for inclusion in the polypeptide having CRF-specific binding activity by post-translational processing through the insertion, deletion, or substitution of one or more amino acid residues into the primary amino acid sequence of the polypeptide having CRF binding activity to form a site for glycosylation. In embodiments having more than one half-life-extending moiety, each additional half-life extending moiety is preferably independently selected.

In preferred aspects of the disclosure the polypeptide having CRF binding activity is CRF binding protein (CRF-BP), CRF receptor type 1 (CRFR1), CRF receptor type 2 (CRFR2), or a CRF-specific binding fragment, sequence variant, or derivative of CRF-BP, CRFR1, or CRFR2, having CRF-specific binding activity under physiological conditions. In particularly preferred embodiments, the polypeptide having CRF-specific binding activity is a CRF-specific binding fragment or derivative of a mammalian CRF-BP, preferably a human (e.g., UniProtKB Accession No P24387) or murine (e.g., mouse UniProtKB Accession No. Q60571 or rat UniProtKB Accession No. P24388) CRF-BP derivative or fragment selected from the group of hCRF-BP(25-234)(SEQ ID NO: 12), hCRF-BP(25-322)(SEQ ID NO: 13), rCRF-BP(25-234)(SEQ ID NO: 14), and rCRF-BP(25-322)(SEQ ID NO: 15). Smaller CRF-specific binding fragments of any of the forgoing are also included within the invention as long as they exhibit CRF-specific binding activity under physiological conditions.

In preferred embodiments, the polypeptide having CRF-specific binding activity and the half-life-extending moiety(ies) is(are) covalently coupled, optionally via a linker moiety, preferably a peptide (i.e., peptidyl) linker, preferably in the context of a fusion protein. Non-peptidyl linkers can also be employed. Particularly preferred embodiments include CRF binding agents that comprise a hCRF-BP(25-234) polypeptide (SEQ ID NO: 12) or a hCRF-BP(25-322) (SEQ ID NO: 13) polypeptide coupled via a peptidyl linker, or via a non-peptidyl linker, to an Fc forming portion of a human immunoglobulin heavy chain, which CRF binding agents are preferably synthesized as fusion proteins engineered using recombinant techniques. Other particularly preferred CRF binding agent embodiments include those that comprise a first element coupled to a second element, wherein the first element comprises a hCRF-BP(25-234)(SEQ ID NO: 12) polypeptide or a hCRF-BP(25-322)(SEQ ID NO: 13) polypeptide coupled via a peptide linker to an Fc forming portion of a human immunoglobulin heavy chain and the second element comprises a hCRF-BP(25-234)(SEQ ID NO: 12) polypeptide, a hCRF-BP(25-322)(SEQ ID NO: 13), or polypeptide coupled via a peptide (peptidyl) linker, or non-peptidyl linker, to an Fc forming portion of a human immunoglobulin heavy chain.

A related aspect of the invention involves engineered nucleic acid molecules that encode CRF binding agents, or portions thereof, that comprise a fusion protein as set out in the claims. Such engineered nucleic acid molecules typically comprise an expression construct that codes for the expression of a fusion protein that comprises (i) a polypeptide having CRF binding activity and (ii) a protein that binds FcRn (for example, the Fc-forming portion of a mammalian immunoglobulin heavy chain), and may additionally code for an albumin, a transferrin, or a transthyretin. As an alternative, the gene coding for the polypeptide having CRF binding activity may be engineered to include one or more non-naturally occurring sites for glycosylation, e.g., for N- and/or O-linked glycosylation. Other related aspects concern recombinant host cells that comprise an engineered nucleic acid molecule of the invention as set out in the claims.

Other related aspects of the invention concern compositions that comprise a CRF binding agent of the invention and a pharmaceutically acceptable carrier, for human or veterinary use, as well as kits that contain such compositions preferably stored in a suitable container such as a vial or ampule. Such containers may be packaged in suitable packaging material that also contains instructions for use of the packaged composition (e.g., a package insert that contains information required by a regulatory authority having jurisdiction over the manufacture, marketing, distribution, and sale of the particular CRF binding agent composition).

Another aspect of the disclosure relates to medical uses or methods of treating diseases, disorders, or conditions, involving administering a therapeutically effective amount of the CRF binding agent of the invention to a subject in need of such treatment.

In another aspect, the disclosure relates to new and more effective treatment methods and medical uses involving administering the inventive CRF binding agent or a pharmaceutical composition containing the CRF binding agent, for treating mammals, particularly humans, for conditions characterized by HPA axis hyperactivity. Such disease, disorders, and conditions include anxiety, depression, Alzheimer's and Parkinson's diseases, obesity, metabolic syndrome, osteoporosis, cardiovascular disease, alcohol and drug abuse, inflammatory bowel disease (IBD) and irritable bowel syndrome (IBS) (see, e.g., 24, 73, 74, 75, 76), as well as other conditions characterized by HPA axis hyperactivity. HPA axis hyperactivity predisposes a subject to a variety of illnesses, including cardiovascular disease, stress-induced obesity, metabolic syndrome, type II diabetes, osteoporosis, inflammatory bowel disease, alcohol and drug abuse, premature aging, and early death (15, 16, 17, 108, 109, 110, 111, 112). The practice of such methods comprises administering a therapeutically effective amount of a CRF binding agent of the invention, preferably as part of suitable composition, to a subject in need of such treatment, i.e., a patient or subject having (or predisposed to have or at risk of relapsing into) a disease, disorder, or condition characterized by HPA axis hyperactivity.

Other features and advantages of the invention will be apparent from the following description and from the claims.

### BRIEF DESCRIPTION OF THE DRAWINGS

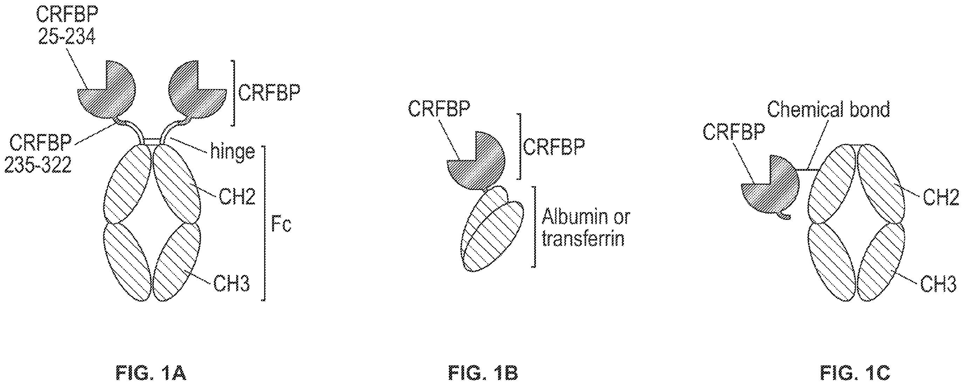

Figure 1A, Figure 1B, and Figure 1C: Some examples of CRF-BP-Fc constructs with extended half-life are shown in schematic format. Figure 1A illustrates CRF-BP fused to the IgG1 Fc domain to generate the full-length mature CRF-BP(25-322) fused to the IgG1 Fc via a naturally occurring IgG hinge to extend its circulating half-life. A fragment of CRF-BP having CRF binding activity under physiological conditions such as a shorter CRF-BP moiety (25-234), which may be proteolytically more stable, can also be used; CRF-BP can be fused directly to the IgG1 hinge or through a linker to provide greater spacing between the CRF-BP moiety and the Fc. Figure 1B illustrates monovalent (or divalent fusions, as also shown in Figure 1A) can also be obtained with albumin or transferrin either by fusion through the N and/or C terminus to obtain longer half-life. Figure 1C illustrates a monovalent, divalent, or polyvalent conjugations of CRF-BP with IgG Fc, albumin, transferrin or another half-life-extending moieties can also be obtained by chemical cross-linking to extend the circulating half-life of CRF-BP.

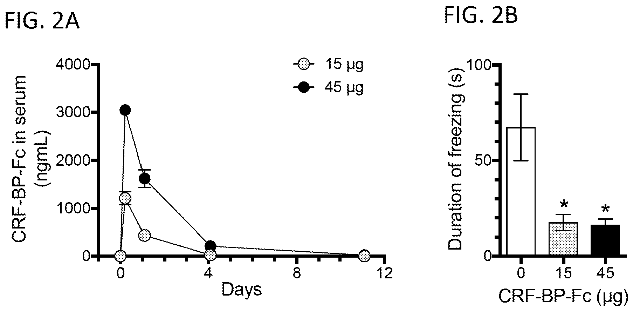

Figure 2A and Figure 2B: Serum concentration of CRF-BP-Fc. Figure 2A: A CRF-BP-Fc fusion protein (SEQ ID NO: 16), composed of CRF-BP (25-322) fused to mouse IgG1 Fc was administered to C57Bl6 mice at 2 doses (15 µg/mouse and 45 µg/mouse). The half-life was determined to be 25 hours after intraperitoneal administration, which is a considerable improvement over the half-life of unmodified CRF-BP (see, e.g., 115). Figure 2B: Freezing in response to mild footshock, a mouse model of stress, was reduced in mice treated with the CRF-BP-Fc fusion protein on the day of treatment (*<0.05 from vehicle-treated mice).

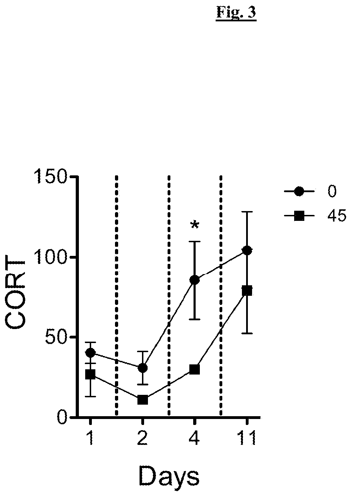

Figure 3: Administration of a CRF-BP-Fc fusion (SEQ ID NO: 16) delayed the increased in basal serum glucocorticoid levels ("CORT" in pg/mL x1000), resulting from repeated stress. Repeated delivery of footshock to mice (dotted vertical lines indicate times for delivery of footshock) results in elevated baseline serum corticosterone (CORT) level in C57Bl6 mice (black circles), indicative of a chronic stress state. A single administration of CRF-BP-Fc fusion protein at a dose of 45 µg/mouse delayed such increase of baseline serum CORT (black squares). 0=vehicle-treated mice; 45= CRF-BP-Fc-treated mice at 45 µg/mouse;*=p<0.05 from CRF-BP-Fc-treated mice; CORT: plasma corticosterone levels (ng/ml).

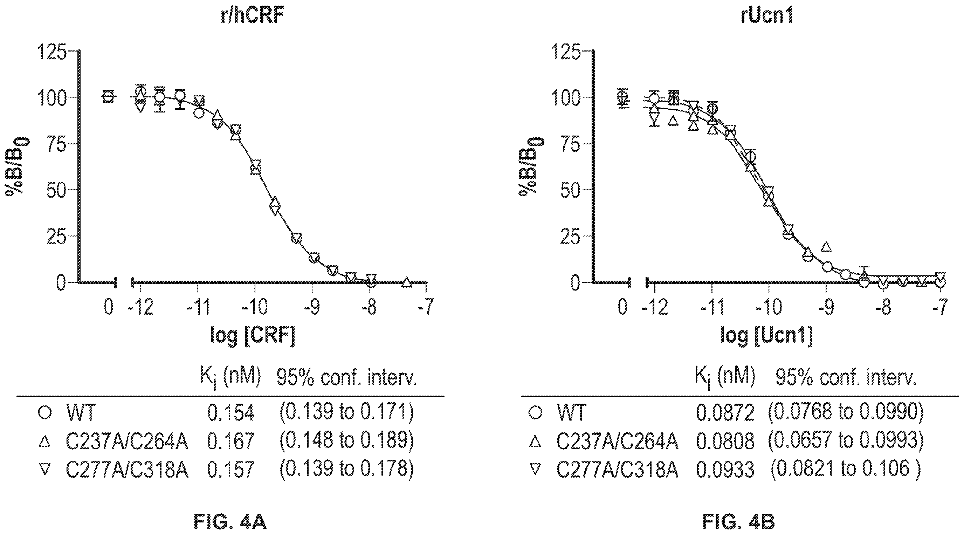

Figure 4A and Figure 4B: CRF-BP Derivatives. Removal of the cysteines forming the fourth (C237/C264) or fifth (C277/C318) disulfide bridge of CRF-BP has no effect on the affinities for r/hCRF or rUcn 1. Fig. 4A-B shows percent displacement (% B/B0) of 125I-[D-Tyr0r/hCRF] by r/hCRF (Figure 4A) or rUcn 1 (Figure 4B). Fig. 4 demonstrates that the affinity of CRFBPC237A/ C264A and CRF-BPC277A/C318A for r/hCRF or rUcn 1 is indistinguishable from that of WT CRF-BP (open symbols, dashed line). Ki values and 95% confidence intervals are derived from two or more separate experiments. (See, Huising et al., Residues of Corticotropin Releasing Factor-binding Protein (CRF-BP) that Selectively Abrogate Binding to CRF but Not to Urocortin 1, J. Biol. Chem. 283(14):8902-8912 (2008)).

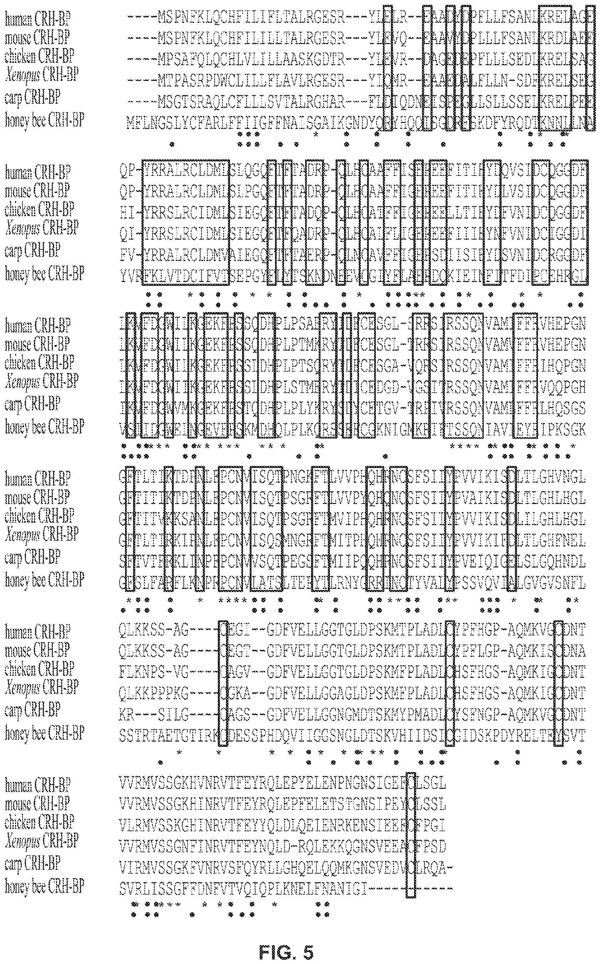

Figure 5. CRF-BP Sequence Alignment. Multiple amino acid alignment of CRF-BP ("CRH-BP") sequences from selected vertebrate and invertebrate species is shown, as published in Huising et al. (See, Huising et al., Residues of Corticotropin Releasing Factor-binding Protein (CRF-BP) that Selectively Abrogate Binding to CRF but Not to Urocortin 1, J. Biol. Chem. 283(14):8902-8912 (2008)). Amino acids targeted as part of our alanine scan are shaded. Asterisks indicate amino acid identity between all sequences in the alignment, while colons and dots indicate decreasing degrees of amino acid similarity. Accession numbers are as follows: human *(Homo sapiens),* UniProtKB No. P24387 (SEQ ID NO:1); mouse *(Mus musculus),* UniProtKB No. Q60571 (SEQ ID NO:2); chicken *(Gallus gallus),* GenBank No. XM_424801 (SEQ ID NO:3); Xenopus *(Xenopus laevis),* UniProtKB No. Q91653 (SEQ ID NO:4); carp *(Cyprinus carpio),* GenBank No. AJ490880 (SEQ ID NO:5); honey bee *(Apis mellifera),* GenBank No. AJ780964 (SEQ ID NO:6).

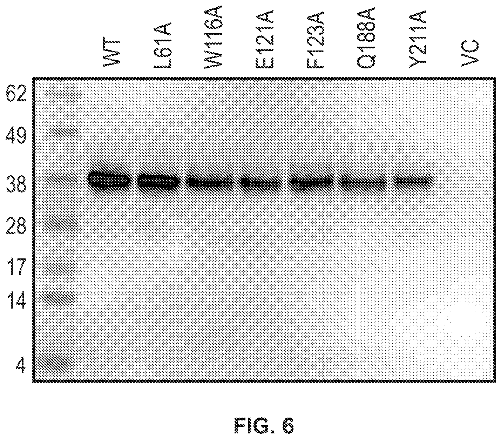

Figure 6. Western Blot. Western immunoblot of wild type (WT) CRF-BP and mutants that display partial (L61A, E121A, F123A, Q188A) or complete (W116A, Y211A) loss of affinity for both r/hCRF and rUcn 1 is shown, as published in Huising et al. (See, Huising et al., Residues of Corticotropin Releasing Factor-binding Protein (CRF-BP) that Selectively Abrogate Binding to CRF but Not to Urocortin 1, J. Biol. Chem. 283(14):8902-8912 (2008)). All mutant proteins express in similar levels compared to WT CRF-BP and have indistinguishable molecular weights as determined by SDS-page and detected by western immunoblot.

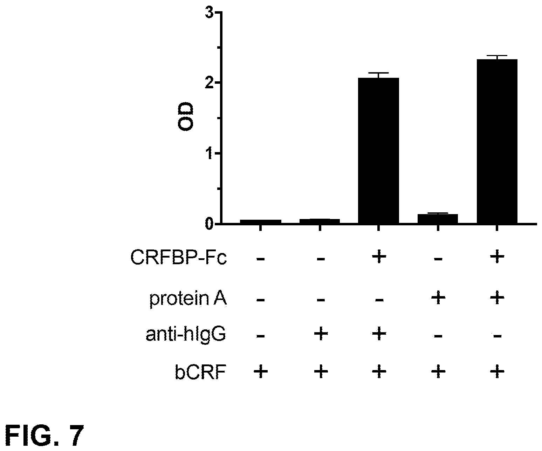

Figure 7 shows that CRFBP Fc fusion does not interfere with binding to CRF. Biotinylated CRF was immobilized on an avidin-coated ELISA plate. A CRFBP-Fc composed of CRF-BP(25-322)(SEQ ID NO:23) was detected by either a human antibody against human Fc (anti-hIgG) conjugated to enzyme horseradish peroxidase (HRP) or a by HRP-conjugated protein-A. On the absence of CRFBP-Fc fusion protein, HRP-conjugated anti-hIgG or protein-A did not detect any sugnal. OD= optical density.
Biotinylated CRF was immobilized on an avidin-coated ELISA plate. A CRFBP-Fc was detected by either a human antibody against human Fc (anti-hIgG) conjugated to enzyme horseradish peroxidase (HRP) or a by HRP-conjugated protein-A. On the absence of CRFBP Fc fusion protein, HRP-conjugated anti-hIgG or protein-A did not detect any sugnal. OD= optical density.

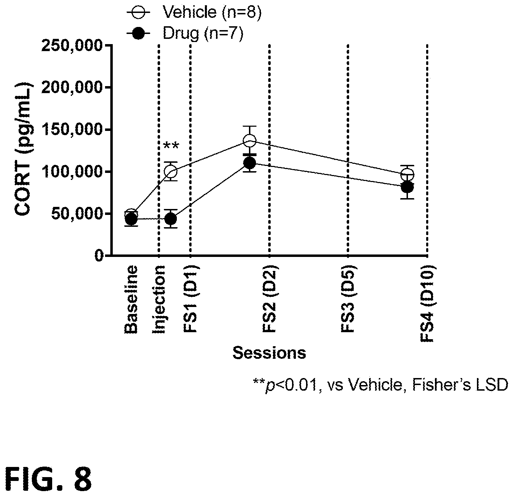

Figure 8 shows baseline corticosterone (CORT) concentration in mice treated with a CRFBP-Fc fusion protein composed of CRF-BP (25-322) fused to human IgG₂ Fc (SEQ ID NO:23), subjected to repeated foot-shock stress. CRFBP-Fc fusion protein was administered to C57Bl6 mice at a dose of 300 µg/mouse. Data points represent CORT concentration before and after injection of CRFBP-Fc containing IgG2-derived Fc (drug) and at 3 time points preceding repeated footshocks (FS). Repeated 2-way ANOVA revealed a significant main effect of Sessions (F_{(3,39)} = 26.48, *p* < 0.0001) and an interaction of Sessions and Treatment (F_{(3,39)} = 3.161, *p* = 0.0352). Post hoc analysis confirmed that drug treated group showed significantly less CORT conc. 3 hr after injection compared to vehicle group (***p* < 0.01, Fisher's LSD).

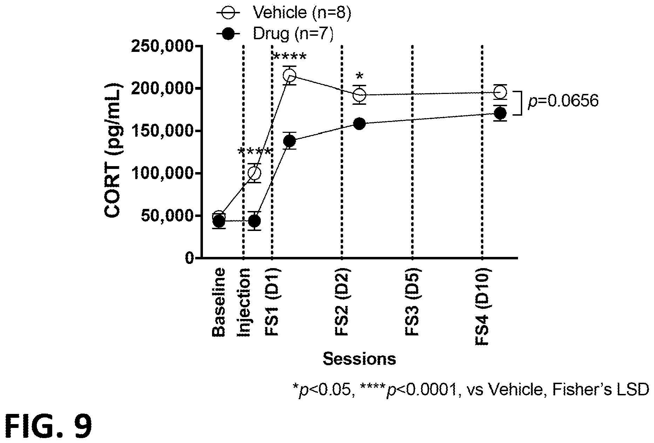

Figure 9 shows stimulated CORT serum concentrations in the same mice tested for Figure 8 above, before and after injection of a CRFBP-Fc fusion protein (SEQ ID NO:23), containing human IgG₂-derived Fc. Stimulated CORT concentrations after injection of CRFBP-Fc containing IgG2-derived Fc and at 3 time points after repeated footshocks (FS). Repeated 2-way ANOVA revealed significant main effects of Sessions (F(4,52) = 126.2, p < 0.0001) and Treatment (F(1,13) = 24.24, p = 0.0003) and an interaction of Sessions and Treatment (F(4,52) = 5.672, p = 0.0007). Post hoc analysis confirmed that drug treated group showed significantly less CORT conc. 3 hr after injection, FS1, and FS2 compared to vehicle group, respectively (*p < 0.05, ****p < 0.0001, Fisher's LSD).

### DETAILED DESCRIPTION

This invention concerns normalizing HPA axis hyperactivity by reducing CRF peak bursts by administering to a subject in need of such treatment an effective amount of an engineered corticotropin-releasing factor (CRF) binding agent as set out in the claims, for example, a fusion protein that comprises CRF binding protein (CRF-BP) coupled (or conjugated) to a half-life-extending moiety such as an Fc region of an antibody. When such a compound is administered in an amount sufficient to neutralize excess CRF activation mediated by CRF peak bursts, the engineered corticotropin-releasing factor (CRF) binding agent of the invention can, for example, decrease pituitary activation. Other half-life-extending moieties include, for example, an Fc forming portion of a mammalian immunoglobulin heavy chain, albumin, transferrin, transthyretin, PEG, and glycans.

The polypeptide having CRF binding activity disclosed herein may be a CRF binding protein (CRF-BP), CRF receptor type 1 (CRFR1), CRF receptor type 2 (CRFR2), or a fragment or derivative of CRF-BP, CRFR1, or CRFR2 having CRF binding activity under physiological conditions. Particularly preferred embodiments of such proteins include fragments or derivatives of a mammalian CRF-BP, preferably a human or murine CRF-BP derivative or fragment, e.g., hCRF-BP(25-234)(SEQ ID NO: 12), hCRF-BP(25-322)(SEQ ID NO: 13), rCRF-BP(25-234)(SEQ ID NO: 14), or rCRF-BP(25-322)(SEQ ID NO: 15).

Preferably, the polypeptide having CRF binding activity and the half-life-extending moiety(ies) is(are) covalently coupled to each other, optionally via a linker, preferably a peptide linker, preferably in the context of a fusion protein. Particularly preferred embodiments include CRF binding agents that comprise a hCRF-BP(25-234) polypeptide or a hCRF-BP(25-322) polypeptide coupled via a peptide linker to an Fc forming portion of a human immunoglobulin heavy chain, which CRF binding agents are preferably synthesized as fusion proteins engineered using recombinant techniques.

The linker's chemical structure is not critical, since it serves primarily as a spacer to position, join, connect, or optimize presentation or position of one functional moiety in relation to one or more other functional moieties of a molecule of the inventive CRF binding agent. The presence of a linker moiety can be useful in optimizing pharmacological activity of some embodiments of the inventive CRF binding agent. The linker is preferably made up of amino acids linked together by peptide bonds. The linker moiety, if present, can be independently the same or different from any other linker, or linkers, that may be present in the inventive CRF binding agent.

As stated above, the linker moiety, if present (whether within the primary amino acid sequence of the CRF binding agent, or as a linker for attaching a therapeutic moiety or half-life extending moiety to the inventive CRF binding agent), can be "peptidyl" in nature (i.e., made up of amino acids linked together by peptide bonds) and made up in length, preferably, of from 1 up to about 40 amino acid residues, more preferably, of from 1 up to about 20 amino acid residues, and most preferably of from 1 to about 10 amino acid residues. Preferably, but not necessarily, the amino acid residues in the linker are from among the twenty canonical amino acids, more preferably, cysteine, glycine, alanine, proline, asparagine, glutamine, and /or serine. Even more preferably, a peptidyl linker is made up of a majority of amino acids that are sterically unhindered, such as glycine, serine, and alanine linked by a peptide bond. It is also desirable that, if present, a peptidyl linker be selected that avoids rapid proteolytic turnover in circulation *in vivo.* Some of these amino acids may be glycosylated, as is well understood by those in the art. For example, a useful linker sequence constituting a sialylation site is X₁X₂NX₄X₅G (SEQ ID NO:27), wherein X1, X₂,X₄ and X₅ are each independently any amino acid residue. CRF-BP contains a single asparagine (Asn)-linked glycosylation site at aminao acid position 204 of SEQ ID NO:1, which is not involved in CRF binding (114) additional glycosylation sites can be added as, for instance in darbepoetin alfa (115), an engineered form of erythropoietin containing 2 new sites for N-linked carbohydrate addition, which is marketed by Amgen under the trade name Aranesp^{®}. The additional glycosylation sites results in a 3-fold longer serum half-life compared to epoetin alpha and epoetin beta.

In other embodiments, the 1 to 40 amino acids of the peptidyl linker moiety are selected from glycine, alanine, proline, asparagine, glutamine, and lysine. Preferably, a linker is made up of a majority of amino acids that are sterically unhindered, such as glycine and alanine. Thus, preferred linkers include polyglycines, polyserines, and polyalanines, or combinations of any of these. Some exemplary peptidyl linkers are poly(Gly)i_8, particularly (Gly)₃, (Gly)₄ (SEQ ID NO:28), (Gly)₅ (SEQ ID NO:29) and (Gly)₇ (SEQ ID NO:30). Other specific examples of peptidyl linkers include (Gly)sLys (SEQ ID NO:31), and (Gly)sLysArg (SEQ ID NO:32). Other examples of useful peptidyl linkers are:
(Gly)₃Lys(Gly)₄ (SEQ ID NO:33);
(Gly)₃AsnGlySer(Gly)₂ (SEQ ID NO:34);
(Gly)₃Cys(Gly)₄ (SEQ ID NO:35); and
GlyProAsnGlyGly (SEQ ID NO:36).

To explain the above nomenclature, for example, (Gly)₃Lys(Gly)₄ means Gly-Gly-Gly-Lys-Gly-Gly-Gly-Gly (SEQ ID NO: 37). Other combinations of Gly and Ala are also useful.

Commonly used peptidyl linkers include GGGGS//SEQ ID NO: 11; GGGGSGGGGS//SEQ ID NO:38); GGGGSGGGGSGGGGSGGGGSGGGGS//SEQ ID NO:39) and any linkers used in the working examples hereinafter.

In some embodiments of the compositions of this invention, which comprise a peptide linker moiety, acidic residues, for example, glutamate or aspartate residues, are placed in the amino acid sequence of the linker moiety.

In some embodiments of the compositions of this invention, which comprise a peptide or peptidyl linker moiety, acidic residues, for example, glutamate or aspartate residues, are placed in the amino acid sequence of the linker moiety.

Examples include the following peptide linker sequences:
GGEGGG (SEQ ID NO: 40);
GGEEEGGG (SEQ ID NO :41);
GEEEG (SEQ ID NO :42);
GEEE (SEQ ID NO:43);
GGDGGG (SEQ ID NO:44);
GGDDDGG (SEQ ID NO:45);
GDDDG (SEQ ID NO:46);
GDDD (SEQ ID NO:47);
GGGGSDDSDEGSDGEDGGGGS (SEQ ID NO:48);
WEWEW (SEQ ID NO:49);
FEFEF (SEQ ID NO:50);
EEEWWW (SEQ ID NO:51);
EEEFFF (SEQ ID NO:52);
WWEEEWW (SEQ ID NO:53); or
FFEEEFF (SEQ ID NO:54).

In other embodiments, the linker constitutes a phosphorylation site, e.g., X₁X₂YX₄X₅G (SEQ ID NO:55), wherein X₁, X₂, X₄, and X₅ are each independently any amino acid residue; X₁X₂SX₄X₅G (SEQ ID NO:56), wherein X₁, X₂, X₄ and X₅ are each independently any amino acid residue; or X₁X₂TX₄X₅G (SEQ ID NO:57), wherein X₁, X₂, X₄ and X₅ are each independently any amino acid residue.

The linkers shown here are exemplary; peptidyl linkers within the scope of this invention may be much longer and may include other residues. A peptidyl linker can contain, e.g., a cysteine, another thiol, or nucleophile for conjugation with a half- life extending moiety. In another embodiment, the linker contains a cysteine or homocysteine residue, or other 2-amino-ethanethiol or 3-amino-propanethiol moiety for conjugation to maleimide, iodoacetaamide or thioester, functionalized half-life extending moiety.

Another useful peptidyl linker is a large, flexible linker comprising a random Gly/Ser/Thr sequence, for example: GSGSATGGSGSTASSGSGSATH (SEQ ID NO:58) or HGSGSATGGSGSTASSGSGSAT (SEQ ID NO:59), that is estimated to be about the size of a 1 kDa PEG molecule. Alternatively, a useful peptidyl linker may be comprised of amino acid sequences known in the art to form rigid helical structures (e.g., Rigid linker: - AEAAAKEAAAKEAAAKAGG-) (SEQ ID NO:60). Additionally, a peptidyl linker can also comprise a non-peptidyl segment such as a 6 carbon aliphatic molecule of the formula -CH₂-CH₂-CH₂-CH₂-CH₂-CH₂-. The peptidyl linkers can be altered to form derivatives as described herein.

Optionally, a non-peptidyl linker moiety is also useful for conjugating the half-life extending moiety to the peptide portion of the half-life extending moiety- conjugated toxin peptide analog. For example, alkyl linkers such as -NH-(CH₂)_{S}- C(O)-, wherein s = 2-20 can be used. These alkyl linkers may further be substituted by any non-sterically hindering group such as lower alkyl (e.g., C₁-C₆) lower acyl, halogen (e.g., CI, Br), CN, NH₂, phenyl, etc. Exemplary non-peptidyl linkers are polyethylene glycol (PEG) linkers having a molecular weight of about 100 to about 5000 Daltons (Da), preferably about 100 to about 500 Da.

In one embodiment, the non-peptidyl linker is aryl. The linkers may be altered to form derivatives in the same manner as described in the art, e.g., in Sullivan et al, Toxin Peptide Therapeutic Agents, US2007/0071764; Sullivan et al, Toxin Peptide Therapeutic Agents, PCT/US2007/022831, published as WO2008/088422, which are all incorporated herein by reference in their entireties.

In addition, PEG moieties may be attached to the N-terminal amine or selected side chain amines by either reductive alkylation using PEG aldehydes or acylation using hydroxysuccinimido or carbonate esters of PEG, or by thiol conjugation.

"Aryl" is phenyl or phenyl vicinally- fused with a saturated, partially-saturated, or unsaturated 3-, 4-, or 5-membered carbon bridge, the phenyl or bridge being substituted by 0, 1, 2 or 3 substituents selected from Ci-s alkyl, C₁₋₄ haloalkyl or halo.

"Heteroaryl" is an unsaturated 5 , 6 or 7 membered monocyclic or partially-saturated or unsaturated 6-, 7-, 8-, 9-, 10- or 11 membered bicyclic ring, wherein at least one ring is unsaturated, the monocyclic and the bicyclic rings containing 1, 2, 3 or 4 atoms selected from N, O and S, wherein the ring is substituted by 0, 1, 2 or 3 substituents selected from C₁₋₈ alkyl, C₁₋₄ haloalkyl and halo.

Non-peptide portions of the inventive composition of matter, such as non-peptidyl linkers or non-peptide half-life extending moieties can be synthesized by conventional organic chemistry reactions.

The above is merely illustrative and not an exhaustive treatment of the kinds of linkers that can optionally be employed in accordance with the present invention.

The CRF binding agents of the invention can be used to treat animals, preferably mammals, particularly humans, for conditions characterized by HPA axis hyperactivity. Such disease, disorders, and conditions include anxiety, depression, Alzheimer's and Parkinson's diseases, obesity, metabolic syndrome, osteoporosis, cardiovascular disease, alcohol and drug abuse, inflammatory bowel disease (IBD) and irritable bowel syndrome (IBS), as well as other conditions characterized by HPA axis hyperactivity such as cardiovascular disease, stress-induced obesity, metabolic syndrome, type II diabetes, osteoporosis, inflammatory bowel disease, alcohol or drug abuse, premature aging, and early death.

### Definitions

In addition to the terms defined in this section, others are defined elsewhere in the specification, as necessary. Unless otherwise expressly defined herein, terms of art used in this specification will have their art-recognized meanings.

"Under physiological conditions" with respect to incubating buffers and other binding assay reagents, *in vitro,* e.g., CRF binding agents and immunoglobulins, means incubation under conditions of temperature, pH, and ionic strength, that permit a biochemical reaction, such as a non-covalent binding reaction, to occur. Typically, the temperature is at room or ambient temperature up to about 37°C and at pH 6.5-7.5. *In vivo,* the term "under physiological conditions" refers to conditions typical of the biological or intracellular environment under which a biochemical reaction or physiological process of interest can take place (e.g., non-covalent binding of a substrate molecule and ligand).

The term "recombinant" indicates that the material (e.g., a nucleic acid or a polypeptide) has been artificially or synthetically (i.e., non-naturally) altered by human intervention. The alteration can be performed on the material within, or removed from, its natural environment or state. For example, a "recombinant nucleic acid" is one that is made by recombining nucleic acids, e.g., during cloning, DNA shuffling or other well known molecular biological procedures. Examples of such molecular biological procedures are found in Maniatis et al., Molecular Cloning. A Laboratory Manual. Cold Spring Harbour Laboratory, Cold Spring Harbour, N.Y(1982). A "recombinant DNA molecule," is comprised of segments of DNA joined together by means of such molecular biological techniques.

The term "recombinant protein" or "recombinant polypeptide" as used herein refers to a protein molecule which is expressed using a recombinant DNA molecule.

A "recombinant host cell" is a cell that contains and/or expresses a recombinant nucleic acid. Recombinant expression technology typically involves a mammalian host cell comprising the recombinant expression vector with the expression cassette or at least the expression cassette, which may for example, be integrated into the host cell genome.

The term "host cell" means a cell that has been transformed, or is capable of being transformed, with a nucleic acid and thereby expresses a gene or coding sequence of interest. The term includes the progeny of the parent cell, whether or not the progeny is identical in morphology or in genetic make-up to the original parent cell, so long as the gene of interest is present. Any of a large number of available and well-known host cells may be used in the practice of this invention to obtain the inventive CRF binding agent. The selection of a particular host is dependent upon a number of factors recognized by the art. These include, for example, compatibility with the chosen expression vector, toxicity of the peptides encoded by the DNA molecule, rate of transformation, ease of recovery of the peptides, expression characteristics, bio-safety and costs. A balance of these factors must be struck with the understanding that not all hosts may be equally effective for the expression of a particular DNA sequence. Within these general guidelines, useful microbial host cells in culture include bacteria (such as *Escherichia coli* sp.), yeast (such as *Saccharomyces* sp.) and other fungal cells, algal or algal-like cells, insect cells, plant cells, mammalian (including human) cells, e.g., CHO cells and HEK-293 cells. Modifications can be made at the DNA level, as well. The peptide-encoding DNA sequence may be changed to codons more compatible with the chosen host cell. For *E*. *coli,* optimized codons are known in the art. Codons can be substituted to eliminate restriction sites or to include silent restriction sites, which may aid in processing of the DNA in the selected host cell. Next, the transformed host is cultured and purified. Host cells may be cultured under conventional fermentation conditions so that the desired compounds are expressed. Such fermentation conditions are well known in the art.

Examples of useful mammalian host cell lines are Chinese hamster ovary cells, including CHO-K1 cells (e.g., ATCC CCL61), DXB-11, DG-44, and Chinese hamster ovary cells/-DHFR (CHO, Urlaub et al, Proc. Natl. Acad. Sci. USA 77: 4216 (1980)); monkey kidney CVl line transformed by SV40 (COS-7, ATCC CRL 1651); human embryonic kidney line (293 or 293 cells subcloned for growth in suspension culture (Graham et al, J. Gen Virol. 36: 59 (1977)); baby hamster kidney cells (BHK, ATCC CCL 10); mouse Sertoli cells (TM4, Mather, Biol. Reprod. 23: 243-251 (1980)); monkey kidney cells (CVl ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human hepatoma cells (Hep G2, HB 8065); mouse mammary tumor (MMT 060562, ATCC CCL51); TRI cells (Mather et al., Annals N.Y Acad. Sci. 383: 44-68 (1982)); MRC 5 cells or FS4 cells; or mammalian myeloma cells, e.g., sp2/0 mouse myeloma cells.

"Cell," "cell line," and "cell culture" are often used interchangeably and all such designations herein include cellular progeny. For example, a cell "derived" from a CHO cell is a cellular progeny of a Chinese Hamster Ovary cell, which may be removed from the original primary cell parent by any number of generations, and which can also include a transformant progeny cell. Transformants and transformed cells include the primary subject cell and cultures derived therefrom without regard for the number of transfers. It is also understood that all progeny may not be precisely identical in DNA content, due to deliberate or inadvertent mutations. Mutant progeny that have the same function or biological activity as screened for in the originally transformed cell are included.

A "half-life extending moiety," or interchangeably, a "carrier moiety," refers to a pharmacologically inactive molecule to which a pharmacologically active chemical moiety, such as the corticotropin-releasing factor (CRF) binding agent of the invention, can be covalently conjugated or fused. Effective half-life extending moieties have been sought to prevent or mitigate *in vivo* degradation of pharmacologically active moieties by proteolysis or other *in vivo* activity-diminishing chemical modifications of the pharmacologically active chemical moiety, or to reduce renal clearance, to enhance *in vivo* half-life or other pharmacokinetic properties of a therapeutic, such as increasing the rate of absorption, reducing toxicity or immunogenicity, improving solubility, and/or increasing manufacturability or storage stability, compared to an unconjugated form of the pharmacologically active moiety.

Examples of such half-life extending moieties that have been employed in the pharmaceutical industry, and which can be employed in practicing the present invention, include polyethylene glycol (see, e.g., Burg et al., Erythropoietin conjugates with polyethylene glycol, WO 01/02017), immunoglobulin Fc domain (see, e.g., Feige et al, Modified peptides as therapeutic agents, US Patent No. 6,660,843), human serum albumin (see, e.g., Rosen et al., Albumin fusion proteins, US Patent No. 6,926,898 and US 2005/0054051; Bridon et al, Protection of endogenous therapeutic peptides from peptidase activity through conjugation to blood components, US 6,887,470), transthyretin (see, e.g., Walker et al., Use of transthyretin peptide/protein fusions to increase the serum half-life of pharmacologically active peptides/proteins, US 2003/0195154 A1; 2003/0191056 A1), or thyroxine-binding globulin, or a combination such as immunoglobulin (light chain+heavy chain) and Fc domain (the heterotrimeric combination a so-called "hemibody"), for example as described in Sullivan et al, Toxin Peptide Therapeutic Agents, PCT/US2007/022831, published as WO 2008/088422. Pharmacologically active moieties have also been conjugated to a peptide or small molecule that has an affinity for a long half-life serum protein. (See, e.g., Blaney et al., Method and compositions for increasing the serum half-life of pharmacologically active agents by binding to transthyretin-selective ligands, US Patent. No. 5,714,142; Sato et al, Serum albumin binding moieties, US 2003/0069395 A1; Jones et al, Pharmaceutical active conjugates, US Patent No. 6,342,225). Fischer et al. described a peptide -immunoglobulin-conjugate, in which the immunoglobulin consisted of two heavy chains or two heavy chains and two light chains, in which the immunoglobulin was not a functionable immunoglobulin (Fischer et al, A peptide-immunoglobulin conjugate, WO 2007/045463 A1).

The term "antibody" ("Ab") or "immunoglobulin" (Ig) refers to any form of a peptide, polypeptide derived from, modeled after or encoded by, an immunoglobulin gene, or fragment thereof, which is capable of binding an antigen or epitope. See, e.g., IMMUNOBIOLOGY, Fifth Edition, Janeway, et al., ed. Garland Publishing (2001). The term "antibody" is used herein in the broadest sense, and encompasses monoclonal, polyclonal or multispecific antibodies, minibodies, heteroconjugates, diabodies, triabodies, chimeric, antibodies, synthetic antibodies, antibody fragments that retain antigen binding activity, and binding agents that employ the complementarity determining regions (CDRs) of a parent antibody. Antibodies are defined herein as retaining at least one desired activity of the parent antibody. Desired activities may include the ability to bind the antigen, the ability to bind the antigen preferentially, and the ability to alter cytokine profile(s) *in vitro.*

Native antibodies (native immunoglobulins) are usually heterotetrameric glycoproteins of about 150 kiloDaltons (kDa), typically composed of two "identical" (in terms of primary amino acid sequence) light (L) chains and two identical heavy (H) chains. The heavy chain is approximately 50 kD in size, and the light chain is approximately 25 kDa. Each light chain is typically linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end. The constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The light chains of antibodies from any vertebrate species can be assigned to one of two clearly distinct types, called kappa (κ) and lambda (λ), based on the amino acid sequences of their constant domains. The ratio of the two types of light chain varies from species to species. As a way of example, the average κ to λ ratio is 20:1 in mice, whereas in humans it is 2:1 and in cattle it is 1:20.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known.

An antibody may be designed and/or prepared from the amino acid sequence of another antibody (often referred to as the "parent" or "native" antibody) that is directed to the same antigen by virtue of addition, deletion, and/or substitution of one or more amino acid residue(s) in the antibody sequence and which retains at least one desired activity of the parent antibody. Desired activities can include the ability to bind the antigen specifically, the ability to inhibit proliferation *in vitro,* the ability to inhibit angiogenesis *in vivo,* and the ability to alter cytokine profile *in vitro.* The amino acid change(s) may be within a variable region or a constant region of a light chain and/or a heavy chain, including in the Fc region, the Fab region, the CH1 domain, the CH2 domain, the CH3 domain, and the hinge region. In some embodiments one or more amino acid substitution(s) are made in one or more hypervariable region(s) of the parent antibody. For example, there may be at least one, e.g., from about one to about ten, and preferably from about two to about five, substitutions in one or more hypervariable regions compared to the parent antibody. Ordinarily, amino acid changes will result in a new antibody amino acid sequence having at least 50% amino acid sequence identity with the parent antibody heavy or light chain variable domain sequences, more preferably at least 65%, more preferably at 80%, more preferably at least 85%, more preferably at least 90%, and most preferably at least 95%. Identity or homology with respect to this sequence is defined herein as the percentage of amino acid residues in the candidate sequence that are identical with the parent antibody residues, after aligning the sequences and introducing gaps, if necessary, to achieve the maximum percent sequence identity. None of N-terminal, C-terminal, or internal extensions, deletions, or insertions into the antibody sequence shall be construed as affecting sequence identity or homology.

As used herein, "antibody fragment", "antigen-binding antibody fragment", and the like refer to a portion of an intact antibody that includes the antigen binding site(s) or variable regions of an intact antibody, wherein the portion can be free of the constant heavy chain domains (*e.g*., CH2, CH3, and CH4) of the Fc region of the intact antibody. Alternatively, portions of the constant heavy chain domains (*e.g*., CH2, CH3, and CH4) can be included in the "antibody fragment." Antibody fragments retain antigen-binding ability and include Fab, Fab', F(ab')2, Fd, and Fv fragments; diabodies; triabodies; single-chain antibody molecules (sc-Fv); minibodies; nanobodies; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen. By way of example, an Fab fragment also contains the constant domain of a light chain and the first constant domain (CH1) of a heavy chain. "Fv" is the minimum antibody fragment that contains a complete antigen-recognition and -binding site. This region consists of a dimer of one heavy chain and one light chain variable domain in tight, non-covalent association. It is in this configuration that the three hypervariable regions (or complementarity determining regions or "CDRs") of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site. "Single-chain Fv" or "sFv" antibody fragments comprise the VH and VL domains of a parent antibody, wherein these domains are present in a single polypeptide chain. Generally, the Fv polypeptide further comprises a polypeptide linker between the VH and VL domains that enables the sFv to form the desired structure for antigen binding. For a review of sFv. See Pluckthun in The Pharmacology of Monoclonal Antibodies, vol. 113, Rosenburg and Moore eds. Springer-Verlag, New York, pp. 269-315 (1994).

The Fab fragment also contains the constant domain of the light chain and the first constant domain (CH1) of the heavy chain. Fab' fragments differ from Fab fragments by the addition of a few residues at the carboxyl terminus of the heavy chain CH1 domain including one or more cysteine(s) from the antibody hinge region. Fab'-SH is the designation for an Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known and are within the scope of the invention.

A "binding partner" is any molecule that is complementary to one or more regions on a chimera composition of the invention via association by chemical or physical means. For the purposes of the present invention, the binding partner may be a compound that facilitates binding of the composition with other members of a protein signaling complex, or a compound that interferes with the association of a known binding pair. Examples of binding partners that can be investigated and/or identified using this invention include, but are not restricted to: peptides; polypeptides; proteins (including derivatized or labeled proteins); antibodies or fragments thereof; small molecules; aptamers; carbohydrates and/or other non-protein binding moieties; derivatives and fragments of a naturally-occurring binding partners; peptidomimetics; and pharmacophores.

The term "biologically active," in the context of CRF binding agent, refers to engineered protein or polypeptide that is capable of binding CRF and in some way exerting a biologic effect. Biological effects include, but are not limited to, CRF binding.

The term "combination therapy" refers to a therapeutic regimen that involves the provision of at least two distinct therapies to achieve an indicated therapeutic effect. For example, a combination therapy may involve the administration of two or more chemically distinct active ingredients. Alternatively, a combination therapy may involve the administration of a CRF binding agent according to the invention together with the delivery of another treatment, such as psychotherapy and/or surgery. In the context of the administration of two or more chemically distinct active ingredients, one of which is a CRF binding agent of the invention, it is understood that the active ingredients may be administered as part of the same composition or as different compositions. When administered as separate compositions, the compositions comprising the different active ingredients may be administered at the same or different times, by the same or different routes, using the same of different dosing regimens, all as the particular context requires and as determined by the attending physician. Similarly, when one or more targeted drug conjugate species of the invention, alone or in conjunction with one or more chemotherapeutic agents, are combined with, for example, radiation and/or surgery, the drug(s) may be delivered before or after surgery or the other treatment(s).

The term "complementary" refers to the topological compatibility or interactive structure of interacting surfaces of a composition of the invention and a binding partner. Thus, the composition of the invention and its identified binding partners can be described as complementary, and furthermore, the contact surface characteristics are each complementary to each other. Preferred complementary structures have binding affinity for each other and the greater the degree of complementarity the structures have for each other the greater the binding affinity between the structures.

The term "CRF" refers to Corticotropin Releasing Factor (also known in the art as Corticotropin-releasing hormone (CRH)), and includes any active fragments, sequence variants, modified peptides, derivatives, or peptidomimetics that are based on corticotrophin releasing factor with substantially the same activity.

The term "CRF-BP," used interchangeably herein with "CRFBP," refers to a binding protein that specifically binds to CRF, and fragments of such a CRF-BP protein that retain such specific binding capacity. Examples of CRF-BPs that occur in nature include CRF-BPs of human (UniProtKB No. P24387 (SEQ ID NO:1); mouse *(Mus musculus),* UniProtKB No. Q60571 (SEQ ID NO:2); chicken *(Gallus gallus),* GenBank No. XM_424801 (SEQ ID NO:3); Xenopus *(Xenopus laevis),* UniProtKB No. Q91653 (SEQ ID NO:4); carp *(Cyprinus carpio),* GenBank No. AJ490880 (SEQ ID NO:5); honey bee (*Apis mellifera*), GenBank No. AJ780964 (SEQ ID NO:6); and CRF-BP of many other species. The present invention relates to engineered CRF-BP agents as set out in the claims.

The term "engineered" refers to a compound that does not occur naturally but instead has been designed by man. As such, "engineered" compounds include those that are derived from a naturally occurring compound, for example, a CRF-BP protein, but have been modified in a desired fashion. Examples of engineered compounds include fusion proteins, Fab fragments, and non-naturally occurring fragments of CRF-BP, for example, hCRF-BP(25-234)(SEQ ID NO:12), hCRF-BP(25-322)(SEQ ID NO:13), rCRF-BP(25-234)(SEQ ID NO: 14), and rCRF-BP(25-322)(SEQ ID NO: 15).

In general, the inventive CRF binding agent, "specifically binds" to CRF when it has a significantly higher binding affinity for, and consequently is capable of distinguishing CRF, compared to its affinity for other unrelated proteins, under similar binding assay conditions. Typically, a CRF binding agent of the invention is said to "specifically bind" its target (i.e., CRF) when the dissociation constant (K_{D}) is 10⁻⁸ M, or less. The CRF binding agent specifically binds CRF with "high affinity" when the K_{D} is 5 x 10⁻⁹ M, or less, and with "very high affinity" when the K_{D} is 5x 10⁻¹⁰ M, or less, e.g., about 10⁻¹⁰ M, about 10⁻¹¹ M, or about 10⁻¹² M. In one embodiment, the inventive CRF binding agent will bind to CRF with a K_{D} of between about 10⁻⁸ M and about 10⁻¹² M, and in yet another embodiment the CRF binding agent will bind CRF with a K_{D} ≤ 5 x 10⁻⁹ M. (See, e.g., Potter et al., Cloning and characterization of the cDNAs for human and rat corticotropin releasing factor-binding proteins, Nature 349:423-26 (1991); Huising et al., Residues of Corticotropin Releasing Factor-binding Protein (CRF-BP) that Selectively Abrogate Binding to CRF but Not to Urocortin 1, J. Biol. Chem. 283(14):8902-8912 (2008)).

An "epitope" or "antigenic determinant" refers to that portion of an antigen that reacts with an antibody antigen-binding portion derived from an antibody.

A "fully human antibody" can refer to an antibody produced in a genetically engineered (*i.e.,* transgenic) mouse (*e.g.,* HUMAB-MOUSE from Medarex Inc., Princeton NJ) that, when presented with an immunogen, can produce a human antibody that does not necessarily require CDR grafting. These antibodies are fully human (100% human protein sequences) from animals such as mice in which the non-human antibody genes are suppressed and replaced with human antibody gene expression. Such antibodies can be generated against desired biological targets (*e.g*., the extracellular domains of proteins expressed on the luminal surfaces of endothelial cells forming caveolae) when presented to genetically engineered mice or other non-human animals engineered to produce human frameworks for the relevant CDRs.

"Humanized" forms of non-human (*e.g*., murine) antibodies are chimeric antibodies that contain minimal sequence derived from a non-human immunoglobulin. Or, looked at another way, a humanized antibody is a human antibody that also contains selected amino acid residues from non-human (*e.g*., murine) antibodies in place of the amino acid residue(s) found at the same amino acid position in corresponding heavy or light Ig chains. A humanized antibody can include conservative amino acid substitutions or non-natural residues from the same or different species that do not significantly alter its binding and/or biologic activity. Such antibodies contain minimal sequence derived from non-human immunoglobulins. For the most part, humanized antibodies are human immunoglobulins (recipient antibody) in which residues from a complementary-determining region (CDR) of the human antibody are replaced by residues from a CDR of a non-human species (donor antibody) such as mouse, rat, camel, bovine, goat, or rabbit having the desired properties. In some instances, framework region (FR) residues of the human immunoglobulin are replaced by corresponding residues from the non-human parent antibody (each replacement being called a "backmutation").

Furthermore, humanized antibodies can comprise residues that are found neither in the recipient antibody nor in the imported CDR or framework sequences. These modifications are made to further refine and maximize antibody performance. Thus, in general, a humanized antibody will comprise variable domains in which all or all of the hypervariable loops correspond to those of a non-human immunoglobulin and all or substantially all of the framework regions are those of a human immunoglobulin sequence. Such humanized antibodies optionally also will comprise at least a portion of an immunoglobulin constant region (Fc), or that of a human immunoglobulin. *See, e.g.,* U.S. patent no. 4,816,567; European patent no. 0,125,023 B1; U.S. patent no. 4,816,397; European patent no. 0,120,694 B1; WO 86/01533; European patent no. 0,194,276 B1; U.S. patent no. 5,225,539; European patent no. 0,239,400 B1; European patent application no. 0,519,596 A1; Queen, et al. (1989), Proc. Nat'l Acad. Sci. USA, vol. 86:10029-10033). For further details, see Jones, et al., Nature 321:522-525 (1986); Reichmann, et al., Nature 332:323-329 (1988); and Presta, Curr Op Struct Biol 2:593-596 (1992). Humanized antibodies may be preferred to nonhuman antibodies for use in humans because the human body may mount an immune response against the nonhuman antibodies that are viewed as a foreign substance. A human anti-mouse antibody (HAMA) response has been observed in a significant fraction of patients given mouse antibody therapy.

The term "fused", in the context of a CRF binding agent of the invention, refers to any mechanistic, chemical, or recombinant approach for attaching a polypeptide having CRF binding activity under physiological conditions with a half-life-extending peptide, polypeptide, or protein. The "fusion" of the second peptide to the first peptide may be a direct fusion of the sequences, with the second peptide directly adjacent to the first peptide, or it may be an indirect fusion, e.g., with intervening amino acid sequence such as an identifier or epitope tag sequence, a domain, a functional peptide, or a larger polypeptide or protein. In some embodiments, the fused polypeptides are expressed from a gene that codes for both of them so that upon expression of the gene, the polypeptides are part of the same protein (e.g., a "fusion protein"). In other embodiments, the two peptides may be "fused" following co-expression in a recombinant host cell, using high affinity binding sequences between the two peptides, such as biotin and avidin or strepavidin. In yet other examples, the two peptides are fused following expression and purification of each polypeptide, after which they are synthetically tethered together, perhaps by linking the C-terminus of the first polypeptide to the N-terminus of the other polypeptide.

To "inhibit," particularly in the context of a biological phenomenon, means to decrease, reduce, suppress or delay.

A "liquid composition" refers to one that, in its filled and finished form as provided from a manufacturer to an end user (*e.g*., a doctor or nurse), is a liquid or solution, as opposed to a solid. Here, "solid" refers to compositions that are not liquids or solutions. For example, solids include dried compositions prepared by lyophilization, freeze-drying, precipitation, and similar procedures.

The term "monoclonal antibody" (mAb) as used herein refers to an antibody obtained from a population of substantially homogeneous antibodies, or to said population of antibodies. The individual antibodies comprising the population are essentially identical, except for possible naturally occurring mutations, post-translational modifications, and the like that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler, et al., Nature 256:495 (1975), or may be made by recombinant DNA methods (*see, e.g.,* U.S. pat. no. 4,816,567). "Monoclonal antibodies" may also be isolated from phage antibody libraries using the techniques described in Clackson, et al., Nature (1991), 352:624-628, and Marks, et al. (1991), J Mol Biol 222:581-597, for example, or by other methods known in the art. The monoclonal antibodies useful in the context of this invention specifically include chimeric antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (*see, e.g.,* U.S. patent no. 4,816,567; and Morrison, et al. (1984), Proc Natl Acad Sci USA 81:6851-6855).

"Monotherapy" refers to a treatment regimen based on the delivery of one therapeutically effective compound, whether administered as a single dose or several doses over time.

The terms "peptide," "polypeptide," and "protein" are used interchangeably herein, and refer to a polymeric form of amino acid residues of any length, which can include coded and non-coded amino acids, chemically or biochemically modified or derivatized amino acids, and polypeptides having modified peptide backbones.

An amino acid substitution in an amino acid sequence is typically designated herein with a one-letter abbreviation for the amino acid residue in a particular position, followed by the numerical amino acid position relative to an original sequence of interest, which is then followed by the one-letter symbol for the amino acid residue substituted in. For example, "T30D" symbolizes a substitution of a threonine residue by an aspartate residue at amino acid position 30, relative to the original sequence of interest. Another example, "W101F" symbolizes a substitution of a tryptophan residue by a phenylalanine residue at amino acid position 101, relative to the original sequence of interest.

Non-canonical amino acid residues can be incorporated into a polypeptide within the scope of the invention by employing known techniques of protein engineering that use recombinantly expressing cells. (See, e.g., Link et al, Non-canonical amino acids in protein engineering, Current Opinion in Biotechnology, 14(6):603-609 (2003)). The term "non-canonical amino acid residue" refers to amino acid residues in D- or L-form that are not among the 20 canonical amino acids generally incorporated into naturally occurring proteins, for example, β-amino acids, homoamino acids, cyclic amino acids and amino acids with derivatized side chains. Examples include (in the L-form or D-form) β-alanine, β-aminopropionic acid, piperidinic acid, aminocaprioic acid, aminoheptanoic acid, aminopimelic acid, desmosine, diaminopimelic acid, N^{α}-ethylglycine, N^{α}-ethylaspargine, hydroxylysine, allo-hydroxylysine, isodesmosine, allo-isoleucine, ω-methylarginine, N^{α}-methylglycine, N^{α}-methylisoleucine, N^{α}-methylvaline, , γ-carboxyglutamate, ε-N,N,N-trimethyllysine, ε-N-acetyllysine, O-phosphoserine, N^{α}-acetylserine, N^{α}-formylmethionine, 3-methylhistidine, 5-hydroxylysine, and other similar non-canonical amino acids, and derivatized forms of any of these as described herein. The skilled practitioner will understand that different abbreviations and nomenclatures may be applicable to the same substance and appear interchangeably herein. Nomenclature and Symbolism for Amino Acids and Peptides by the UPAC-IUB Joint Commission on Biochemical Nomenclature (JCBN) have been published in the following documents: Biochem. J., 1984, 219, 345-373; Eur. J. Biochem., 1984, 138, 9-37; 1985, 152, 1; 1993, 213, 2; Intemat. J. Pept. Prot. Res., 1984, 24, following p 84; J. Biol. Chem., 1985, 260, 14-42; Pure Appl. Chem., 1984, 56, 595-624; Amino Acids and Peptides, 1985, 16, 387-410; Biochemical Nomenclature and Related Documents, 2nd edition, Portland Press, 1992, pages 39-69.

The one or more useful modifications to peptide domains of the inventive CRF binding agent can include amino acid additions or insertions, amino acid deletions, peptide truncations, amino acid substitutions, and/or chemical derivatization of amino acid residues, accomplished by known chemical techniques. For example, the thusly modified amino acid sequence includes at least one amino acid residue inserted or substituted therein, relative to the amino acid sequence of the native sequence of interest, in which the inserted or substituted amino acid residue has a side chain comprising a nucleophilic or electrophilic reactive functional group by which the peptide is conjugated to a linker and/or half-life extending moiety. In accordance with the invention, useful examples of such a nucleophilic or electrophilic reactive functional group include, but are not limited to, a thiol, a primary amine, a seleno, a hydrazide, an aldehyde, a carboxylic acid, a ketone, an aminooxy, a masked (protected) aldehyde, or a masked (protected) keto functional group. Examples of amino acid residues having a side chain comprising a nucleophilic reactive functional group include, but are not limited to, a lysine residue, a homolysine, an α,β-diaminopropionic acid residue, an α,γ-diaminobutyric acid residue, an omithine residue, a cysteine, a homocysteine, a glutamic acid residue, an aspartic acid residue, or a selenocysteine residue.

Amino acid residues are commonly categorized according to different chemical and/or physical characteristics. The term "acidic amino acid residue" refers to amino acid residues in D- or L-form having side chains comprising acidic groups. Exemplary acidic residues include aspartatic acid and glutamatic acid residues. The term "basic amino acid residue" refers to amino acid residues in D- or L-form having side chains comprising basic groups. Exemplary basic amino acid residues include histidine, lysine, homolysine, ornithine, arginine, N-methyl-arginine, ω-aminoarginine, ω-methyl-arginine, 1- methyl-histidine, 3-methyl-histidine, and homoarginine (hR) residues. The term "hydrophilic amino acid residue" refers to amino acid residues in D- or L-form having side chains comprising polar groups. Exemplary hydrophilic residues include cysteine, serine, threonine, histidine, lysine, asparagine, aspartate, glutamate, glutamine, and citrulline (Cit) residues. The terms "lipophilic amino acid residue" refers to amino acid residues in D- or L-form having sidechains comprising uncharged, aliphatic or aromatic groups. Exemplary lipophilic sidechains include phenylalanine, isoleucine, leucine, methionine, valine, tryptophan, and tyrosine. Alanine (A) is amphiphilic- it is capable of acting as a hydrophilic or lipophilic residue. Alanine, therefore, is included within the definition of both "lipophilic residue" and "hydrophilic residue." The term "nonfunctional amino acid residue" refers to amino acid residues in D- or L-form having side chains that lack acidic, basic, or aromatic groups. Exemplary neutral amino acid residues include methionine, glycine, alanine, valine, isoleucine, leucine, and norleucine (Nle) residues.

Additional useful embodiments of the invention can result from conservative modifications of the amino acid sequences of the polypeptides disclosed herein. Conservative modifications will produce half-life extending moiety-conjugated peptides having functional, physical, and chemical characteristics similar to those of the conjugated (e.g., PEG-conjugated) peptide from which such modifications are made. Such conservatively modified forms of the conjugated polypeptides disclosed herein are also contemplated as being an embodiment of the present invention.

In contrast, substantial modifications in the functional and/or chemical characteristics of peptides may be accomplished by selecting substitutions in the amino acid sequence that differ significantly in their effect on maintaining (a) the structure of the molecular backbone in the region of the substitution, for example, as an a-helical conformation, (b) the charge or hydrophobicity of the molecule at the target site, or (c) the size of the molecule.

For example, a "conservative amino acid substitution" may involve a substitution of a native amino acid residue with a normative residue such that there is little or no effect on the polarity or charge of the amino acid residue at that position. Furthermore, any native residue in the polypeptide may also be substituted with alanine, as has been previously described for "alanine scanning mutagenesis" (see, for example, MacLennan et al, Acta Physiol. Scand. SuppL, 643:55-67 (1998); Sasaki et al, 1998, Adv. Biophys. 35: 1-24 (1998), which discuss alanine scanning mutagenesis).

Desired amino acid substitutions (whether conservative or non- conservative) can be determined by those skilled in the art at the time such substitutions are desired. For example, amino acid substitutions can be used to identify important residues of the peptide sequence, or to increase or decrease the affinity of the peptide or vehicle-conjugated peptide molecules described herein.

Naturally occurring residues may be divided into classes based on common side chain properties:
1) hydrophobic: norleucine (Nor or Nle), Met, Ala, Val, Leu, Ile;
2) neutral hydrophilic: Cys, Ser, Thr, Asn, Gln;
3) acidic: Asp, Glu;
4) basic: His, Lys, Arg;
5) residues that influence chain orientation: Gly, Pro; and
6) aromatic: Trp, Tyr, Phe.

Conservative amino acid substitutions may involve exchange of a member of one of these classes with another member of the same class. Conservative amino acid substitutions may encompass non-naturally occurring amino acid residues, which are typically incorporated by chemical peptide synthesis rather than by synthesis in biological systems. These include peptidomimetics and other reversed or inverted forms of amino acid moieties.

Non-conservative substitutions may involve the exchange of a member of one of these classes for a member from another class. Such substituted residues may be introduced into regions of the toxin peptide analog.

In making such changes, according to certain embodiments, the hydropathic index of amino acids may be considered. Each amino acid has been assigned a hydropathic index on the basis of its hydrophobicity and charge characteristics. They are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art (see, for example, Kyte et al, 1982, J. Mol. Biol. 157: 105-131). It is known that certain amino acids may be substituted for other amino acids having a similar hydropathic index or score and still retain a similar biological activity. In making changes based upon the hydropathic index, in certain embodiments, the substitution of amino acids whose hydropathic indices are within ±2 is included. In certain embodiments, those that are within ±1 are included, and in certain embodiments, those within ±0.5 are included.

It is also understood in the art that the substitution of like amino acids can be made effectively on the basis of hydrophilicity, particularly where the biologically functional protein or peptide thereby created is intended for use in immunological embodiments, as disclosed herein. In certain embodiments, the greatest local average hydrophilicity of a protein, as governed by the hydrophilicity, of its adjacent amino acids, correlates with its immunogenicity and antigenicity, i.e., with a biological property of the protein.

The following hydrophilicity values have been assigned to these amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). In making changes based upon similar hydrophilicity, values, in certain embodiments, the substitution of amino acids whose hydrophilicity values are within ±2 is included, in certain embodiments, those that are within ±1 are included, and in certain embodiments, those within ±0.5 are included. One may also identify epitopes from primary amino acid sequences on the basis of hydrophilicity. These regions are also referred to as "epitopic core regions."

Examples of conservative substitutions include the substitution of one nonpolar (hydrophobic) amino acid residue such as isoleucine, valine, leucine norleucine, alanine, or methionine for another, the substitution of one polar (hydrophilic) amino acid residue for another such as between arginine and lysine, between glutamine and asparagine, between glycine and serine, the substitution of one basic amino acid residue such as lysine, arginine or histidine for another, or the substitution of one acidic residue, such as aspartic acid or glutamic acid for another. The phrase "conservative amino acid substitution" also includes the use of a chemically derivatized residue in place of a nonderivatized residue, provided that such polypeptide displays the requisite bioactivity. Other exemplary amino acid substitutions that can be useful in accordance with the present invention are set forth in Table 1 below.

**Table 1. Some Useful Amino Acid Substitutions**

| Original Residue | Exemplary Residue Substitutions |
|---|---|
| Ala | Val, Leu, Ile |
| Arg | Lys, Gln, Asn |
| Asn | Gin |
| Asp | Glu |
| Cys | Ser, Ala |
| Gln | Asn |
| Glu | Asp |
| Gly | Pro, Ala |
| His | Asn, Gln, Lys, Arg |
| Ile | Leu, Val, Met, Ala, Phe, Norleucine |
| Leu | Norleucine, Ile, Val, Met, Ala, Phe |
| Lys | Arg, 1,4-Diamino-butyric Acid, Gln, Asn |
| Met | Leu, Phe, Ile |
| Phe | Leu, Val, Ile, Ala, Tyr |
| Pro | Ala |
| Ser | Thr, Ala, Cys |
| Thr | Ser |
| Trp | Tyr, Phe |
| Tyr | Trp, Phe, Thr, Ser |
| Val | Ile, Met, Leu, Phe, Ala, Norleucine |

Ordinarily, amino acid sequence variants of a polypeptide will have an amino acid sequence having at least 60% amino acid sequence identity with the original polypeptide, or at least 65%, or at least 70%, or at least 75% or at least 80% identity, more preferably at least 85% identity, even more preferably at least 90%) identity, and most preferably at least 95% identity, including for example, 80%>, 81%, 82%, 83%, 84%, 85%, 86%, 87%, 88%, 89%, 90%, 91%, 92%, 93%, 94%, 95%, 96%, 97%, 98%, 99%, and 100%. Identity or homology with respect to this sequence is defined herein as the percentage of amino acid residues in the candidate sequence that are identical with the original sequence, after aligning the sequences and introducing gaps, if necessary, to achieve the maximum percent sequence identity, and not considering any conservative substitutions as part of the sequence identity. None of N-terminal, C-terminal, or internal extensions, deletions, or insertions into a CRF binding agent, immunoglobulin or antibody sequence shall be construed as affecting sequence identity or homology.

Amino acid sequence insertions include amino- and/or carboxyl-terminal fusions ranging in length from one residue to polypeptides containing a hundred or more residues, as well as intra-sequence insertions of single or multiple amino acid residues. Examples of terminal insertions include a CRF binding agent with an N- terminal methionyl residue of the CRF binding agent fused to an epitope tag or a salvage receptor binding epitope. Other insertional sequence variants of the CRF binding agent include the fusion to a polypeptide which increases the serum half-life of the CRF binding agent, e.g. at the N-terminus or C-terminus.

Some useful embodiments of the inventive engineered CRF binding agent include a CRF binding agent engineered to effectively remove a proteolytic site by substituting one or more amino acid residues in the site, or by deleting one or more amino acid residues in the proteolytic site. For example, a proteolytic site can be removed by a substitution or deletion of the serine and alanine at amino acid residue positions 234-235 of SEQ ID NO: 1; an amino acid substitution or deletion to effectively remove a proteolytic site having the sequence KSSAG//SEQ ID NO:25 (e.g., at amino acid residue positions 232-236 of SEQ ID NO: 1); or an amino acid substitution or deletion to effectively remove a proteolytic site having the sequence KKSSAGC//SEQ ID NO:26 (e.g., at amino acid residue positions 231-237 of SEQ ID NO: 1). Alternatively, an amino acid substitution can be made to introduce a site of glycosylation, for instance a second N-linked glycosylation site, at or near a sequence corresponding to amino acid residue positions 234-235 of SEQ ID NO: 1.

The term "peptidomimetic" as used herein refers to a protein-like chain designed to mimic a peptide. They typically arise from modification of an existing peptide in order to alter the molecule's properties. For example, they may arise from modifications to change a molecule's stability, biological activity, or bioavailability.

The term "pharmaceutically acceptable salt" refers to a salt, such as used in formulation, which retains the biological effectiveness and properties of the agents and compounds of this and which are is biologically or otherwise desirable. In many cases, the agents and compounds disclosed herein are capable of forming acid and/or base salts by virtue of the presence of charged groups, for example, charged amino and/or carboxyl groups or groups similar thereto. Pharmaceutically acceptable acid addition salts may be prepared from inorganic and organic acids, while pharmaceutically acceptable base addition salts can be prepared from inorganic and organic bases. For example a salt of a protein of interest (such as a CRF binding agent), e.g., a fusion protein or an immunoglobulin, such as an antibody, or any other protein of interest, or a salt of an amino acid, such as, but not limited to, a lysine, histidine, or proline salt, means any salt, or salts, that are known or later discovered to be pharmaceutically acceptable. Some non-limiting examples of pharmaceutically acceptable salts are: acetate salts; trifluoroacetate salts; hydrohalides, such as hydrochloride (e.g., monohydrochloride or dihydrochloride salts) and hydrobromide salts; sulfate salts; citrate salts; maleate salts; tartrate salts; glycolate salts; gluconate salts; succinate salts; mesylate salts; besylate salts; salts of gallic acid esters (gallic acid is also known as 3,4, 5 trihydroxybenzoic acid) such as PentaGalloylGlucose (PGG) and epigallocatechin gallate (EGCG), salts of cholesteryl sulfate, pamoate salts, tannate salts, and oxalate salts. For a review of pharmaceutically acceptable salts (see Berge, et al. (1977) J Pharm Sci, vol. 66, 1-19).

A "plurality" means more than one.

The term "recombinant DNA" refers to nucleic acids and gene products expressed therefrom that have been engineered, created, or modified by man. "Recombinant" polypeptides or proteins are polypeptides or proteins produced by recombinant DNA techniques, for example, from cells transformed by an exogenous DNA construct encoding the desired polypeptide or protein. "Synthetic" polypeptides or proteins are those prepared by chemical synthesis.

The terms "separated", "purified", "isolated", and the like mean that one or more components of a sample contained in a sample-holding vessel are or have been physically removed from, or diluted in the presence of, one or more other sample components present in the vessel. Sample components that may be removed or diluted during a separating or purifying step include, chemical reaction products, non-reacted chemicals, proteins, carbohydrates, lipids, and unbound molecules.

The term "small molecule" refers to a molecule of a size comparable to those organic molecules generally used in pharmaceuticals. The term excludes biological macromolecules (e.g., proteins, nucleic acids, etc.). Preferred small organic molecules range in size up to about 5000 Da, more preferably up to 2000 Da, and most preferably up to about 1000 Da.

The term "species" is used herein in various contexts, e.g., a particular species of targeted drug conjugate. In each such context, the term refers to a population of chemically indistinct compounds of the sort referred in the particular context.

The term "specific" or "specificity" in the context of the interactions of members of a binding pair (e.g., antibody and antigen, receptor and ligand, etc.), refers to the selective, non-random interaction between the members of the binding pair. Such interactions typically depend on the presence of structural, hydrophobic/hydrophilic, and/or electrostatic features that allow appropriate chemical or molecular interactions between the molecules. Thus, one member of a binding pair (e.g., an antibody or receptor) is commonly said to "bind" (or "specifically bind") or be "reactive with" (or "specifically reactive with), or, equivalently, "reactive against" (or "specifically reactive against") the other member of the pair, (e.g., the target antigen, ligand, etc.). "Specifically associate" and "specific association" and the like also refer to a specific, non-random interaction between two molecules, which interaction depends on the presence of structural, hydrophobic/hydrophilic, and/or electrostatic features that allow appropriate chemical or molecular interactions between the molecules.

A "subject" or "patient" refers to an animal in need of treatment that can be effected by compositions disclosed herein. Animals that can be treated include vertebrates, with mammals such as bovine, canine, equine, feline, ovine, porcine, and primate (including humans and non-human primates) animals being particularly preferred examples.

A "therapeutic agent" refers to a drug or compound that is intended to provide a therapeutic effect including, but not limited to, small molecule or biologic chemotherapeutic drugs.

A "therapeutically effective amount" (or "effective amount") refers to an amount of an active ingredient sufficient to effect treatment when administered to a subject in need of such treatment. Accordingly, what constitutes a therapeutically effective amount of a composition may be readily determined by one of ordinary skill in the art. In the context of cancer therapy, a "therapeutically effective amount" is one that produces an objectively measured change in one or more parameters associated with cancer cell survival or metabolism, including an increase or decrease in the expression of one or more genes correlated with the particular cancer, reduction in tumor burden, cancer cell lysis, the detection of one or more cancer cell death markers in a biological sample (e.g., a biopsy and an aliquot of a bodily fluid such as whole blood, plasma, serum, urine, *etc*.), induction of induction apoptosis or other cell death pathways, *etc.* Of course, the therapeutically effective amount will vary depending upon the particular subject and condition being treated, the weight and age of the subject, the severity of the disease condition, the particular compound chosen, the dosing regimen to be followed, timing of administration, the manner of administration and the like, all of which can readily be determined by one of ordinary skill in the art. It will be appreciated that in the context of combination therapy, what constitutes a therapeutically effective amount of a particular active ingredient may differ from what constitutes a therapeutically effective amount of the active ingredient when administered as a monotherapy (*i.e.,* a therapeutic regimen that employs only one chemical entity as the active ingredient).

The compositions described herein are used in therapeutic methods. As used herein, the terms "therapy" and "therapeutic" encompasses the full spectrum of prevention and/or treatments for a disease or disorder or condition. A "therapeutic" agent may act in a manner that is prophylactic or preventive, including those that incorporate procedures designed to target individuals that can be identified as being at risk (*e.g*., via pharmacogenetics); or in a manner that is ameliorative or curative in nature; or may act to slow the rate or extent of the progression of at least one symptom of a disease or disorder being treated; or may act to minimize the time required, the occurrence or extent of any discomfort or pain, or physical limitations associated with recuperation from a disease, disorder, or physical trauma; or may be used as an adjuvant to other therapies and treatments.

The term "treatment" or "treating" means any treatment of a disease or disorder, including preventing or protecting against the disease or disorder (that is, causing the clinical symptoms not to develop); inhibiting the disease or disorder (*i.e.,* arresting, delaying or suppressing the development of clinical symptoms; and/or relieving the disease or disorder *(i.e.,* causing the regression of clinical symptoms). As will be appreciated, it is not always possible to distinguish between "preventing" and "suppressing" a disease or disorder because the ultimate inductive event or events may be unknown or latent. Those "in need of treatment" include those already with the disorder as well as those in which the disorder is to be prevented. Accordingly, the term "prophylaxis" will be understood to constitute a type of "treatment" that encompasses both "preventing" and "suppressing". The term "protection" thus includes "prophylaxis".

The term "therapeutic regimen" means any treatment of a disease, disorder, or condition using one or more appropriate therapeutic agents and/or therapies.

The practice of the techniques described herein may employ, unless otherwise indicated, conventional techniques and descriptions of organic chemistry, polymer technology, molecular biology (including recombinant techniques), cell biology, biochemistry, and sequencing technology, which are within the skill of the art. Such conventional techniques include recombinant DNA techniques, antibody preparation, hybridization and ligation of polynucleotides, and detection of hybridization using a label. Specific illustrations of suitable techniques can be had by reference to the examples herein, although other equivalent procedures now known or later developed can also be used. Such conventional techniques and descriptions can be found in standard laboratory manuals such as Green, et al., Eds. (1999), Genome Analysis: A Laboratory Manual Series (Vols. I-IV); Weiner, Gabriel, Stephens, Eds. (2007), Genetic Variation: A Laboratory Manual; Dieffenbach, Dveksler, Eds. (2003), PCR Primer: A Laboratory Manual; Bowtell and Sambrook (2003), DNA Microarrays: A Molecular Cloning Manual; Mount (2004), Bioinformatics: Sequence and Genome Analysis; Sambrook and Russell (2006), Condensed Protocols from Molecular Cloning: A Laboratory Manual; and Sambrook and Russell (2002), Molecular Cloning: A Laboratory Manual (all from Cold Spring Harbor Laboratory Press); Stryer, L. (1995) Biochemistry (4th Ed.) W.H. Freeman, New York N.Y.; Gait, "Oligonucleotide Synthesis: A Practical Approach" 1984, IRL Press, London; Nelson and Cox (2000), Lehninger, Principles of Biochemistry 3.sup.rd Ed., W. H. Freeman Pub., New York, N.Y.; and Berg et al. (2002) Biochemistry, 5.sup.th Ed., W.H. Freeman Pub., New York, N.Y., all of which are herein incorporated in their entirety by reference for all purposes. Before the present compositions, research tools, and methods are described, it is to be understood that this invention is not limited to the particular methods, compositions, targets and uses described, as such may, of course, vary. It is also to be understood that the terminology used herein is for the purpose of describing particular embodiments only, and is not intended to limit the scope of the present invention that will be limited only by appended claims.

In the following description, numerous specific details are set forth to provide a more thorough understanding of the present invention. However, it will be apparent to one of skill in the art upon reading the specification that the present invention may be practiced without one or more of these specific details; the invention is set out in the claims. In other instances, well-known features and procedures well known to those skilled in the art have not been described in order to avoid obscuring the invention.

Unless defined otherwise, all technical and scientific terms used herein have the same meaning as commonly understood by one of ordinary skill in the art to which this invention belongs.

### Overview of the Invention

This invention concerns engineered corticotropin-releasing factor (CRF) binding agents as set out in the claims, compositions containing such agents, and methods of using such agents to normalize HPA axis hyperactivity by reducing CRF peak bursts. While CRF-BP is present in plasma, due to its low concentration it does not prevent CRF bursts from activating the pituitary. Administration of a polypeptide that binds CRF under physiological conditions in amounts sufficient to neutralize excess CRF activation mediated by CRF peak bursts can decrease such pituitary activation.

Therapeutic concentrations of CRF-BP (or another polypeptide that binds RF under physiological conditions) can be achieved at doses commonly used for other biologicals, such as recombinant antibodies currently in use in clinical settings (114). Since CRF-BP has a very short half-life (115), the invention provides CRF-BP derivatives (and other polypeptides that bind CRF under physiological conditions) with sufficient half-lives to provide a suitable therapeutic effect. Some preferred CRF binding agents of the invention comprise CRF-BP derivatives (and other polypeptides that bind CRF under physiological conditions) fused to Fc (as either N-terminal or C-terminal fusions), thereby allowing the resultant engineered fusion or otherwise-conjugated proteins to bind to the neonatal Fc receptor (FcRn) or Brambell receptor. Such Fc-containing agents use the IgG recycling pathway to extend these engineered molecules' plasma half-lives (116, 117). The experiments and results described below show that representative examples of such molecules are potent CRF-BP-based biologicals that, as with other CRF-binding agents of the invention, can be used to reduce HPA axis hyperactivity in affected patients or subjects.

Patterns of cortisol secretion have been studied in detail in depression and neurodegenerative diseases such as Alzheimer's Disease (AD) and Parkinson's Disease (PD) (see, e.g., 47, 75). Interestingly, HPA axis hyperactivation in these distinct diseases have in common larger, rather than more frequent, bursts of HPA activation (47, 75, 104-107). Importantly, transgenic mice expressing CRF-BP in their serum had significantly reduced LPS-stimulated HPA activation (119). Thus, reducing CRF peak bursts can control HPA axis hyperactivity, potentially leading over time to resetting HPA axis regulation and reduced elevated baseline glucocorticoid levels. As the pituitary portal system is continuous with the general circulation, blood: brain barrier penetration for the CRF-binding agents of the invention is not required. The inventive engineered CRF antagonist agents and engineered CRF-binding agents have a therapeutically-effective long half-life and are restricted to peripheral circulation.

As is known, both CRF and Ucn1 are bound by CRF-BP with sub-nanomolar affinity (K_{D} of 0.2 nM and 0.1 nM, respectively) (120). The circulating serum concentration of CRF-BP in humans has been measured to be 50-200 ng/ml (121) with a plasma half-life of minutes (115). Therefore, this invention uses half-life-extending moieties, e.g., CRF-BP (or a CRF-binding fragment thereof) fused to an IgG Fc region, to extend the plasma half-life of a polypeptide having CRF binding activity under physiological conditions. In some preferred embodiments, the half-life-extending moiety is an IgG Fc region, which engages the neonatal Fc receptor (FcRn), resulting in extended plasma half-life (116, 117). (See, also review: Strohl WR, Fusion Proteins for Half-Life Extension of Biologics as a Strategy to Make Biobetters. BioDrugs 29:215-239 (2015)).

As described below, results indicate that circulating serum concentrations of a preferred CRF-BP-Fc according to the invention administered at a dosage of 1-3 µg/ml can be effective in reducing behavioral responses to stress in mice. In the preferred method of treatment, appropriate for human patients, the pharmaceutical composition for use of the invention achieves a circulating serum concentration of about 1 µg/mL to about 150 µg/mL of the inventive CRF binding agent. A preferred therapeutic dose for humans achieves a circulating serum concentration of about 3 µg/ml or higher (e.g., 3-10 µg/mL up to about 100-150 µg/mL) of a CRF binding agent (e.g., a CRF-BP-derived) for 5-7 days. Due to the high affinity of CRF-BP (see, e.g., 120), this circulating serum concentration can often be lower than the circulating serum concentrations reported for therapeutic antibodies, e.g., bevacizumab (10-30 µg/ml) (114). The desired serum concentration of a circulating CRF-binding polypeptide, as part of a CRF binding agent, can be achieved through a combination of increased half-life and appropriate dosing.

### Polypeptides Having CRF Binding Activity In Vivo

The engineered corticotropin-releasing factor (CRF) binding agents of the disclosure include a polypeptide having CRF binding activity under physiological conditions. Preferred examples of such polypeptides are CRF binding protein (CRF-BP), CRF receptor type 1 (CRFR1), CRF receptor type 2 (CRFR2), or a fragment or derivative of CRF-BP, CRFR1, or CRFR2 that has CRF binding *in vivo.* Particularly preferred examples are engineered fragments, variants, or derivatives of a mammalian (e.g., human, mouse, rat) CRF-BP. Representative examples of mammalian CRF-BP fragments or derivatives are hCRF-BP(25-234), hCRF-BP(25-322), rCRF-BP(25-234), and rCRF-BP(25-322), where "h" and "r" refer to the species of origin of the particular CRF-BP and the parenthetical number refers to the amino acid residues of the parent CRF-BP in the particular fragment. For example, "hCRF-BP(25-234)" refers to an engineered CRF-BF fragment that includes residues 25-234 of human CRF-BP. In accordance with the invention, the polypeptide(s) having CRF binding activity is(are) covalently coupled, optionally via a linker, preferably a peptide linker, to a half-life-extending moiety(ies) as set out in the claims, , preferably in the context of a fusion protein. Particularly preferred CRF binding agents are synthesized as fusion proteins engineered using recombinant techniques and expressed in recombinant host cells. Other particularly preferred CRF binding agent embodiments include those that comprise a first element coupled to a second element, wherein the first element comprises a hCRF-BP(25-234) polypeptide or a hCRF-BP(25-322) polypeptide coupled via a peptide linker to an Fc forming portion of a human immunoglobulin heavy chain and the second element comprises a hCRF-BP(25-234) polypeptide or a hCRF-BP(25-322) polypeptide coupled via a peptide linker to an Fc forming portion of a human immunoglobulin heavy chain.

In preferred embodiments, CRF binding proteins have a Kₐ of greater than or equal to about 10⁴ M⁻¹, greater than or equal to about 10⁶ M⁻¹, greater than or equal to about 10⁷ M⁻¹, greater than or equal to about 10⁸ M⁻¹. Affinities of even greater than about 10⁸ M⁻¹ are suitable, such as affinities equal to or greater than about 10⁹ M⁻¹, about 10¹⁰ M⁻¹, about 10¹¹ M⁻¹, and about 10¹² M⁻¹.

### Half-Life Extension

More than 180 therapeutic proteins and peptides have been approved by the U.S. FDA for a wide variety of indications, ranging from treating various cancers and rheumatoid arthritis to alleviating neuropathic pain to replaing enzymes for lysosomal storage diseases. Many of these proteins and peptides have less than optimal pharmacokinetic properties, often because they are smaller than the kidney filtration cutoff of around 70 kDa and/or are subject to metabolic turnover by peptidases, which significantly limits their *in vivo* half-lives. Moreover, for nearly all of these biologic drugs, dosing is parenteral, with each dose being delivered by either a subcutaneous or intravenous injection. High dosing frequency, a small area under the curve (AUC), and patient inconvenience are limitations of shortacting compounds. Thus, in many cases, modifications have been developed to improve protein or peptide drug pharmacokinetic profiles to decrease protease sensitivity and glomerular filtration by the kidney.

Pharmacokinetics is sometimes described as what the body does to a drug, while pharmacodynamics concerns what a drug does to the body. The pharmacokinetics of proteins and peptides is governed by the parameters of absorption, biodistribution, metabolism, and elimination. They are absorbed generally via the lymphatic system. Biodistribution is generally limited to the extracellular space in the central compartment (e.g., 3-8 L). The volume of distribution is generally less than 15 L. Metabolism occurs through enzymatic cleavage by proteases and peptidases, and proteins and peptides are eliminated from the serum by several different tissue- and receptor-mediated mechanisms. The most common routes of clearance include endocytosis and membrane transportmediated clearance by liver hepatocytes for larger proteins, and glomerular filtration for smaller proteins and peptides.

While not all of the parameters involved in glomerular filtration of peptides and proteins are fully understood, it is clear that size, shape, hydrodynamic radius, and charge all play significant roles. Generally, proteins and peptides smaller than approximately 70 kDa are more likely to be eliminated by kidney filtration than are larger proteins. Additionally, charge plays a significant role in glomerular filtration. Negatively charged peptides or smaller proteins may be eliminated less readily than neutral polypeptides because of repulsion by the negatively charged basement membrane of the kidney. Cationic polypeptides, on the other hand, tend to be removed even more quickly. Thus, two strategies that can be employed to improve the pharmacokinetics of smaller proteins and peptides are increasing size and hydrodynamic radius, and increasing the negative charge of the target protein or peptide. A third strategy, similar to that employed with small molecules, is to increase the level of serum protein binding of the peptide or protein by association with albumin or immunoglobulins.

Another suitable modification to improve peptide or protein pharmacokinetics is via conjugation to either linear or branched-chain monomethoxy poly-ethylene glycol (PEG), which results in increases in the molecular mass and hydrodynamic radius and decreases in glomerular filtration. PEG is a highly flexible, uncharged, mostly nonimmunogenic, hydrophilic, non-biodegradable molecule classified as a GRAS (generally recognized as safe) substance by the FDA. PEGylation has been used widely as a way to lengthen the half-life of proteins, e.g., PegIntron^{®} [PEGylated interferon (IFN)-α_{2b}] and Pegasys^{®} (PEGylated IFN-α₂ₐ) to treat hepatitis B, Neulasta^{®} (a PEG-conjugated granulocyte colony-stimulating factor (G-CSF) to treat chemotherapy-induced neutropenia), and Mycera^{®} (a PEGylated form of epoetin-β). PEGYLATION typically requires chemical conjugation to the desired protein (here, a polypeptide that binds CRF *in vivo*), followed by repurification of the protein-PEG conjugate. (See, also review: Strohl WR, Fusion Proteins for Half-Life Extension of Biologies as a Strategy to Make Biobetters. BioDrugs 29:215-239 (2015); see, also, Chapman, PEGylated antibodies and antibody fragments for improved therapy: a review, Advanced Drug Delivery Reviews 54:531-545 (2002); Jevsevar et al., PEGylation of Antibody Fragments for Half-Life Extension, Chapter 15 in Antibody Methods and Protocols, Gabriele Proetzel and Hilmar Ebersbach (eds.), Methods in Molecular Biology, vol. 901, DOI 10.1007/978-1-61779-931-0_15, published by Springer Science+Business Media, LLC (2012)).

Another approach that can utilized to improve pharmacokinetic parameters of polypeptides includes modification of glycosylation patterns, for example, by adding or removing one or more N-linked or O-linked glycosylation sites of the CRF binding polypeptide of a CRF binding agent of the invention, which can result in reduced clearance and half-life extension (see, e.g., 140, 141). An example of this approach is Aranesp^{®} (darbepoetin-α), a second-generation epoetin with modified glycosylation, which has a threefold longer half-life than epoetin-α. (See, e.g., Reference 115). The skilled artisan can predict N-glycosylation sites and O-glycosylation sites in human proteins, e.g., by publicly available artificial intelligence tools, such as the NetNGlyc 1.0 Server (cbs.dtu.dk/services/NetNGlyc/) and NetOGlyc 4.0 Server (cbs.dtu.dk/services/NetOGlyc/), respectively. An example of a useful CRF-BP polypeptide, having an inserted N-glycosylation site, is a modified hCRF-BP(25-322)(SEQ ID NO:63), which is also modified to remove a proteolytic site:

YLELREAADYDPFLLFSANLKRELAGEQPYRRALRCLDMLSLQGQFTF
TADRPQLHCAAFFISEPEEFITIHYDQVSIDCQGGDFLKVFDGWILKGEKFPSSQDH
PLPSAERYIDFCESGLSRRSIRSSQNVAMIFFRVHEPGNGFTLTIKTDPNLFPCNVIS
QTPNGKFTLVVPHQHRNCSFSIIYPVVIKISDLTLGHVNGLQLKKNCSGCEGIGDFV
ELLGGTGLDPSKMTPLADLCYPFHGPAQMKVGCDNTVVRMVSSGKHVNRVTFE
YRQLEPYELENPNGNSIGEFCLSGL//SEQ ID NO:63.

### Protein Fusion Methods

As discussed above, protein fusion methods can be used improve protein pharmacokinetics. As described herein, adding a half-life extending moiety can improve the pharmacokinetic parameters of the CRF binding polypeptide of a CRF binding agent of the invention, thereby making the CRF binding polypeptide of the CRF binding agent a more efficacious, less frequently dosed, better targeted, and/or a better tolerated drug. The most widely used of these approaches include fusion of the biologically active protein or peptide to human serum albumin (HSA), fusion to the constant fragment (Fc) domain of a human IgG, or fusion to non-structured polypeptides such as XTEN. (See, Haeckel A, Appler F, Ariza de Schellenberger A, Schellenberger E. 2016. XTEN as Biological Alternative to PEGylation Allows Complete Expression of a Protease-Activatable Killin-Based Cytostatic. PLoS One 11:e0157193).

Among the general strategies for prolongation of *in vivo* half-life that can be employed in the context of the disclosure are:
1. Genetic fusion of a polypeptide having CRF binding activity to a naturally long-half-life protein or protein domain, e.g., Fc, transferrin (Tf), or albumin.
2. Genetic fusion of a polypeptide having CRF binding activity to an inert polypeptide, e.g., XTEN, a homo-amino acid polymer (HAP; HAPylation), a proline-alanine-serine polymer (PAS; PASylation), or an elastin-like peptide (ELP; ELPylation).
3. Increasing the hydrodynamic radius of the polypeptide having CRF binding activity by chemical conjugation of the pharmacologically active peptide or protein to repeat chemical moieties, e.g., to PEG (PEGylation) or hyaluronic acid.
4. (a) Significantly increasing the negative charge of the polypeptide having CRF binding activity by polysialylation; or, alternatively, (b) fusing a negatively charged, highly sialylated peptide (e.g., carboxy-terminal peptide (CTP; of chorionic gonadotropin (CG) β-chain)) known to extend the half-life of natural proteins such as human CG β-subunit to a polypeptide having CRF binding activity.
5. Binding non-covalently, via association of a polypeptide having CRF binding activity to normally long-half-life proteins such as HAS, human IgG, or transferrin.
6. Chemical conjugation of a polypeptide having CRF binding activity to a long-half-life protein such as human IgG, an Fc moiety, or HSA.

The half-life of peptides and proteins, including polypeptides having CRF binding activity, in human serum, is dictated by several factors, including size, charge, proteolytic sensitivity, turnover rate of proteins they bind, and other factors. In some cases, the half-life of proteins in human serum roughly correlates with their size. As mentioned previously, peptides and proteins smaller than about 70 kDa can be eliminated via kidney filtration, so they generally possess very short serum half-lives. Larger proteins, however, may persist for several days. Three types of proteins - human IgGs, HSA, and transferrin - persist much longer in human serum than would be predicted just by their sizes. The exaggerated persistence of human IgGs and HSA has been determined to be due to their binding to the neonatal Fc receptor (FcRn)(Roopenian DC, Akilesh S. 2007. FcRn: the neonatal Fc receptor comes of age. Nat Rev Immunol 7:715-725.).

FcRn is a heterodimeric receptor, closely related to major histocompatibility complex (MHC) class I receptors, which is widely expressed in vascular epithelial cells, endothelial cells, intestinal epithelial cells, mammary epithelial cells, placental membranes, monocytes, macrophages, dendritic cells, and polymorphonuclear (PMN) leukocytes. FcRn contains a 45 kDa, transmembrane α-chain with a short cytoplasmic tail, and a ~17 kDa β-2 microglobulin β-chain. While FcRn functions to translocate IgGs from the mother to the fetus, it also has a significant function in both IgG and HSA homeostasis (Roopenian DC, Akilesh S. 2007. FcRn: the neonatal Fc receptor comes of age. Nat Rev Immunol 7:715-725.). Upon pinocytosis of serum proteins by cells of the reticuloendothelial system, human IgG₁, IgG₂, and IgG₄ isotypes and HSA bind FcRn in a pH-dependent manner. As the vesicles are acidified, the IgGs and HSA bind FcRn, which allows them to be translocated back to the cell surface for recycling back into the circulation, while non-FcRn-bound proteins are targeted for lysosomal degradation. Upon exposure to the neutral pH at the cell surface, the IgGs and HSA are released back into the circulation (Roopenian DC, Akilesh S. 2007. FcRn: the neonatal Fc receptor comes of age. Nat Rev Immunol 7:715-725.). This recycling mechanism confers a nominal 14- to 21-day half-life on human IgG₁, IgG₂, and IgG₄, and a ~19-day half-life on HAS. Human IgA, IgM, IgD, and IgE do not bind FcRn and do not possess an extended half-life, and human IgG₃ has an altered residue in the FcRnbinding domain that decreases its ability to bind FcRn, resulting in a diminished half-life of about 5-7.5 days (Roopenian DC, Akilesh S. 2007. FcRn: the neonatal Fc receptor comes of age. Nat Rev Immunol 7:715-725.). The IgGs bind to FcRn at a different epitope than HSA, so the molecules do not compete. It has been calculated that for every IgG molecule recycled by FcRn, approximately 700 molecules of HSA are recycled. Thus, FcRn plays a significant role in the homeostasis of both human IgGs and HSA, the most abundant proteins in human serum (Roopenian DC, Akilesh S. 2007. FcRn: the neonatal Fc receptor comes of age. Nat Rev Immunol 7:715-725.).

### Fc Fusions

As described above, human IgG isotypes 1, 2, and 4 bind to FcRn in a pH-dependent manner to effect their recycling by epithelial cells (117). This binding occurs via specific residues in the Fc of the antibody, giving these IgG isotypes a nominal 2- to 3-week half-life in human serum. Examples of using IgG Fc as a fusion partner to significantly increase the half-life of a therapeutic peptide or protein include Enbrel^{®} (etanercept), the first Fc fusion protein to be marketed (approved in 1998). Also, an IgG2 Fc was selected for the anti-CD3 antibody visilizumab (Nuvion) on the basis of a side-by-side comparison of all four isotypes (122). For CDP571, a humanized monoclonal antibody to TNF-alpha, *in vivo* comparison of IgG1 and IgG4 resulted in selection of the IgG4 isotype (123). Further optimizations include stabilized or non-stabilized IgG4 to further reduce effector function, e.g., as used in the anti-human CD4 antibody clenoliximab (117, 124, 125). Today, at least 11 Fc fusion proteins have been approved for marketing by the FDA. For eight of these, the Fc moiety was fused to a protein or peptide to enhance its pharmacokinetics.

In some embodiments, a monomeric Fc fusion molecule is generated by linking a polypeptide having CRF binding activity to only one arm of a dimeric Fc. The resulting complex is "monovalent" for the pharmacologically active "head" moiety but retains the normal bivalent Fc structure and function. Examples of monomeric protein-Fc technology used to develop other extended-half-life biologics include IFN-0-Fc, IFN-α-Fc, epoetin-Fc, B-domain-deleted Factor VIII-Fc, and Factor IX-Fc. Two of these monomeric Fc fusion proteins have been approved by the FDA: Alprolix^{®} (eftrenonacog-α; monomeric Factor IX-Fc of approximately 98 kDa) and Eloctate^{®} (efraloctocog-α; monomeric B-domain-deleted Factor VIII-Fc of approximately 220 kDa). In clinical trials, Factor IX-Fc (Alprolix^{®}) was shown to have a terminal half-life in the range of 57-83 h, about threefold longer than the -18 h half-life obtained with other formulations of Factor IX alone. For Factor VIII (Eloctate^{®}), the Fc fusion improved the half-life by about 50 %, from a range of ~12 h for historical Factor VIII preparations to about 19 h for Factor VIII-Fc.

Other constructs may have naturalistic extended hinges, such as by including an extra CH1 motif (126) or an extra-long hinge derived from naturally-occurring Ig molecules (127, 128).

In the context of the present disclosure, a polypeptide having CRF binding activity under physiological conditions, e.g., CRF-BP or a CRF-binding fragment thereof, can be fused to an IgG Fc domain using genetic engineering or chemical conjugation (see, e.g., Fig. 1A or 1C, respectively).

### Albumin Fusions

The 66.5 kDa protein HSA, similar to human IgGs, has a long average half-life of about 19-day range. At a concentration of ~50 mg/mL (~600 µM), HSA is the most abundant protein in human plasma, where it has several functions, including maintenance of plasma pH, metabolite and fatty acid transport, and a role in maintaining blood pressure. HSA, which is at the upper size limit for glomerular filtration, is strongly anionic, which further helps retard its filtration via the kidney. Like IgGs, HSA also binds FcRn in a pH-dependent manner, albeit at a site different from, and via a mechanism distinct from, that of IgG, and is recycled similarly to IgGs, resulting in its extended half-life.

Fusing peptides or proteins with inherently short half-lives to HSA to prolong serum half-life has been broadly investigated since the early 1990s. Since then, dozens of different peptides and small proteins have been fused to HSA. EMA- or FDA-approved HSA-peptide or protein fusion products include Tanzeum^{®} (Eperzan^{®} in the EU), a DPP-4-resistant GLP-1-HSA fusion protein that improves the half-life of GLP-1 from 1-2 min for native GLP-1 to 4-7 days, allowing for once weekly dosing. Another example is Idelvion^{®} (albutrepenonacog alfa), a fusion protein linking recombinant coagulation Factor IX with recombinant albumin that provides factor IX therapy with up to 14-day dosing.

Modified versions of recombinant HSA with improved FcRn binding are also known and may be used in the practice of the invention to provide even longer half-life properties. For example, a K573P mutant of HSA has been found to possess 12-fold greater affinity for FcRn and to confer a longer half-life on HSA than wild type in both mice and cynomolgus monkeys.

An alternative protein-based half-life extender is albumin or an albumin derivative. Alternative albumin designs include N-terminal fusions with albumin or a minimal albumin-binding domain (ABD) (129-133).

### Transferrin Fusions

Other half-life extenders include human high affinity monoclonal antibodies and various fusions with transferrin (129, 131, 134, 135, 136, 137). Transferrin (Tf) is a highly abundant serum glycoprotein, found in serum at 3-4 mg/mL. It binds iron tightly but reversibly and functions to carry iron to tissues. Transferrin has 679 amino acid residues, is about 80 kDa in size, and possesses two high-affinity Fe³⁺-binding sites, one in the N-terminal domain and the other in the C-terminal domain. Human transferrin has a half-life reported to be 7-12 days. The aglycosylated form of human transferrin, which makes up about 2-8 % of the total transferrin pool, has a slightly longer half-life of 14-17 days. The extended persistence of transferrin in human serum is due to a clathrin-dependent transferrin receptor-mediated mechanism, which recycles transferrin receptor-bound transferrin back into the circulation.

Fusions of polypeptides to the N- and C- termini human transferrin have been made, as well as to the centrally located hinge region that links the two major transferrin lobes together. The N-terminus of transferrin is free and can be fused directly. The C-terminus is more buried and is constrained by a nearby disulfide bond, so flexible linkers are typically used to fuse proteins to its C-terminus.

### CTP Fusions

Fusions of desired proteins to a carboxy terminal peptide (CTP) can also extend serum half-life of polypeptides having CRF binding activity. Thyroid-stimulating hormone (TSH) and the three gonadotropins, follicle-stimulating hormone (FSH), luteinizing hormone (LH), and CG, are heterodimeric glycohormones that have a common α-subunit and unique β-subunits that confer their different activities. The half-life of human CG (HCG) is significantly longer than that of its counterparts, FSH, LH, and TSH. This difference stems from the HCG β-subunit (HCG-β), which possesses a ~31-amino-acid-residue CTP having the sequence FQSSSS*KAPPPS*LPSPS*RLPGPS*DTPILPQ, which possesses four *O*-glycosylation sites (denoted by S*) terminating with a sialic acid residue. CTP naturally extends the protein's half-life in human serum because the negatively charged, heavily sialylated CTP impairs renal clearance. A long-acting FSH-CTP fusion, corifollitropin-α (Org 36286), used to treat infertility in women, consistently demonstrated about a twofold improvement in half-life over recombinant FSH whether it was dosed subcutaneously or intravenously. Org 36286 has been approved by the EMA as the long-acting fertility drug Elonva^{®} (Merck and Co.).

Genetic fusions of CTP to proteins such as polypeptides having CRF binding activity *in vivo* are preferably expressed in Chinese hamster ovary (CHO) or other mammalian cell systems so that the CTP portions of the resultant recombinant proteins are O-glycosylated. The resulting products have improved half-lives and are negatively charged (because of the multiple sialylations) are thus less susceptible to renal clearance.

### Other Approaches

Other moieties that can be used to extend the half-life of a CRF binding polypeptide include those that do not involve a fusion protein modification. For example, half-life can be extended by PEGylation (134).

While PEG can be used to increase the hydrodynamic radius of proteins, including polypeptides having CRF binding activity, to increase their half-lives in human serum, other strategies can also be used as an alternative (or in addition) to chemical conjugation to PEG or other non-biologic polymers. Common to these other strategies is the fusion of inert peptide repeat polymers to a polypeptide having CRF binding activity. This approach yields four immediate advantages over PEGylation: (1) the cost of the PEG moiety and the time and process cost for the chemistry to couple it to the protein are eliminated; (2) the entire construct can be made as a single expression product; (3) only a single round of purification is required, rather than protein purification followed by conjugation and then repurification post-PEGylation; and (4) the fusion proteins, while largely resistant to extracellular proteases, are nonetheless slowly degraded by natural processes *in vivo.* Examples of such inert peptide repeat polymers include XTEN polymers, ELPylation, and PASylation.

XTEN sequences are amino acid repeating polymers that contain the amino acid residues A, E, G, P, S, and T. (See, Haeckel A, Appler F, Ariza de Schellenberger A, Schellenberger E. 2016. XTEN as Biological Alternative to PEGylation Allows Complete Expression of a Protease-Activatable Killin-Based Cytostatic. PLoS One 11:e0157193). XTEN lengths of 288 (~32 kDa) to 1008 (~111 kDa) residues have been shown to extend peptide and protein half-lives from about 12- to 125-fold. XTEN sequences include inexact repeats of GSEGEG/SEQ ID NO:9 and similar sequences to much more highly randomized sequences containing longer inexact repeats of residues similar to AESPGPGTSPSGESSTAPGT/SEQ ID NO:10. An optimized glucagon-XTEN compound designed to treat nocturnal hypoglycemia contained 144 amino acid residues fused to the C-terminus of glucagon.

ELPylation uses ELPs, which are repeating peptide units containing sequences commonly found in elastin. (See, Yeboah A, Cohen RI, Rabolli C, Yarmush ML, Berthiaume F. 2016. Elastin-like polypeptides: A strategic fusion partner for biologics. Biotechnol Bioeng 113:1617-1627). The ELP sequence contains repeats of V-P-G-x-G, where x is any amino acid except proline. This sequence can be degraded over time *in vivo* by human elastases, making ELP polymers biologically degradable. In ELPylation, ELP repeat sequences are genetically fused to a target protein, such as a polypeptide that binds CRF *in vivo,* to enhance a thermally responsive phase transition. At higher temperatures, above the so-called transition temperature, ELPs aggregate and precipitate from solution. When the temperature is decreased below the transition point, they become fully soluble again. This property facilitates purification. The fusion of ELP sequences also enhances the half-life of proteins by increasing their hydrodynamic radius, thus reducing renal clearance.

PASylation is another approach that also uses polypeptide repeat sequences. (See, Ahmadpour S, Hosseinimehr SJ. 2017. PASylation as a Powerful Technology for Improving the Pharmacokinetic Properties of Biopharmaceuticals. Curr Drug Deliv doi:10.2174/1567201814666171120122352). Here, polymers are generated using three repeating amino acids, proline, alanine, and serine (i.e., PAS). PAS polymers of 100-200 repeats in length improve the pharmacokinetics of small proteins by 3.5- to 10-fold over non-PASylated proteins.

Another approach that utilizes inert polypeptide chains to extend the half-life of CRF-binding polypeptides is "HAPylation", which uses inert repeat sequences similar or identical to (Gly₄Ser)*ₙ*. Such sequences can also been used as linker sequences to link subunits, single chains, and peptides together.

As CRF-BP affinity for CRF is extremely high, antibodies of comparable affinities to a self-antigen like CRF can be generated using affinity maturation.

### Vectors, Host Cells, and Recombinant Methods

For recombinant production of fusion protein of the invention, a nucleic acid encoding it is isolated and inserted into a replicable vector for further cloning (amplification of the DNA) or for expression. DNA encoding the fustion protein is readily isolated and sequenced using conventional procedures (e.g., by using oligonucleotide probes that are capable of binding specifically to genes encoding the heavy and light chains of the antibody). Any suitable vector now known of later developed can be used, and will depend in part on the host cell to be used. Generally, preferred host cells are of either prokaryotic or eukaryotic (generally mammalian) origin.

Polynucleotide sequences encoding polypeptide components of the fusion proteins of the invention can be obtained using standard recombinant techniques. Alternatively, polynucleotides can be synthesized using nucleotide synthesizer or PCR techniques. Once obtained, sequences encoding a desired fusion polypeptide are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides.

### Generating Fusion Proteins Using Prokaryotic Host Cells

### i. Vector Construction; Host Cells

For prokaryotic expression, the vector components generally include, but are not limited to: an origin of replication; a selection marker gene; a promoter; a ribosome binding site (RBS); the heterologous nucleic acid insert; and a transcription termination sequence. If the recombinant protein is to be secreted, the expression construct in the vector should also encode a signal peptide appropriate for the particular host cell.

Prokaryotic host cells suitable for expressing fusion proteins of the invention include Archaebacteria and Eubacteria, such as Gram-negative or Gram-positive organisms. Examples of useful bacteria include Escherichia (e.g., E. coli), Bacilli (e.g., B. subtilis), Enterobacteria, Pseudomonas species (e.g., P. aeruginosa), Salmonella typhimurium, Serratia marcescans, Klebsiella, Proteus, Shigella, Rhizobia, Vitreoscilla, or Paracoccus. Preferably, the host cell should produce minimal amounts of proteolytic enzymes, and additional protease inhibitors may desirably be incorporated in the cell culture.

### ii. Protein Production

Host cells are transformed with the above-described expression vectors and cultured in conventional nutrient media modified as appropriate for inducing promoters, selecting transformants, or amplifying the genes encoding the desired sequences. Prokaryotic cells used to produce the polypeptides of the invention are grown in media known in the art and suitable for culture of the selected host cells. The media may also contain a selection agent, chosen based on the construction of the expression vector, to selectively permit growth of prokaryotic cells containing the expression vector. Any necessary supplements besides carbon, nitrogen, and inorganic phosphate sources may also be included at appropriate concentrations introduced alone or as a mixture with another supplement or medium such as a complex nitrogen source. Optionally, the culture medium may contain one or more reducing agents. The prokaryotic host cells are cultured at suitable temperatures and pH. If an inducible promoter is used in the expression vector of the invention, protein expression is induced under conditions suitable for the activation of the promoter.

The expressed fusion proteins of the present invention may be secreted into and recovered from the periplasm of the host cells. Protein recovery typically involves disrupting the host cells, generally by such means as osmotic shock, sonication, or lysis. Once cells are disrupted, cell debris or whole cells may be removed by centrifugation or filtration. The proteins may be further purified, for example, by affinity chromatography. Alternatively, proteins can be secreted into the culture medium, from which they can then be isolated. Cells may be removed from the culture and the culture supernatant filtered and concentrated for subsequent protein purification.

### iii. Protein Purification

Standard protein purification methods known in the art can be employed to purify fusion proteins of the invention. Suitable representative purification procedures include fractionation on immunoaffinity or ion-exchange columns, ethanol precipitation, reverse phase HPLC, chromatography on silica or on a cation-exchange resin such as DEAE, chromatofocusing, SDS-PAGE, ammonium sulfate precipitation, and gel filtration using, for example, Sephadex G-75. In some embodiments, Protein A immobilized on a solid phase is used for immunoaffinity purification of fusion proteins that include an Fc region; after the fusion proteins bind to the immobilized Protein A, they are eluted from the solid phase.

### Generating Fusion Proteins Using Eukaryotic Host Cells

### i. Vector Construction: Host Cells

For eukaryotic expression, the vector components generally include, but are not limited to one or more of the following: a signal sequence; an origin of replication; one or more marker genes; an enhancer element; a promoter; and a transcription termination sequence. DNA encoding the fusion protein is ligated in reading frame into the vector.

Suitable host cells for cloning or expressing DNA encoding a desired fusion protein include higher eukaryote cells, including vertebrate and invertebrate host cells. Examples of useful mammalian host cell lines are COS-7 (ATCC CRL 1651), human embryonic kidney cell lines, baby hamster kidney cells (e.g., BHK, ATCC CCL 10), and Chinese hamster ovary cells (CHO). Invertebrate cell lines, such as various insect cell lines, as well as yeast and other fungal, can also be used.

### ii. Protein Production

Eukaryotic host cells transformed with the above-described expression or cloning vectors for fusion protein production are cultured in conventional nutrient media modified as appropriate for inducing promoters, selecting transformants, and/or amplifying the genes encoding the desired sequences. Such media may be supplemented as necessary with hormones and/or other growth factors, buffers, nucleotides, antibiotics, trace elements (i.e., inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those known to be useful in the context of the particular host cell, as will be apparent to the ordinarily skilled artisan.

### iii. Protein Purification

When using recombinant techniques, a fusion protein as described herein can be produced intracellularly or be directly secreted into the culture medium. If produced intracellularly, as a first step, particulate debris is first removed, for example, by centrifugation or ultrafiltration. If secreted into the medium, the medium is generally first concentrated using a commercially available protein concentration filter, for example, an Amicon or Millipore Pellicon ultrafiltration unit. A protease inhibitor may be included in any of the foregoing steps to inhibit proteolysis, and antibiotics may be included to prevent the growth of adventitious contaminants.

The resulting fusion protein-containing composition can be further purified using, for example, hydroxylapatite chromatography, gel electrophoresis, dialysis, and affinity chromatography, with Protein A affinity chromatography being a preferred purification method when the half-life extending moiety is an Fc domain.

After purification, the fusion protein can be formulated into a suitable pharmaceutical composition.

### Formulations

Therapeutic formulations comprising an engineered corticotropin-releasing factor (CRF) binding agent of the invention are prepared for storage by mixing the agent having the desired degree of purity with optional physiologically or pharmaceutically acceptable carriers, excipients, or stabilizers (Remington: The Science and Practice of Pharmacy 20th edition (2000)) in the form of aqueous solutions or lyophilized or other dried formulations. Acceptable carriers, excipients, and stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™} or PLURONICS^{™}.

The active ingredients may also be entrapped in microcapsules prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are described in Remington: The Science and Practice of Pharmacy 20th edition (2000).

The compositions of the invention that are intended for *in vivo* administration must be sterile. This is readily accomplished by filtration through sterile filtration membranes.

The invention also includes sustained-release preparations of the compositions of the invention. Suitable examples of sustained-release preparations include semipermeable matrices of solid hydrophobic polymers containing the immunoglobulin of the invention, which matrices are in the form of shaped articles, e.g., films, or microcapsules. Examples of sustained-release matrices include polyesters, hydrogels, poly(vinylalcohol)), polylactides, and copolymers. While polymers such as ethylene-vinyl acetate and lactic acid-glycolic acid enable release of molecules for over 100 days, certain hydrogels release proteins for shorter time periods. When encapsulated in such formulations, the agents of the invention remain in the body for a long time. If desired, such preparations can be stabilized to preserve activity of the active ingredients, for example, by modifying sulfhydryl residues to limit intermolecular S--S bond formation, controlling moisture content, using appropriate additives, and developing specific polymer matrix compositions.

Optionally, but preferably, the composition contains a pharmaceutically acceptable salt, preferably sodium chloride, and preferably at about physiological concentrations. Optionally, the composition of the invention can contain a pharmaceutically acceptable preservative. The preservative concentration may be in the range of from about 0.1 to about 2.0%, typically v/v. Suitable preservatives include those known in the pharmaceutical arts, for example, benzyl alcohol, phenol, m-cresol, methylparaben, and propylparaben. Optionally, the compositions of the invention can also include a pharmaceutically acceptable surfactant, preferably at a concentration of about 0.005 to about 0.02%.

### Dosages; Administration

The engineered CRF binding agents of the invention compositions are formulated, dosed, and administered in a fashion consistent with good medical or veterinary practice, as the case may be. Factors for consideration in this context include the particular disorder being treated, the particular mammal being treated, the clinical condition of the individual subject, the cause of the disorder, the site of delivery of the agent, the method of administration, the scheduling of administration, and other factors known to medical practitioners. The "therapeutically effective amount" of the CRF binding agent to be administered will be governed by such considerations, and is the minimum amount necessary to prevent, ameliorate, or treat the particular condition characterized by HPA axis hyperactivity that the subject presents. Such disease, disorders, and conditions include anxiety, depression, Alzheimer's and Parkinson's diseases, obesity, metabolic syndrome, osteoporosis, cardiovascular disease, alcohol and drug abuse, IBD and IBS, as well as other conditions characterized by HPA axis hyperactivity such as cardiovascular disease, stress-induced obesity, metabolic syndrome, type II diabetes, osteoporosis, inflammatory bowel disease, alcohol and drug abuse, premature aging, and early death.

A composition of the invention may be administered to a subject in accordance with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes.

### Articles of Manufacture and Kits

Another aspect of the disclosure concerns articles of manufacture containing materials useful for the treatment of conditions characterized by HPA axis hyperactivity. Such articles comprise a container and a label or package insert on or associated with the container. Suitable containers include, for example, bottles, vials, syringes, etc. The containers may be formed from a variety of materials such as glass or plastic. The container holds a composition that includes an engineered CRF binding agent of the invention that is effective for treating the condition and may have a sterile access port (for example the container may be an intravenous solution bag or a vial having a stopper pierceable by a hypodermic injection needle). The label or package insert indicates that the composition is used for treating the particular condition. The label or package insert will further comprise instructions for administering the composition to the subject. As will be understood, a "package insert" refers to instructions customarily included in commercial packages of therapeutic products that contain information about the indications, usage, dosage, administration, contraindications, and/or warnings concerning the use of such therapeutic products.

Additionally, the article of manufacture may further comprise a second container comprising a pharmaceutically-acceptable buffer, such as bacteriostatic water for injection (BWH), phosphate-buffered saline, Ringer's solution, and dextrose solution. It may further include other materials from a commercial and user standpoint, including other buffers, diluents, filters, needles, and syringes.

Disclosed herein is an engineered corticotropin-releasing factor (CRF) binding agent, comprising a polypeptide having CRF-specific binding activity under physiological conditions, coupled to one or more half-life-extending moieties, or a pharmaceutically acceptable salt of the corticotropin-releasing factor binding agent.

The polypeptide disclosed herein may be selected from the group consisting of CRF binding protein (CRF-BP), CRF receptor type 1 (CRFR1), CRF receptor type 2 (CRFR2), and a CRF-specific binding fragment, sequence variant, modification, or derivative of CRF-BP, CRFR1, or CRFR2 that has CRF-specific binding activity under physiological conditions.

The polypeptide disclosed herein may be engineered to remove a proteolytic site by substituting one or more amino acid residues in the proteolytic site, or by deleting one or more amino acid residues in the proteolytic site.

The one or more amino acid residues substituted or deleted may be in a proteolytic site having the amino acid sequence of SEQ ID NO:25 or SEQ ID NO:26.

The polypeptide disclosed herein may be a derivative of a mammalian CRF-BP, optionally a human or murine CRF-BP derivative selected from the group of hCRF-BP(25-234)(SEQ ID NO: 12), hCRF-BP(25-322)(SEQ ID NO: 13), hCRF-BP(25-322)(SEQ ID NO:63); rCRF-BP(25-234)(SEQ ID NO: 14), and rCRF-BP(25-322)(SEQ ID NO: 15), or a CRF-specific binding fragment, sequence variant, or derivative of any of these members.

The half-life-extending moiety(ies) disclosed herein is(are) independently selected from the group consisting of an Fc forming portion of a mammalian immunoglobulin heavy chain, an Fc region of an antibody (optionally an Fc region of a human antibody), albumin, transferrin, transthyretin, and polyethylene glycol (PEG); or one or more engineered glycosylating moieties.

The polypeptide and half-life-extending moiety(ies) disclosed herein may be covalently coupled, optionally via a linker, optionally a peptidyl linker.

The engineered CRF binding agent disclosed herein may comprise a hCRF-BP(25-234)(SEQ ID NO:12) polypeptide, a hCRF-BP(25-322)(SEQ ID NO: 13) polypeptide, or a modified hCRF-BP(25-322)(SEQ ID NO:63) polypeptide, coupled via a peptidyl linker to an Fc forming portion of a human immunoglobulin heavy chain.

The engineered CRF binding agent disclosed herein may comprise a first element coupled to a second element, wherein the first element comprises a hCRF-BP(25-234)(SEQ ID NO:12) polypeptide, a hCRF-BP(25-322)(SEQ ID NO: 13) polypeptide, or a modified hCRF-BP(25-322)(SEQ ID NO: 63) polypeptide, coupled via a peptide linker to an Fc forming portion of a human immunoglobulin heavy chain; and the second element comprises a hCRF-BP(25-234)(SEQ ID NO: 12) polypeptide, a hCRF-BP(25-322)(SEQ ID NO: 13) polypeptide, or a modified hCRF-BP(25-322)(SEQ ID NO:63) polypeptide, coupled via a peptide linker to an Fc forming portion of a human immunoglobulin heavy chain.

The polypeptide having CRF-specific binding activity disclosed herein may have been engineered to encode at least one site for N-linked glycosylation and/or O-linked glycosylation.

Also disclosed herein is a pharmaceutical composition, comprising the engineered CRF binding agent described herein, and a pharmaceutically acceptable carrier, excipient, or stabilizer.

Also disclosed is the engineered CRF binding agent as described herein, for use in treating a disease or disorder.

The disease or disorder may be in a human.

The disease or disorder may be characterized by HPA axis hyperactivity.

The disease or disorder may be selected from anxiety, depression, Alzheimer's disease, Parkinson's disease, obesity, metabolic syndrome, type 2 diabetes, osteoporosis, cardiovascular disease, alcohol or drug abuse, inflammatory bowel disease (IBD), and irritable bowel syndrome (IBS).

Disclosed herein is an engineered nucleic acid molecule, comprising an expression construct that codes for the expression of a fusion protein that comprises (i) a polypeptide having CRF-specific binding activity and (ii) an Fc forming portion of a mammalian immunoglobulin heavy chain, an albumin, a transthyretin, or a transferrin.

Also disclosed is an engineered nucleic acid molecule, comprising an expression construct that codes for the expression of a polypeptide having CRF binding activity, which polypeptide has been engineered to encode at least one site for N-linked glycosylation and/or O-linked glycosylation.

Provided herein is a recombinant host cell, comprising the engineered nucleic acid molecule as described herein.

The CRF binding agent disclosed herein may bind CRF with high affinity or very high affinity.

Disclosed herein is a therapeutic dose of the engineered CRF binding agent described herein, wherein the CRF binding agent is delivered to a subject in need of treatment to achieve a circulating serum concentration of the CRF binding agent in the subject of about 1 µg/mL to about 150 µg/mL.

Also disclosed is an engineered corticotropin-releasing factor (CRF) antagonist agent, comprising a polypeptide having CRF antagonist activity under physiological conditions, coupled to one or more half-life-extending moieties as set out in the claims, or a pharmaceutically acceptable salt of the corticotropin-releasing factor antagonist agent. Also disclosed but not claimed is an engineered corticotropin-releasing factor (CRF) antagonist agent, comprising a small molecule antagonist having CRF antagonist activity under physiological conditions, coupled to one or more half-life-extending moieties, or a pharmaceutically acceptable salt of the corticotropin-releasing factor antagonist agent.

The polypeptide (or small molecule antagonist which is disclosed but not claimed) having CRF antagonist activity may have CRF1-selective antagonist activity.

The polypeptide (or small molecule antagonist which is disclosed but not claimed) having CRF antagonist activity may be selected from the molecules listed in Table 2.

The foregoing detailed description is considered to be sufficient to enable one skilled in the art to practice the invention. All of the compositions and methods described and claimed herein can be made and executed without undue experimentation in light of the present disclosure. While the compositions and compositions for use of this invention have been described in terms of preferred embodiments, it will be apparent to those of skill in the art that variations may be applied to the compositions and methods. All such similar substitutes and modifications apparent to those skilled in the art are deemed to be within the scope of the invention as defined by the appended claims.

All patents, patent applications, and publications mentioned in the specification are indicative of the levels of those of ordinary skill in the art to which the invention pertains.

The invention illustratively described herein suitably may be practiced in the absence of any element(s) not specifically disclosed herein. Thus, for example, in each instance herein any of the terms "comprising", "consisting essentially of", and "consisting of" may be replaced with either of the other two terms. Also, it must be noted that as used herein and in the appended claims, the singular forms "a," "and," and "the" include plural referents unless the context clearly dictates otherwise. Thus, for example, reference to "a composition" refers to one or mixtures of such compositions, and reference to "an assay" includes reference to equivalent steps and methods known to those skilled in the art, and so forth.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes both of the limits, ranges excluding either of those included limits are also included in the invention.

The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The following working examples are illustrative and not to be construed in any way as limiting the scope of the invention.

### EXAMPLES

The following Examples are provided for illustrative purposes only and not to limit the scope of the invention in any way. Indeed, various modifications of the invention in addition to those shown and described herein will become apparent to those skilled in the art from the foregoing description and fall within the scope of the appended claims.

### EXAMPLE 1

Introduction. As described above, increased peak bursts of HPA axis activation characterize pathologic HPA hyperactivity in depression and neurodegeneration. Thus, such patterns of secretion can be mitigated by therapeutic interventions that increase CRF BP binding capacity. Additionally, the high affinity (K_{D} of 0.1-0.2 nM) (120) and low concentrations of CRF-BP (50-200 ng/ml) (121) support the instant therapeutic strategy of increasing CRF-BP binding capacity through CRF-BP-based biologicals with extended half-life. To this end, this invention provides CRF-BP derivatives fused to immunoglobulin (IgG) Fc portions or innovative fusions with albumin (e.g., human serum albumin (HSA), among other half-life extending moieties. Such a strategy allow protein to engage the neonatal Fc receptor (FcRn) or Brambell receptor and to use the IgG recycling pathway to extend the plasma half-life of the conjugate (see, e.g., 116, 117). Normalization of HPA hyperactivity over time resets HPA axis dysregulation and reduce the detrimental effects of chronically elevated glucocorticoid levels on the central nervous system, which itself contributes to HPA hyperactivity, and peripheral organs. As a result, the invention provides potent biological(s) that are useful in treating various conditions with hyperactive HPA, including anxiety and depression, metabolic syndrome, substance abuse, Alzheimer's and Parkinson's diseases, and IBD.

Thus, in one aspect the invention provides CRF-BP derivatives conj ugated to Fc or albumin moieties via protein fusion techniques or chemical conjugation, be it directly or via a chemical linker, e.g., a peptidyl or non-peptidyl linker. These Fc- or albumin-conjugated CRF-BP derivatives exhibit extended half-lives and effectiveness in preventing excessive activation of the HPA activation *in vivo.* Since the C terminus of CRF-BP in certain settings can be proteolytically cleaved, in the context of the invention a CRF-BP derivative includes mature, full-length CRF-BP as well as CRF-BP polypeptide derivatives that have been truncated and/or possess one or more amino acid substitutions or deletions to remove a proteolytic site. In some preferred embodiments, the CRF-BP derivative portion of the conjugate is based on the major proteolytic fragment of CRF-BP, which has the same high affinity for CRF as full-length CRF-BP. An example of a useful CRF-BP polypeptide, modified to remove a proteolytic site, is a modified hCRF-BP(25-322)(SEQ ID NO: 63), which also includes an inserted N-glycosylation site.

### Experimental design.

Divalent CRF-BP + Fc fusion proteins and monovalent albumin fusion proteins are prepared and tested.

Fc fusions. There are several examples of established Fc fusion proteins that serve as benchmarks. Among them are three well-characterized examples: Fc-Factor VIII (FVIII-Fc), erythropoietin-Fc fusion (Fc-EPO), and the soluble tumor necrosis factor-alpha (TNF-α) receptor-Fc fusion, etanercept, the first FDA-approved therapeutic Fc fusion protein with a half-life of roughly 70 hours, which allows for weekly or twice weekly dosing (142). Naturally occurring FVIII has a half-life of approximately 12 hours, requiring 3-4 intravenous infusions weekly, which reduces adherence (143). FVIII-Fc have half-lives only 1.5-2.5 times longer than FVIII, but they allow for weekly administration (143, 144). Among them, Eloctate^{®}, the FVIII-Fc recently approved by the FDA, has a half-life of 19 hours, only about 50% longer than FVIII preparations (145). E xtended half-life of EPO has been accomplished by changes in glycosylation, which led to commercial versions of EPO. Half-lives of Fc-EPO constructs currently under development are 8-30 hours (146-148).

Studies showed a half-life for an initial recombinant fusion CRF-BP-Fc of approximately 25 hours for an IgG1-based CRFBP fusion (SEQ ID NO: 16) (see, Fig. 2A), which compared well with most of the previously reported Fc-fusions reviewed above. Additionally, a single administration of this CRF-BP-Fc construct reduced freezing in response to a mild footshock in a mouse model of stress (Fig. 2B) and delayed the increased serum glucocorticoid levels induced by repeated stress (Fig. 2), which indicative of a chronic stress state (149, 150). These results support the use of CRF-BP fusion proteins as biologics for treatment of HPA hyperactivity and validate the mouse as an appropriate model for validation of such molecules, consistent with previous studies on the effects of CRF-BP overexpression in mice (119). Results in Figure 7 show that the CRFBP-Fc fusion (SEQ ID NO:23) did not interfere with binding to CRF.

Albumin Fusions. Fusions with albumin have also emerged as an effective strategy to improve pharmacokinetic profiles (129, 130, 131, 137). (See, also, Mueller et al., Improved Pharmacokinetics of Recombinant Bispecific Antibody Molecules by Fusion to Human Serum Albumin, J. Biol. Chem. 282(17): 12650-12660 (2007); Ru et al., Expression and bioactivity of recombinant human serum albumin and dTMP fusion proteins in CHO cells, Appl. Microbiol. Biotechnol. DOI 10.1007/s00253-016-7447-2 (2016); Carter et al., Fusion partners can increase the expression of recombinant interleukins via transient transfection in 2936E cells, Protein Science 19:357-362 (2010); Miyakawa et al., Prolonged Circulation Half-life of Interferon γ Activity by Gene Delivery of Interferon γ-Serum Albumin Fusion Protein in Mice, J. Pharm. Sci. 100:2350-2357 (2011); Sheffield et al., Effects of genetic fusion of factor IX to albumin on in vivo clearance in mice and rabbits, Brit. J. Haematol. 126: 565-573 (2004); Strohl WR, Fusion Proteins for Half-Life Extension of Biologics as a Strategy to Make Biobetters. BioDrugs 29:215-239 (2015)). GlaxoSmithKline developed an albumin fusion of GLP-1 (albiglutide), approved in 2014 by the FDA, which improves the half-life of GLP-1 from 1-2 min to 4-7 days, allowing for weekly dosing (137). Similarly, Tanzeum^{®} is a "biobetter" version of first generation GLP-1 receptor agonists and is approved for weekly dosing in type-2 diabetes (137). CSL654 is an albumin fusion of rhFactor IX recently approved by the FDA, with a half-life of 89-96 hours (137). Albutropin, an hGH albumin fusion, showed a half-life of 4-6 hours, a fourfold increase (151). An ErbB2 scFv antibody-HSA-anti-Her2 extended the scFvs half-life from 1 hr to 86-90 hours (137). IFN-alpha2a-HSA fusion and rFVIIa-HAS are in preclinical development, while other albumin fusions, e.g., G-CSF-HAS and Albuferon^{®}, have been abandoned (137). These examples also show that the half-life of the original molecule can be dominant in the half-life of the fusion protein and limit the benefit of the construct.

Construct design. The following constructs are generated: (A) full length CRF-BP fused to IgG1 Fc (used in the studies described above); (B) full length CRF-BP fused to IgG1 Fc through a linker; (C) CRF-BP234 fused to IgG1 Fc through a linker; (D) full length CRF-BP C-terminal fusion with albumin; and (E) CRF-BP234 C-terminal fusion with albumin.

Rationale: These constructs are motivated by results (see Figs. 1-3) that involve fusion of full length CRF-BP to mouse IgG1 Fc, which did not alter CRF binding (data not shown); it was also observed that direct fusion of CRF-BP234 with the IgG1 Fc did not correctly assemble consistent with CRF-BP C-terminal (amino acid residues 235-322) acting as a flexible spacer between the CRF-BP moieties and the hinge. A conventional linker with small, polar amino acids, e.g., (GGGGS/SEQ ID NO:11)n that provides good flexibility and helps stability in aqueous solvent through formation of hydrogen bonds with water (152). Flexible (GGGGS)n linkers are extensively used in Ig derived fusions including single chain antibodies and Fc fusions resulting in improved folding and (152, 153, 154, 155). The length of the linker can be adjusted to achieve appropriate separation of the functional domains by adjusting the copy number of GGGGS (SEQ ID NO: 11) modules as warranted, e.g., (GGGGS)n, where n=1, n=2, n=3, n=4, or n=5. More natural-type linkers can be used, including extended hinges, such as inclusion of an extra CH1 motif (126) or an extra-long hinge derived from naturally-occurring Ig molecules (127, 128), as well as other sequences (134, 152, 153, 154, 156, 157, 158, 159, 160).

Monovalent fusions to the C terminus or N terminus of albumin, which has a 19-day half-life in human serum, are a viable alternative to Fc fusion since albumin also binds FcRn and is recycled similarly to IgGs, resulting in its extended half-life (129, 130). The constructs in points D and E, above, test fusions with C terminal albumin fusions to extend the half-life of CRF-BP and CRF-BP234 in monovalent constructs with lower potential of reduced FcRn binding due to steric hindrance.

Doses. To evaluate the half-life in serum of the constructs under testing, single-dose pharmacokinetics (PK) in mice are conducted. To this end, mice are injected intraperitoneally (i.p.) with the test compound at a dose of 450 µg/mouse in 100 µl phosphate buffered saline (PBS). Concomitant measures of body temperature are carried out for stress-induced hyperthermia (SIH) determination. Doses for shock-induced freezing (SIF) are 45, 150, and 450 µg.

Pharmacokinetics - determination of half-life. Mice are bled at 1, 6, and 12 hours and at 1, 2, 4, 6, 8, and 12 days post-injection by retro-orbital bleeding. Data obtained from serum concentrations (Fig. 2A) of two doses (15 and 45 µg) in mice shows linear pharmacokinetics with a biexponential profile. The present blood sampling times presuppose a half-life value closer to those reported for human IgG in the mouse. These data are analyzed by means of non-compartmental analysis and, if results are inconclusive for selection of the best molecule, a two-compartmental pharmacokinetic model is applied.

Analytical methods. Concentrations in sera over time of CRF-BP-Fc or CRF-BP-albumin constructs are measured with a sandwich ELISA, which has already been established with a lower limit of detection of 1 ng/ml in serum. This ELISA assay consists of immobilized biotinylated CRF to capture serum CRF-BP-Fc on avidin plates followed by detection with a commercial anti-Fc antibody. This assay is used to monitor the serum concentration of CRF-BP-Fc in preliminary studies. CRF-BP-albumin constructs are detected with the same design using antibodies to albumin and blocking reagents designed for albumin ELISA quantification (e.g., Serum Albumin Sandwich ELISA Kit from LifeSpan BioSciences). To allow for half-life determination of CRF-BP (not Fc or albumin fused), the use of the aforementioned sandwich ELISA using a commercial anti-CRF-BP antibody instead of an anti-Fc antibody, or a sandwich ELISA is compared with 2 different specificities of anti-CRF-BP antibodies as an alternative strategy. With regard to detection of CRF-BP and CRF-BP-Fc or CRF-BP-albumin in the mouse, it is important to note that, while the biological actions of peripheral CRF-BP in the mouse resemble those of humans (119) and Fig. 1, mice and other rodents do not have endogenous CRF-BP in their serum, as discussed above, which facilitates the present analysis. To measure corticosterone levels, a commercial ELISA is conveniently used.

### Behavioral methods.

Stress-induced hyperthermia (SIH). Individually housed mice are subjected to two sequential rectal temperature measurements with a 10-min interval (161, 162). The first measurement captures the animal's basal core temperature (T1), while the second temperature (T2) captures the stress-enhanced temperature. The difference between the first and second temperatures (T2-T1 or ΔT) is the SIH. Temperature measurements are made to the nearest 0.1°C with a lubricated thermistor probe inserted into the mouse rectum. Measurements are combined with collection of blood for corticosterone and compound measures (162). SIH can be repeated several times in the same animal and the results are very consistent over time (161). Additionally, SIH does not depend on locomotor activity, like most anxiety tests (161, 162), and therefore is an attractive orthogonal test to the main efficacy assay proposed (Shock-induced freezing, see next).

Shock-induced freezing (SIF). SIF is a defensive behavior observed in response to fearful stimuli exposure (163), and it is used to determine the efficacy of the constructs under study (Fig. 1B). Testing takes place in a Mouse NIR Video Fear Conditioning System (Med Associates, St. Albans, VT) housed in soundproof boxes. The session consists of a 2-min habituation period followed by three 1.5 mA footshocks lasting 1 sec and separated by 20 sec. Duration of freezing behavior is measured for 15 min. Freezing behavior, i.e., the absence of all voluntary movements except breathing, is measured in all sessions by real-time digital video recordings calibrated to distinguish between subtle movements such as whisker twitch or tail flick and freezing behavior. This test is performed at days 1 and 4 after administration. Before and after footshock, mice are bled as outlined above for determination of serum corticosterone and compound levels.

Statistical analyses of behavioral data. If the data do not violate the assumption of homogeneity of variance, appropriate analyses of variance (ANOVAs) are performed, predominantly according to mixed-factorial (split-plot) or latin square designs. Data violating homogeneity of variance are transformed to meet the ANOVA assumptions. Comparisons among individual means are made by simple effects and/or Newman-Keuls *post-hoc* tests following overall ANOVA. Ordinal, equal interval, and ratio data from samples not meeting the assumptions of the generally more powerful parametric statistics are analyzed using appropriate non-parametric tests (see, 164, 165).

### Specific Aim 2. To convert the most promising constructs into their whole human counterpart(s).

Objective. Conversions into their human counterpart(s) of 1-2 Fc or albumin fusion compounds are performed, and the resulting fusion proteins are tested in mice with the humanized FcRn receptor: FcRn^{-/-}/hFcRn (line 276) transgenic (Tg) mice, which express the human FcRn α-chain and carry a null mutation for the mouse FcRn gene (118). Compound half-life and efficacy (SIH) are tested. Initially, mouse IgG1 Fc fusion constructs are converted to human IgG2, which, like mouse IgG1, have reduced Fc effector function; mouse albumin fusions are converted to human albumin fusions for testing in FcRn⁻/-/hFcRn Tg mice, where they are expected to reveal greater half-lives since in normal mice, albumin half-life is shorter than in humans (166, 167). Further optimizations then take place (see below). CRF binding of human compounds are verified by ELISA and surface plasmon resonance (BiaCore). *In vivo* testing is conducted, as described above, and involves determination of half-life (see Pharmacokinetics above) and concomitant measures of SIH.

Follow-on activities and alternative strategies. Subsequent to the studies above, further Fc optimization tests Fc derived from IgG2 and IgG4 moieties, which are characterized by low to none effector function and long half life, and thus are preferred for the neutralization of soluble antigens (117). For instance, IgG2 Fc was selected for the anti-CD3 antibody visilizumab (Nuvion) on the basis of a side-by-side comparison of all four isotypes (122). For CDP571, a humanized monoclonal antibody to TNF-α, *in vivo* comparison of IgG1 and IgG4 resulted in selection of the IgG4 isotype (123). Further optimizations include stabilized or non-stabilized IgG4 to further reduce effector function, e.g., as used in the anti-human CD4 antibody clenoliximab (117, 124, 125). Constructs with naturalistic extended hinges, such as inclusion of an extra CH1 motive (126) or an extra-long hinge derived from naturally-occurring Ig molecules (127, 128), can also be explored on the basis of the results of studies. Alternatively, albumin fusions are capable of extending half-life, and alternative albumin designs can be tested, including N-terminal fusions with albumin and with minimal albumin-binding domain (ABD) (see, e.g., 129, 130, 131, 132, 133). Alternative strategies also include non-fusion protein modification, e.g., by PEGylation (see, e.g., 134) and fusion with transferrin (see, e.g., 129, 131, 134, 135, 136, 137). As both Fc and CRF-BP are glycosylated (139), glycosylation can be altered to improve the functional properties of the proposed fusion proteins (see, e.g., 140, 141).

### EXAMPLE 2

The invention also encompasses engineered CRF antagonist agents that comprise a polypeptide CRF (e.g., CRF1) antagonist covalently conjugated to a half-life-extending moiety which is is an Fc forming portion of a mammalian immunoglobulin heavy chain, or an Fc region of an antibody, optionally an Fc region of a human antibody, as set out in the claims. Other half-life extending moieties includealbumin, transthyretin, transferrin, PEG, etc. A molecule of the invention possessing: 1) long *in vivo* half-life; 2) peripheral-only penetration (i.e., therapeutic agent does not penetrate the blood brain barrier); and 3) is a CRF antagonist (preferably having CRF1-selective antagonist activity), can also be constructed by covalently conjugating a CRF antagonist to a half-life extending moiety as set out in the claims, as described herein for CRF binding agents, in general, with or without linkers (peptidyl or non-peptidyl linkers), as desired. The CRF antagonist agents of the invention can be included in pharmaceutical compositions and used in a method of treating a disease or disorder, or condition characterized by HPA axis hyperactivity, e.g., anxiety, depression, Alzheimer's and Parkinson's diseases, obesity, metabolic syndrome, type 2 diabetes, osteoporosis, cardiovascular disease, alcohol or drug abuse, inflammatory bowel disease (IBD), and irritable bowel syndrome (IBS).

Some representative CRF antagonist polypeptides with CRF1 antagonist activity that can be covalently conjugated to a half-life extending moiety, and used in the inventive pharmaceutical compositions and methods of treatment, include molecules in the following Table 2. Small molecules with CRF1 antagonist activity are also disclosed in Table 2, but are not per se encompassed by claim 1.

**Table 2. Representative CRF1 anatagonists.**

| Compound number | Name and references | Structure / sequence |
|---|---|---|
| 1 | α-helical CRF 9-41 | DLTFHLLREMLEMAKAEQEAEQAALNRLLLEEA// SEQ ID NO:7 |
| 2 | Antalarmin | 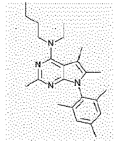 |
| 3 | D-His 13-hCRF | MGGHPQLRLVKA[D-His] LLLGLNPVSASLQDQHCESLSLASNISGLQCNASVDL IGTCWPRSPAGQLVVRPCPAFFYGVRYNTTNNGYRE CLANGSWAARVNYSECQEILNEEKKSKVHYHVAVII NYLGHCISLVALLVAFVLFLRLRPGCTHWGDQADG ALEVGAPWSGAPFQVRRSIRCLRNIIHWNLISAFILR NATWFVVQLTMSPEVHQSNVGWCRLVTAAYNYFH VTNFFWMFGEGCYLHTAIVLTYSTDRLRKWMFICIG WGVPFPIIVAWAIGKLYYDNEKCWFGKRPGVYTDYI YQGPMILVLLINFIFLFNIVRILMTKLRASTTSETIQYR KAVKATLVLLPLLGITYMLFFVNPGEDEVSRVVFIYF NSFLESFQGFFVSVFYCFLNSEVRSAIRKRWHRWQD KHSIRARVARAMSIPTSPTRVSFHSIKQSTAV//SEQ ID NO:61 |
| 4 | D-Phe-hCRF12-41 | [D-Phe]ALLLLGLNP VSASLQDQHC ESLSLASNIS G//SEQ ID NO:62 |
| 5 | astressin | cyclo(30-33)[D-Phe12, Nle21, Glu30,Lys38, Nle38]hCRF(12-41), i.e., cyclo(30-33)[D-Phe]LLLLGLNP [Nle]SASLQDQHE ESLSLAS[X]IS G, where X is E or Nle // SEO ID NO:8 |
| 6 | DMP-904 | 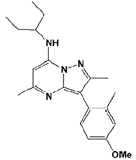 |
| | 3-(4-methoxy-2-methylphe nyl)-2,5-dimethyl-N-pentan-3-ylpyrazolo[ 1,5-a]pyrimidi n-7-amine | |
| 7 | DMP696 | 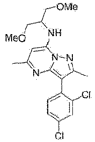 |
| | 8-(2,4-dichlorophe nyl)-N-(1,3-dimethoxyp ropan-2-yl)-2,7-dimethylpy razolo[1,5-a][1,3,5]tria zin-4-amine | |
| 8 | Verucerfon t (GSK-561,679) | 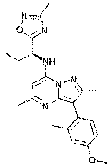 |
| 9 | R121919 (NBI30775 ) | 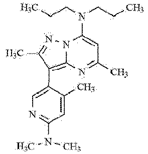 |
| 10 | CP-154526 | 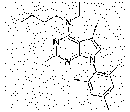 |
| 11 | R-278995 | 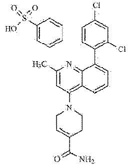 |
| 12 | SSR-125543A | 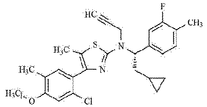 |
| 13 | NBI27914 | 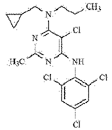 |
| 13 | Pexacerfon t (BMS-562,086) | 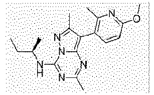 |

### EXAMPLE 3

Methods. Fifteen male C57BL/6J mice (Jackson Labs, Bar Harbor, ME) at 12 weeks of age (housed 3-4 mice per cage) were used in another experiment. All mice were tail bled prior to the initiation of the study (Day 1). 5 mice were then injected IP with 15 µg test compound (CRFBP-Fc with the Fc derived from mouse IgG1, i.e., SEQ ID NO: 16) in 0.2 ml saline, 5 mice received 45 µg test compound (CRFBP-Fc, i.e., SEQ ID NO: 16), and 5 mice received saline only. Then, 3-hours post-injection, the mice were tail bled again and then tested in the shock-induced freezing test. Following testing, mice were again bled. On Days 2, 5, and 10, mice were tail bled, tested in the shock-induced freezing test and then tail bled a second time.

Tail bleeding. Tails were nicked with a sterile blade approximately 2 mm from the tip and 80-100 µl of blood was collected into heparinized capillary tubes. Subsequent sampling on the same day did not require cutting, but only the gentle removal of the scab. Samples were immediately transferred to Eppendorf tubes containing EDTA (0.23 g/ml, 10 µl per tube) and centrifuged at 2000 rpm for 20 min. Ten microliters of plasma was removed to a fresh Eppendorf tube for corticosterone measurement and the remaining plasma was divided into two additional tubes. All samples were then stored at -80°C until analysis. Results showing serum concentrations of the test compound (SEQ ID NO:16) are shown in Figure 2A.

Shock-induced freezing. Mice were placed in a Mouse NIR Video Fear Conditioning System (Med Associates, St. Albans, VT) housed in a soundproofed box, allowed to habituate for 2 min, and then were exposed to three 1.5 mA, 1-sec footshocks, separated by 20 sec. Freezing, a CRF/CRFR1-dependent defensive response (see, Kalin et al., Antagonism of endogenous CRH systems attenuates stress-induced freezing behavior in rats. Brain research 457:130-135 (1988)), was measured automatically from real time video recordings (30 frames per second) across 15 min using Video Fear Conditioning Software, Med Associates which distinguishes between subtle movements such as whisker twitch or tail flick and freezing behavior. Results are shown in Figure 2B.

In a different experiment, sixteen male C57BL/6J mice (Jackson Labs, Bar Harbor, ME) at 12 weeks of age (housed 4 mice per cage) were used in this experiment. All mice were tail bled prior to the initiation of the study (Day 1). 8 mice were then injected I.P. with 300 µg of test compound (CRFBP-Fc, with the Fc moiety derived from human IgG2 sequence, i.e., SEQ ID NO:23) in 0.11 ml saline and 8 mice received saline only. Then 3 hours post injection, mice were tail bled again and were subjected to footshock, as described above. Following footshock, mice were again bled, as outlined above. On Days 2, 5, and 10, mice were tail bled, subjected to footshock and then tail bled a second time. Samples were handled as described above in this Example 3. Results showing baseline and stimulated serum concentrations of the test compound (SEQ ID NO:23) are shown in Figure 8 and Figure 9, respectively.

### References

1. Herman JP, Figueiredo H, Mueller NK, Ulrich-Lai Y, Ostrander MM, Choi DC, Cullinan WE. 2003. Central mechanisms of stress integration: hierarchical circuitry controlling hypothalamo-pituitary-adrenocortical responsiveness. Front Neuroendocrinol 24:151-180.
2. Blackwell RE, Guillemin R. 1973. Hypothalamic control of adenohypophysial secretions. Annu Rev Physiol 35:357-390.
3. Behan DP, Khongsaly O, Liu XJ, Ling N, Goland R, Nasman B, Olsson T, De Souza EB. 1996. Measurement of corticotropin-releasing factor (CRF), CRF-binding protein (CRF-BP), and CRF/CRF-BP complex in human plasma by two-site enzyme-linked immunoabsorbant assay. J Clin Endocrinol Metab 81:2579-2586.
4. McEwen BS, Stellar E. 1993. Stress and the individual. Mechanisms leading to disease. Arch Intern Med 153:2093-2101.
5. Dallman MF, la Fleur SE, Pecoraro NC, Gomez F, Houshyar H, Akana SF. 2004. Minireview: glucocorticoids--food intake, abdominal obesity, and wealthy nations in 2004. Endocrinology 145:2633-2638.
6. Uhart M, Wand GS. 2009. Stress, alcohol and drug interaction: an update of human research. Addiction biology 14:43-64.
7. Prpic-Krizevac I, Canecki-Varzic S, Bilic-Curcic I. 2012. Hyperactivity of the hypothalamic-pituitary-adrenal axis in patients with type 2 diabetes and relations with insulin resistance and chronic complications. Wien Klin Wochenschr 124:403-411.
8. Vicennati V, Pasquali R. 2000. Abnormalities of the hypothalamic-pituitary-adrenal axis in nondepressed women with abdominal obesity and relations with insulin resistance: evidence for a central and a peripheral alteration. J Clin Endocrinol Metab 85:4093-4098.
9. Vrkljan M, Thaller V, Lovricevic I, Gacina P, Resetic J, Bekic M, Sonicki Z. 2001. Depressive disorder as possible risk factor of osteoporosis. Coll Antropol 25:485-492.
10. Tsigos C, Chrousos GP. 2002. Hypothalamic-pituitary-adrenal axis, neuroendocrine factors and stress. J Psychosom Res 53:865-871.
11. Evans JF, Ragolia L. 2012. Systemic and local ACTH produced during inflammatory states promotes osteochondrogenic mesenchymal cell differentiation contributing to the pathologic progression of calcified atherosclerosis. Med Hypotheses 79:823-826.
12. Goodwin JE. 2015. Glucocorticoids and the Cardiovascular System. Adv Exp Med Biol 872:299-314.
13. Fekete EM, Zorrilla EP. 2007. Physiology, pharmacology, and therapeutic relevance of urocortins in mammals: ancient CRF paralogs. Front Neuroendocrinol 28:1-27.
14. Bornstein SR, Webster EL, Torpy DJ, Richman SJ, Mitsiades N, Igel M, Lewis DB, Rice KC, Joost HG, Tsokos M, Chrousos GP. 1998. Chronic effects of a nonpeptide corticotropin-releasing hormone type I receptor antagonist on pituitary-adrenal function, body weight, and metabolic regulation. Endocrinology 139:1546-1555.
15. Kiank C, Tache Y, Larauche M. 2010. Stress-related modulation of inflammation in experimental models of bowel disease and post-infectious irritable bowel syndrome: role of corticotropin-releasing factor receptors. Brain, behavior, and immunity 24:41-48.
16. Overman EL, Rivier JE, Moeser AJ. 2012. CRF induces intestinal epithelial barrier injury via the release of mast cell proteases and TNF-alpha. PloS one 7:e39935.
17. Paschos KA, Kolios G, Chatzaki E. 2009. The corticotropin-releasing factor system in inflammatory bowel disease: prospects for new therapeutic approaches. Drug Discov Today 14:713-720.
18. Wiley KE, Davenport AP. 2004. CRF2 receptors are highly expressed in the human cardiovascular system and their cognate ligands urocortins 2 and 3 are potent vasodilators. British journal of pharmacology 143:508-514.
19. Walczewska J, Dzieza-Grudnik A, Siga O, Grodzicki T. 2014. The role of urocortins in the cardiovascular system. J Physiol Pharmacol 65:753-766.
20. Brar BK, Jonassen AK, Egorina EM, Chen A, Negro A, Perrin MH, Mjos OD, Latchman DS, Lee KF, Vale W. 2004. Urocortin-II and urocortin-III are cardioprotective against ischemia reperfusion injury: an essential endogenous cardioprotective role for corticotropin releasing factor receptor type 2 in the murine heart. Endocrinology 145:24-35; discussion 21-23.
21. Coste SC, Kesterson RA, Heldwein KA, Stevens SL, Heard AD, Hollis JH, Murray SE, Hill JK, Pantely GA, Hohimer AR, Hatton DC, Phillips TJ, Finn DA, Low MJ, Rittenberg MB, Stenzel P, Stenzel-Poore MP. 2000. Abnormal adaptations to stress and impaired cardiovascular function in mice lacking corticotropin-releasing hormone receptor-2. Nat Genet 24:403-409.
22. Charlton BG, Ferrier IN, Perry RH. 1987. Distribution of corticotropin-releasing factor-like immunoreactivity in human brain. Neuropeptides 10:329-334.
23. Swanson LW, Simmons DM. 1989. Differential steroid hormone and neural influences on peptide mRNA levels in CRH cells of the paraventricular nucleus: a hybridization histochemical study in the rat. The Journal of comparative neurology 285:413-435.
24. Vyas S, Maatouk L. 2013. Contribution of glucocorticoids and glucocorticoid receptors to the regulation of neurodegenerative processes. CNS Neurol Disord Drug Targets 12:1175-1193.
25. Makino S, Schulkin J, Smith MA, Pacak K, Palkovits M, Gold PW. 1995. Regulation of corticotropin-releasing hormone receptor messenger ribonucleic acid in the rat brain and pituitary by glucocorticoids and stress. Endocrinology 136:4517-4525.
26. Makino S, Gold PW, Schulkin J. 1994. Corticosterone effects on corticotropin-releasing hormone mRNA in the central nucleus of the amygdala and the parvocellular region of the paraventricular nucleus of the hypothalamus. Brain research 640:105-112.
27. Makino S, Gold PW, Schulkin J. 1994. Effects of corticosterone on CRH mRNA and content in the bed nucleus of the stria terminalis; comparison with the effects in the central nucleus of the amygdala and the paraventricular nucleus of the hypothalamus. Brain research 657:141-149.
28. Sapolsky RM, Krey LC, McEwen BS. 1984. Glucocorticoid-sensitive hippocampal neurons are involved in terminating the adrenocortical stress response. Proceedings of the National Academy of Sciences of the United States of America 81:6174-6177.
29. Sapolsky RM, Krey LC, McEwen BS. 1985. Prolonged glucocorticoid exposure reduces hippocampal neuron number: implications for aging. The Journal of neuroscience : the official journal of the Society for Neuroscience 5:1222-1227.
30. Koob GF. 1999. Corticotropin-releasing factor, norepinephrine, and stress. Biological psychiatry 46:1167-1180.
31. Schulkin J, Gold PW, McEwen BS. 1998. Induction of corticotropin-releasing hormone gene expression by glucocorticoids: implication for understanding the states of fear and anxiety and allostatic load. Psychoneuroendocrinology 23:219-243.
32. Gold PW, Chrousos GP. 2002. Organization of the stress system and its dysregulation in melancholic and atypical depression: high vs low CRH/NE states. Mol Psychiatry 7:254-275.
33. Gold PW. 2015. The organization of the stress system and its dysregulation in depressive illness. Mol Psychiatry 20:32-47.
34. Arborelius L, Owens MJ, Plotsky PM, Nemeroff CB. 1999. The role of corticotropin-releasing factor in depression and anxiety disorders. The Journal of endocrinology 160:1-12.
35. Galard R, Catalan R, Castellanos JM, Gallart JM. 2002. Plasma corticotropin-releasing factor in depressed patients before and after the dexamethasone suppression test. Biological psychiatry 51:463-468.
36. Catalan R, Gallart JM, Castellanos JM, Galard R. 1998. Plasma corticotropin-releasing factor in depressive disorders. Biological psychiatry 44: 15-20.
37. Raadsheer FC, van Heerikhuize JJ, Lucassen PJ, Hoogendijk WJ, Tilders FJ, Swaab DF. 1995. Corticotropin-releasing hormone mRNA levels in the paraventricular nucleus of patients with Alzheimer's disease and depression. Am J Psychiatry 152:1372-1376.
38. Lehner M, Wislowska-Stanek A, Skorzewska A, Plaznik A. 2015. Chronic restraint increases apoptosis in the hippocampus of rats with high responsiveness to fear stimuli. Neuroscience letters 586:55-59.
39. Wellman CL. 2001. Dendritic reorganization in pyramidal neurons in medial prefrontal cortex after chronic corticosterone administration. J Neurobiol 49:245-253.
40. Liston C, Miller MM, Goldwater DS, Radley JJ, Rocher AB, Hof PR, Morrison JH, McEwen BS. 2006. Stress-induced alterations in prefrontal cortical dendritic morphology predict selective impairments in perceptual attentional set-shifting. The Journal of neuroscience : the official journal of the Society for Neuroscience 26:7870-7874.
41. Liu RJ, Aghajanian GK. 2008. Stress blunts serotonin- and hypocretin-evoked EPSCs in prefrontal cortex: role of corticosterone-mediated apical dendritic atrophy. Proceedings of the National Academy of Sciences of the United States of America 105:359-364.
42. Hains AB, Vu MA, Maciejewski PK, van Dyck CH, Gottron M, Arnsten AF. 2009. Inhibition of protein kinase C signaling protects prefrontal cortex dendritic spines and cognition from the effects of chronic stress. Proceedings of the National Academy of Sciences of the United States of America 106:17957-17962.
43. Gourley SL, Swanson AM, Koleske AJ. 2013. Corticosteroid-induced neural remodeling predicts behavioral vulnerability and resilience. The Journal of neuroscience : the official journal of the Society for Neuroscience 33:3107-3112.
44. Goldstein DS, McEwen B. 2002. Allostasis, homeostats, and the nature of stress. Stress 5:55-58.
45. McEwen BS. 2002. Sex, stress and the hippocampus: allostasis, allostatic load and the aging process. Neurobiology of aging 23:921-939.
46. McEwen BS. 2001. From molecules to mind. Stress, individual differences, and the social environment. Annals of the New York Academy of Sciences 935:42-49.
47. Carroll BJ, Cassidy F, Naftolowitz D, Tatham NE, Wilson WH, Iranmanesh A, Liu PY, Veldhuis JD. 2007. Pathophysiology of hypercortisolism in depression. Acta Psychiatr Scand Suppl:90-103.
48. Wingenfeld K, Wolf OT. 2015. Effects of cortisol on cognition in major depressive disorder, posttraumatic stress disorder and borderline personality disorder - 2014 Curt Richter Award Winner. Psychoneuroendocrinology 51:282-295.
49. Ising M, Horstmann S, Kloiber S, Lucae S, Binder EB, Kem N, Kunzel HE, Pfennig A, Uhr M, Holsboer F. 2007. Combined dexamethasone/corticotropin releasing hormone test predicts treatment response in major depression - a potential biomarker? Biological psychiatry 62:47-54.
50. Nemeroff CB. 1996. The corticotropin-releasing factor (CRF) hypothesis of depression: new findings and new directions. Mol Psychiatry 1:336-342.
51. Holsboer F. 2000. The corticosteroid receptor hypothesis of depression. Neuropsychopharmacology : official publication of the American College of Neuropsychopharmacology 23:477-501.
52. Yehuda R. 2002. Current status of cortisol findings in post-traumatic stress disorder. The Psychiatric clinics of North America 25:341-368, vii.
53. de Kloet C, Vermetten E, Lentjes E, Geuze E, van Pelt J, Manuel R, Heijnen C, Westenberg H. 2008. Differences in the response to the combined DEX-CRH test between PTSD patients with and without co-morbid depressive disorder. Psychoneuroendocrinology 33:313-320.
54. Sigalas PD, Garg H, Watson S, McAllister-Williams RH, Ferrier IN. 2012. Metyrapone in treatment-resistant depression. Ther Adv Psychopharmacol 2:139-149.
55. Zobel AW, Nickel T, Kunzel HE, Ackl N, Sonntag A, Ising M, Holsboer F. 2000. Effects of the high-affinity corticotropin-releasing hormone receptor 1 antagonist R121919 in major depression: the first 20 patients treated. J Psychiatr Res 34:171-181.
56. Binneman B, Feltner D, Kolluri S, Shi Y, Qiu R, Stiger T. 2008. A 6-week randomized, placebo-controlled trial of CP-316,311 (a selective CRH1 antagonist) in the treatment of major depression. Am J Psychiatry 165:617-620.
57. Coric V, Feldman HH, Oren DA, Shekhar A, Pultz J, Dockens RC, Wu X, Gentile KA, Huang SP, Emison E, Delmonte T, D'Souza BB, Zimbroff DL, Grebb JA, Goddard AW, Stock EG. 2010. Multicenter, randomized, double-blind, active comparator and placebo-controlled trial of a corticotropin-releasing factor receptor-1 antagonist in generalized anxiety disorder. Depress Anxiety 27:417-425.
58. Kwako LE, Spagnolo PA, Schwandt ML, Thorsell A, George DT, Momenan R, Rio DE, Huestis M, Anizan S, Concheiro M, Sinha R, Heilig M. 2015. The corticotropin releasing hormone-1 (CRH1) receptor antagonist pexacerfont in alcohol dependence: a randomized controlled experimental medicine study. Neuropsychopharmacology : official publication of the American College of Neuropsychopharmacology 40:1053-1063.
59. Ratka A, Sutanto W, Bloemers M, de Kloet ER. 1989. On the role of brain mineralocorticoid (type I) and glucocorticoid (type II) receptors in neuroendocrine regulation. Neuroendocrinology 50:117-123.
60. Spiga F, Harrison LR, Wood SA, Atkinson HC, MacSweeney CP, Thomson F, Craighead M, Grassie M, Lightman SL. 2007. Effect of the glucocorticoid receptor antagonist Org 34850 on basal and stress-induced corticosterone secretion. Journal of neuroendocrinology 19:891-900.
61. Fleck BA, Hoare SR, Pick RR, Bradbury MJ, Grigoriadis DE. 2012. Binding kinetics redefine the antagonist pharmacology of the corticotropin-releasing factor type 1 receptor. J Pharmacol Exp Ther 341:518-531.
62. Ayala AR, Pushkas J, Higley JD, Ronsaville D, Gold PW, Chrousos GP, Pacak K, Calis KA, Gerald M, Lindell S, Rice KC, Cizza G. 2004. Behavioral, adrenal, and sympathetic responses to long-term administration of an oral corticotropin-releasing hormone receptor antagonist in a primate stress paradigm. J Clin Endocrinol Metab 89:5729-5737.
63. Kunzel HE, Zobel AW, Nickel T, Ackl N, Uhr M, Sonntag A, Ising M, Holsboer F. 2003. Treatment of depression with the CRH-1-receptor antagonist R121919: endocrine changes and side effects. J Psychiatr Res 37:525-533.
64. DeBattista C, Belanoff J, Glass S, Khan A, Home RL, Blasey C, Carpenter LL, Alva G. 2006. Mifepristone versus placebo in the treatment of psychosis in patients with psychotic major depression. Biological psychiatry 60:1343-1349.
65. Eckstein N, Haas B, Hass MD, Pfeifer V. 2014. Systemic therapy of Cushing's syndrome. Orphanet J Rare Dis 9:122.
66. Repunte-Canonigo V, Shin W, Vendruscolo LF, Lefebvre C, Koob GF, Califano A, Sanna PP. 2015. Identifying candidate drivers of alcohol dependence-induced excessive drinking by assembly and interrogation of brain-specific regulatory networks. Genome Biology:16:68 DOI: 10.1186/s13059-13015-10593-13055
67. Vendruscolo LF, Barbier E, Schlosburg JE, Misra KK, Whitfield TW, Jr., Logrip ML, Rivier C, Repunte-Canonigo V, Zorrilla EP, Sanna PP, Heilig M, Koob GF. 2012. Corticosteroid-dependent plasticity mediates compulsive alcohol drinking in rats. The Journal of neuroscience : the official journal of the Society for Neuroscience 32:7563-7571.
68. Vendruscolo LF, Estey D, Goodell V, Macshane LG, Logrip ML, Schlosburg JE, McGinn MA, Zamora-Martinez ER, Belanoff JK, Hunt HJ, Sanna PP, George O, Koob GF, Edwards S, Mason BJ. 2015. Glucocorticoid receptor antagonism decreases alcohol seeking in alcohol-dependent individuals. J Clin Invest.
69. Fiancette JF, Balado E, Piazza PV, Deroche-Gamonet V. 2010. Mifepristone and spironolactone differently alter cocaine intravenous self-administration and cocaine-induced locomotion in C57BL/6J mice. Addiction biology 15:81-87.
70. Ambroggi F, Turiault M, Milet A, Deroche-Gamonet V, Parnaudeau S, Balado E, Barik J, van der Veen R, Maroteaux G, Lemberger T, Schutz G, Lazar M, Marinelli M, Piazza PV, Tronche F. 2009. Stress and addiction: glucocorticoid receptor in dopaminoceptive neurons facilitates cocaine seeking. Nat Neurosci 12:247-249.
71. Wolkowitz OM. 1994. Prospective controlled studies of the behavioral and biological effects of exogenous corticosteroids. Psychoneuroendocrinology 19:233-255.
72. Sandeep TC, Yau JL, MacLullich AM, Noble J, Deary IJ, Walker BR, Seckl JR. 2004. 11Beta-hydroxy steroid dehydrogenase inhibition improves cognitive function in healthy elderly men and type 2 diabetics. Proceedings of the National Academy of Sciences of the United States of America 101:6734-6739.
73. Djamshidian A, Lees AJ. 2014. Can stress trigger Parkinson's disease? J Neurol Neurosurg Psychiatry 85:878-881.
74. Hou G, Tian R, Li J, Yuan TF. 2014. Chronic stress and Parkinson's disease. CNS Neurosci Ther 20:1-2.
75. Hartmann A, Veldhuis JD, Deuschle M, Standhardt H, Heuser I. 1997. Twenty-four hour cortisol release profiles in patients with Alzheimer's and Parkinson's disease compared to normal controls: ultradian secretory pulsatility and diurnal variation. Neurobiology of aging 18:285-289.
76. Notarianni E. 2013. Hypercortisolemia and glucocorticoid receptor-signaling insufficiency in Alzheimer's disease initiation and development. Curr Alzheimer Res 10:714-731.
77. de Quervain DJ, Poirier R, Wollmer MA, Grimaldi LM, Tsolaki M, Streffer JR, Hock C, Nitsch RM, Mohajeri MH, Papassotiropoulos A. 2004. Glucocorticoid-related genetic susceptibility for Alzheimer's disease. Human molecular genetics 13:47-52.
78. van Rossum EF, de Jong FJ, Koper JW, Uitterlinden AG, Prins ND, van Dijk EJ, Koudstaal PJ, Hofman A, de Jong FH, Lamberts SW, Breteler MM. 2008. Glucocorticoid receptor variant and risk of dementia and white matter lesions. Neurobiology of aging 29:716-723.
79. Popp J, Wolfsgruber S, Heuser I, Peters O, Hull M, Schroder J, Moller HJ, Lewczuk P, Schneider A, Jahn H, Luckhaus C, Perneczky R, Frolich L, Wagner M, Maier W, Wiltfang J, Kornhuber J, Jessen F. 2015. Cerebrospinal fluid cortisol and clinical disease progression in MCI and dementia of Alzheimer's type. Neurobiology of aging 36:601-607.
80. Dong H, Goico B, Martin M, Csernansky CA, Bertchume A, Csemansky JG. 2004. Modulation of hippocampal cell proliferation, memory, and amyloid plaque deposition in APPsw (Tg2576) mutant mice by isolation stress. Neuroscience 127:601-609.
81. Rissman RA, Lee KF, Vale W, Sawchenko PE. 2007. Corticotropin-releasing factor receptors differentially regulate stress-induced tau phosphorylation. The Journal of neuroscience : the official journal of the Society for Neuroscience 27:6552-6562.
82. Rothman SM, Herdener N, Camandola S, Texel SJ, Mughal MR, Cong WN, Martin B, Mattson MP. 2012. 3xTgAD mice exhibit altered behavior and elevated Abeta after chronic mild social stress. Neurobiology of aging 33:830 e831-812.
83. Touma C, Ambree O, Gortz N, Keyvani K, Lewejohann L, Palme R, Paulus W, Schwarze-Eicker K, Sachser N. 2004. Age- and sex-dependent development of adrenocortical hyperactivity in a transgenic mouse model of Alzheimer's disease. Neurobiology of aging 25:893-904.
84. Justice NJ, Huang L, Tian JB, Cole A, Pruski M, Hunt AJ, Jr., Flores R, Zhu MX, Arenkiel BR, Zheng H. 2015. Posttraumatic stress disorder-like induction elevates beta-amyloid levels, which directly activates corticotropin-releasing factor neurons to exacerbate stress responses. The Journal of neuroscience : the official journal of the Society for Neuroscience 35:2612-2623.
85. Piedrahita JA, Zhang SH, Hagaman JR, Oliver PM, Maeda N. 1992. Generation of mice carrying a mutant apolipoprotein E gene inactivated by gene targeting in embryonic stem cells. Proceedings of the National Academy of Sciences of the United States of America 89:4471-4475.
86. Raber J, Akana SF, Bhatnagar S, Dallman MF, Wong D, Mucke L. 2000. Hypothalamic-pituitary-adrenal dysfunction in Apoe(-/-) mice: possible role in behavioral and metabolic alterations. The Journal of neuroscience : the official journal of the Society for Neuroscience 20:2064-2071.
87. Grootendorst J, Enthoven L, Dalm S, de Kloet ER, Oitzl MS. 2004. Increased corticosterone secretion and early-onset of cognitive decline in female apolipoprotein E-knockout mice. Behavioural brain research 148:167-177.
88. Park HJ, Ran Y, Jung JI, Holmes O, Price AR, Smithson L, Ceballos-Diaz C, Han C, Wolfe MS, Daaka Y, Ryabinin AE, Kim SH, Hauger RL, Golde TE, Felsenstein KM. 2015. The stress response neuropeptide CRF increases amyloid-beta production by regulating gamma-secretase activity. EMBO J 34:1674-1686.
89. Zhang C, Kuo CC, Moghadam SH, Monte L, Campbell SN, Rice KC, Sawchenko PE, Masliah E, Rissman RA. 2015. Corticotropin-releasing factor receptor-1 antagonism mitigates beta amyloid pathology and cognitive and synaptic deficits in a mouse model of Alzheimer's disease. Alzheimers Dement.
90. Yaffe K, Vittinghoff E, Lindquist K, Barnes D, Covinsky KE, Neylan T, Kluse M, Marmar C. 2010. Posttraumatic stress disorder and risk of dementia among US veterans. Arch Gen Psychiatry 67:608-613.
91. Qureshi SU, Kimbrell T, Pyne JM, Magruder KM, Hudson TJ, Petersen NJ, Yu HJ, Schulz PE, Kunik ME. 2010. Greater prevalence and incidence of dementia in older veterans with posttraumatic stress disorder. J Am Geriatr Soc 58:1627-1633.
92. Bhatnagar S, Bell ME, Liang J, Soriano L, Nagy TR, Dallman MF. 2000. Corticosterone facilitates saccharin intake in adrenalectomized rats: does corticosterone increase stimulus salience? Journal of neuroendocrinology 12:453-460.
93. Schwartz MW, Woods SC, Porte D, Jr., Seeley RJ, Baskin DG. 2000. Central nervous system control of food intake. Nature 404:661-671.
94. Fleseriu M, Molitch ME, Gross C, Schteingart DE, Vaughan TB, 3rd, Biller BM. 2013. A new therapeutic approach in the medical treatment of Cushing's syndrome: glucocorticoid receptor blockade with mifepristone. Endocr Pract 19:313-326.
95. Mooij CF, Kroese JM, Claahsen-van der Grinten HL, Tack CJ, Hermus AR. 2010. Unfavourable trends in cardiovascular and metabolic risk in paediatric and adult patients with congenital adrenal hyperplasia? Clin Endocrinol (Oxf) 73:137-146.
96. Oakley RH, Cidlowski JA. 2015. Glucocorticoid signaling in the heart: A cardiomyocyte perspective. The Journal of steroid biochemistry and molecular biology 153:27-34.
97. Kumari M, Grahame-Clarke C, Shanks N, Marmot M, Lightman S, Vallance P. 2003. Chronic stress accelerates atherosclerosis in the apolipoprotein E deficient mouse. Stress 6:297-299.
98. Gross KJ, Pothoulakis C. 2007. Role of neuropeptides in inflammatory bowel disease. Inflamm Bowel Dis 13:918-932.
99. Muramatsu Y, Fukushima K, Iino K, Totsune K, Takahashi K, Suzuki T, Hirasawa G, Takeyama J, Ito M, Nose M, Tashiro A, Hongo M, Oki Y, Nagura H, Sasano H. 2000. Urocortin and corticotropin-releasing factor receptor expression in the human colonic mucosa. Peptides 21:1799-1809.
100. Saruta M, Takahashi K, Suzuki T, Torii A, Kawakami M, Sasano H. 2004. Urocortin 1 in colonic mucosa in patients with ulcerative colitis. J Clin Endocrinol Metab 89:5352-5361.
101. Gabry KE, Chrousos GP, Rice KC, Mostafa RM, Sternberg E, Negrao AB, Webster EL, McCann SM, Gold PW. 2002. Marked suppression of gastric ulcerogenesis and intestinal responses to stress by a novel class of drugs. Mol Psychiatry 7:474-483, 433.
102. Tache Y, Martinez V, Million M, Wang L. 2001. Stress and the gastrointestinal tract III. Stress-related alterations of gut motor function: role of brain corticotropin-releasing factor receptors. Am J Physiol Gastrointest Liver Physiol 280:G173-177.
103. Ramsey SJ, Attkins NJ, Fish R, van der Graaf PH. 2011. Quantitative pharmacological analysis of antagonist binding kinetics at CRF1 receptors in vitro and in vivo. British journal of pharmacology 164:992-1007.
104. Halbreich U, Asnis GM, Shindledecker R, Zumoff B, Nathan RS. 1985. Cortisol secretion in endogenous depression. II. Time-related functions. Arch Gen Psychiatry 42:909-914.
105. Halbreich U, Asnis GM, Shindledecker R, Zumoff B, Nathan RS. 1985. Cortisol secretion in endogenous depression. I. Basal plasma levels. Arch Gen Psychiatry 42:904-908.
106. Linkowski P, Mendlewicz J, Leclercq R, Brasseur M, Hubain P, Golstein J, Copinschi G, Van Cauter E. 1985. The 24-hour profile of adrenocorticotropin and cortisol in major depressive illness. J Clin Endocrinol Metab 61:429-438.
107. Pfohl B, Sherman B, Schlechte J, Stone R. 1985. Pituitary-adrenal axis rhythm disturbances in psychiatric depression. Arch Gen Psychiatry 42:897-903.
108. Sharma R, Markar HR. 1994. Mortality in affective disorder. J Affect Disord 31:91-96.
109. Goosens KA, Sapolsky RM. 2007. Stress and Glucocorticoid Contributions to Normal and Pathological Aging. In Riddle DR (ed.), Brain Aging: Models, Methods, and Mechanisms, Boca Raton (FL).
110. Soufer R, Burg MM. 2007. The heart-brain interaction during emotionally provoked myocardial ischemia: implications of cortical hyperactivation in CAD and gender interactions. Cleve Clin J Med 74 Suppl LS59-62.
111. Cocco G, Chu D. 2007. Stress-induced cardiomyopathy: A review. Eur J Intern Med 18:369-379.
112. Rozanski A, Blumenthal JA, Kaplan J. 1999. Impact of psychological factors on the pathogenesis of cardiovascular disease and implications for therapy. Circulation 99:2192-2217.
113. Linton EA, Behan DP, Saphier PW, Lowry PJ. 1990. Corticotropin-releasing hormone (CRH)-binding protein: reduction in the adrenocorticotropin-releasing activity of placental but not hypothalamic CRH. J Clin Endocrinol Metab 70:1574-1580.
114. Bergsland E, Dickler MN. 2004. Maximizing the potential of bevacizumab in cancer treatment. Oncologist 9 Suppl 1:36-42.
115. Woods RJ, Grossman A, Saphier P, Kennedy K, Ur E, Behan D, Potter E, Vale W, Lowry PJ. 1994. Association of human corticotropin-releasing hormone to its binding protein in blood may trigger clearance of the complex. J Clin Endocrinol Metab 78:73-76.
116. Rath T, Baker K, Dumont JA, Peters RT, Jiang H, Qiao SW, Lencer WI, Pierce GF, Blumberg RS. 2015. Fc-fusion proteins and FcRn: structural insights for longer-lasting and more effective therapeutics. Crit Rev Biotechnol 35:235-254.
117. Salfeld JG. 2007. Isotype selection in antibody engineering. Nature biotechnology 25:1369-1372.
118. Petkova SB, Akilesh S, Sproule TJ, Christianson GJ, Al Khabbaz H, Brown AC, Presta LG, Meng YG, Roopenian DC. 2006. Enhanced half-life of genetically engineered human IgG1 antibodies in a humanized FcRn mouse model: potential application in humorally mediated autoimmune disease. Int Immunol 18:1759-1769.
119. Lovejoy DA, Aubry JM, Turnbull A, Sutton S, Potter E, Yehling J, Rivier C, Vale WW. 1998. Ectopic expression of the CRF-binding protein: minor impact on HPA axis regulation but induction of sexually dimorphic weight gain. Journal of neuroendocrinology 10:483-491.
120. Vaughan J, Donaldson C, Bittencourt J, Perrin MH, Lewis K, Sutton S, Chan R, Tumbull AV, Lovejoy D, Rivier C, et al. 1995. Urocortin, a mammalian neuropeptide related to fish urotensin I and to corticotropin-releasing factor. Nature 378:287-292.
121. Kasckow JW, Lupien SJ, Behan DP, Welge J, Hauger RJ. 2001. Circulating human corticotropin-releasing factor-binding protein levels following cortisol infusions. Life sciences 69:133-142.
122. Cole MS, Anasetti C, Tso JY. 1997. Human IgG2 variants of chimeric anti-CD3 are nonmitogenic to T cells. J Immunol 159:3613-3621.
123. Suitters AJ, Foulkes R, Opal SM, Palardy JE, Emtage JS, Rolfe M, Stephens S, Morgan A, Holt AR, Chaplin LC, et al. 1994. Differential effect of isotype on efficacy of anti-tumor necrosis factor alpha chimeric antibodies in experimental septic shock. J Exp Med 179:849-856.
124. Datta-Mannan A, Witcher DR, Tang Y, Watkins J, Wroblewski VJ. 2007. Monoclonal antibody clearance. Impact of modulating the interaction of IgG with the neonatal Fc receptor. The Journal of biological chemistry 282:1709-1717.
125. Davis PM, Abraham R, Xu L, Nadler SG, Suchard SJ. 2007. Abatacept binds to the Fc receptor CD64 but does not mediate complement-dependent cytotoxicity or antibody-dependent cellular cytotoxicity. J Rheumatol 34:2204-2210.
126. Scallon B, Cai A, Radewonuk J, Naso M. 2004. Addition of an extra immunoglobulin domain to two anti-rodent TNF monoclonal antibodies substantially increased their potency. Mol Immunol 41:73-80.
127. Spiess C, Zhai Q, Carter PJ. 2015. Alternative molecular formats and therapeutic applications for bispecific antibodies. Mol Immunol 67:95-106.
128. Pack P, Pluckthun A. 1992. Miniantibodies: use of amphipathic helices to produce functional, flexibly linked dimeric FV fragments with high avidity in Escherichia coli. Biochemistry 31:1579-1584.
129. Chaudhury C, Mehnaz S, Robinson JM, Hayton WL, Pearl DK, Roopenian DC, Anderson CL. 2003. The major histocompatibility complex-related Fc receptor for IgG (FcRn) binds albumin and prolongs its lifespan. J Exp Med 197:315-322.
130. Roopenian DC, Akilesh S. 2007. FcRn: the neonatal Fc receptor comes of age. Nat Rev Immunol 7:715-725.
131. Baker K, Qiao SW, Kuo T, Kobayashi K, Yoshida M, Lencer WI, Blumberg RS. 2009. Immune and non-immune functions of the (not so) neonatal Fc receptor, FcRn. Semin Immunopathol 31:223-236.
132. Meyer S, Nederend M, Jansen JH, Reiding KR, Jacobino SR, Meeldijk J, Bovenschen N, Wuhrer M, Valerius T, Ubink R, Boross P, Rouwendal G, Leusen JH. 2015. Improved in vivo anti-tumor effects of IgA-Her2 antibodies through half-life extension and serum exposure enhancement by FcRn targeting. MAbs:1-12.
133. Andersen JT, Pehrson R, Tolmachev V, Daba MB, Abrahmsen L, Ekblad C. 2011. Extending half-life by indirect targeting of the neonatal Fc receptor (FcRn) using a minimal albumin binding domain. The Journal of biological chemistry 286:5234-5241.
134. Chen X, Lee HF, Zaro JL, Shen WC. 2011. Effects of receptor binding on plasma half-life of bifunctional transferrin fusion proteins. Mol Pharm 8:457-465.
135. Yeh P, Landais D, Lemaitre M, Maury I, Crenne JY, Becquart J, Murry-Brelier A, Boucher F, Montay G, Fleer R, et al. 1992. Design of yeast-secreted albumin derivatives for human therapy: biological and antiviral properties of a serum albumin-CD4 genetic conjugate. Proceedings of the National Academy of Sciences of the United States of America 89:1904-1908.
136. Ding Y, Peng Y, Deng L, Wu Y, Fu Q, Jin J. 2014. The effects of fusion structure on the expression and bioactivity of human brain natriuretic peptide (BNP) albumin fusion proteins. Curr Pharm Biotechnol 15:856-863.
137. Strohl WR. 2015. Fusion Proteins for Half-Life Extension of Biologics as a Strategy to Make Biobetters. BioDrugs 29:215-239.
138. Wu H, Pfarr DS, Johnson S, Brewah YA, Woods RM, Patel NK, White WI, Young JF, Kiener PA. 2007. Development of motavizumab, an ultra-potent antibody for the prevention of respiratory syncytial virus infection in the upper and lower respiratory tract. J Mol Biol 368:652-665.
139. Behan DP, De Souza EB, Lowry PJ, Potter E, Sawchenko P, Vale WW. 1995. Corticotropin releasing factor (CRF) binding protein: a novel regulator of CRF and related peptides. Front Neuroendocrinol 16:362-382.
140. Wang Y, Santos M, Guttman A. 2013. Comparative core fucosylation analysis of some major therapeutic antibody N-glycans by direct infusion ESI-MS and CE-LIF detection. J Sep Sci 36:2862-2867.
141. Lohse S, Meyer S, Meulenbroek LA, Jansen JH, Nederend M, Kretschmer A, Klausz K, Moginger U, Derer S, Rosner T, Kellner C, Schewe D, Sondermann P, Tiwari S, Kolarich D, Peipp M, Leusen JH, Valerius T. 2015. An anti-EGFR IgA that displays improved pharmacokinetics and myeloid effector cell engagement in vivo. Cancer research.
142. Braun J, McHugh N, Singh A, Wajdula JS, Sato R. 2007. Improvement in patient-reported outcomes for patients with ankylosing spondylitis treated with etanercept 50 mg once-weekly and 25 mg twice-weekly. Rheumatology (Oxford) 46:999-1004.
143. Nolan B, Mahlangu J, Perry D, Young G, Liesner R, Konkle B, Rangarajan S, Brown S, Hanabusa H, Pasi KJ, Pabinger I, Jackson S, Cristiano LM, Li X, Pierce GF, Allen G. 2015. Long-term safety and efficacy of recombinant factor VIII Fc fusion protein (rFVIIIFc) in subjects with haemophilia A. Haemophilia.
144. Dumont JA, Liu T, Low SC, Zhang X, Kamphaus G, Sakorafas P, Fraley C, Drager D, Reidy T, McCue J, Franck HW, Merricks EP, Nichols TC, Bitonti AJ, Pierce GF, Jiang H. 2012. Prolonged activity of a recombinant factor VIII-Fc fusion protein in hemophilia A mice and dogs. Blood 119:3024-3030.
145. Powell JS, Josephson NC, Quon D, Ragni MV, Cheng G, Li E, Jiang H, Li L, Dumont JA, Goyal J, Zhang X, Sommer J, McCue J, Barbetti M, Luk A, Pierce GF. 2012. Safety and prolonged activity of recombinant factor VIII Fc fusion protein in hemophilia A patients. Blood 119:3031-3037.
146. Shi X, Yang J, Zhu H, Ye L, Feng M, Li J, Huang H, Tao Q, Ye D, Sun LH, Sun BN, Sun CR, Han G, Liu Y, Yao M, Zhou P, Ju D. 2013. Pharmacokinetics and pharmacodynamics of recombinant human EPO-Fc fusion protein in vivo. PloS one 8:e72673.
147. Way JC, Lauder S, Brunkhorst B, Kong SM, Qi A, Webster G, Campbell I, McKenzie S, Lan Y, Marelli B, Nguyen LA, Degon S, Lo KM, Gillies SD. 2005. Improvement of Fc-erythropoietin structure and pharmacokinetics by modification at a disulfide bond. Protein Eng Des Sel 18:111-118.
148. Bitonti AJ, Dumont JA, Low SC, Peters RT, Kropp KE, Palombella VJ, Stattel JM, Lu Y, Tan CA, Song JJ, Garcia AM, Simister NE, Spiekermann GM, Lencer WI, Blumberg RS. 2004. Pulmonary delivery of an erythropoietin Fc fusion protein in non-human primates through an immunoglobulin transport pathway. Proceedings of the National Academy of Sciences of the United States of America 101:9763-9768.
149. Keeney AJ, Hogg S, Marsden CA. 2001. Alterations in core body temperature, locomotor activity, and corticosterone following acute and repeated social defeat of male NMRI mice. Physiology & behavior 74:177-184.
150. Hammamieh R, Chakraborty N, De Lima TC, Meyerhoff J, Gautam A, Muhie S, D'Arpa P, Lumley L, Carroll E, Jett M. 2012. Murine model of repeated exposures to conspecific trained aggressors simulates features of post-traumatic stress disorder. Behavioural brain research 235:55-66.
151. Osborn BL, Sekut L, Corcoran M, Poortman C, Sturm B, Chen G, Mather D, Lin HL, Parry TJ. 2002. Albutropin: a growth hormone-albumin fusion with improved pharmacokinetics and pharmacodynamics in rats and monkeys. European journal of pharmacology 456:149-158.
152. Silacci M, Baenziger-Tobler N, Lembke W, Zha W, Batey S, Bertschinger J, Grabulovski D. 2014. Linker length matters, fynomer-Fc fusion with an optimized linker displaying picomolar IL-17A inhibition potency. The Journal of biological chemistry 289:14392-14398.
153. Trinh R, Gurbaxani B, Morrison SL, Seyfzadeh M. 2004. Optimization of codon pair use within the (GGGGS)3 linker sequence results in enhanced protein expression. Mol Immunol 40:717-722.
154. Huston JS, Levinson D, Mudgett-Hunter M, Tai MS, Novotny J, Margolies MN, Ridge RJ, Bruccoleri RE, Haber E, Crea R, et al. 1988. Protein engineering of antibody binding sites: recovery of specific activity in an anti-digoxin single-chain Fv analogue produced in Escherichia coli. Proceedings of the National Academy of Sciences of the United States of America 85:5879-5883.
155. Hagemeyer CE, von Zur Muhlen C, von Elverfeldt D, Peter K. 2009. Single-chain antibodies as diagnostic tools and therapeutic agents. Thromb Haemost 101:1012-1019.
156. Chen X, Zaro JL, Shen WC. 2013. Fusion protein linkers: property, design and functionality. Adv Drug Deliv Rev 65:1357-1369.
157. Heim R, Tsien RY. 1996. Engineering green fluorescent protein for improved brightness, longer wavelengths and fluorescence resonance energy transfer. Curr Biol 6:178-182.
158. Lieschke GJ, Rao PK, Gately MK, Mulligan RC. 1997. Bioactive murine and human interleukin-12 fusion proteins which retain antitumor activity in vivo. Nature biotechnology 15:35-40.
159. Saitoh M, Pullen N, Brennan P, Cantrell D, Dennis PB, Thomas G. 2002. Regulation of an activated S6 kinase 1 variant reveals a novel mammalian target of rapamycin phosphorylation site. The Journal of biological chemistry 277:20104-20112.
160. Schmidt A, Echtermeyer F, Alozie A, Brands K, Buddecke E. 2005. Plasmin- and thrombin-accelerated shedding of syndecan-4 ectodomain generates cleavage sites at Lys(114)-Arg(115) and Lys(129)-Val(130) bonds. The Journal of biological chemistry 280:34441-34446.
161. Olivier B, Zethof T, Pattij T, van Boogaert M, van Oorschot R, Leahy C, Oosting R, Bouwknecht A, Veening J, van der Gugten J, Groenink L. 2003. Stress-induced hyperthermia and anxiety: pharmacological validation. European journal of pharmacology 463:117-132.
162. Groenink L, van der Gugten J, Zethof T, van der Heyden J, Olivier B. 1994. Stress-induced hyperthermia in mice: hormonal correlates. Physiology & behavior 56:747-749.
163. Kalin NH, Sherman JE, Takahashi LK. 1988. Antagonism of endogenous CRH systems attenuates stress-induced freezing behavior in rats. Brain research 457:130-135.
164. Kullback S. 1968. Information Theory and Statistics. Dover ,New York.
165. Siegel S. 1956. Nonparametric Statistics for the Behavioral Sciences. McGraw Hill Co ,New York.
166. Dennis MS, Zhang M, Meng YG, Kadkhodayan M, Kirchhofer D, Combs D, Damico LA. 2002. Albumin binding as a general strategy for improving the pharmacokinetics of proteins. The Journal of biological chemistry 277:35035-35043.
167. Andersen JT, Cameron J, Plumridge A, Evans L, Sleep D, Sandlie I. 2013. Single-chain variable fragment albumin fusions bind the neonatal Fc receptor (FcRn) in a species-dependent manner: implications for in vivo half-life evaluation of albumin fusion therapeutics. The Journal of biological chemistry 288:24277-24285.

## Claims

1. An engineered corticotropin-releasing factor (CRF) binding agent, comprising a polypeptide having CRF-specific binding activity under physiological conditions, coupled to one or more half-life-extending moieties, or a pharmaceutically acceptable salt thereof, wherein the polypeptide is a CRF binding protein (CRF-BP), and wherein the one or more half-life-extending moieties comprises an Fc forming portion of a mammalian immunoglobulin heavy chain, or an Fc region of an antibody, optionally an Fc region of a human antibody.

2. The engineered CRF binding agent according to claim 1, wherein the polypeptide is engineered to remove a proteolytic site by substituting one or more amino acid residues in the proteolytic site, or by deleting one or more amino acid residues in the proteolytic site, wherein, optionally, the one or more amino acid residues substituted or deleted are in a proteolytic site having the amino acid sequence of SEQ ID NO: 25 or SEQ ID NO: 26.

3. The engineered CRF binding agent according to claim 1, wherein the polypeptide is selected from the group consisting of hCRF-BP(25-234) consisting of SEQ ID NO: 12, hCRF-BP(25-322) consisting of SEQ ID NO: 13, hCRF-BP(25-322) consisting of SEQ ID NO:63, rCRF-BP(25-234) consisting of SEQ ID NO: 14, and rCRF-BP(25-322) consisting of SEQ ID NO: 15.

4. The engineered CRF binding agent according to claim 1, wherein the polypeptide and the one or more half-life-extending moieties are covalently coupled, optionally via a linker, optionally a peptidyl linker.

5. The engineered CRF binding agent according to claim 1, comprising a hCRF-BP(25-234) polypeptide consisting of SEQ ID NO: 12, a hCRF-BP(25-322) polypeptide consisting of SEQ ID NO: 13, or a hCRF-BP(25-322) polypeptide consisting of SEQ ID NO: 63 coupled via a peptidyl linker to an Fc forming portion of a human immunoglobulin heavy chain.

6. The engineered CRF binding agent according to claim 1, comprising a first element coupled to a second element, wherein the first element comprises a hCRF-BP(25-234) polypeptide consisting of SEQ ID NO: 12, a hCRF-BP(25-322) polypeptide consisting of SEQ ID NO: 13, or a hCRF-BP(25-322) polypeptide consisting of SEQ ID NO: 63, coupled via a peptide linker to an Fc forming portion of a human immunoglobulin heavy chain; and the second element comprises a hCRF-BP(25-234) polypeptide consisting of SEQ ID NO: 12, a hCRF-BP(25-322) polypeptide consisting of SEQ ID NO: 13, or a hCRF-BP(25-322) polypeptide consisting of SEQ ID NO: 63, coupled via a peptide linker to an Fc forming portion of a human immunoglobulin heavy chain.

7. The engineered CRF binding agent according to claim 1, wherein the polypeptide having CRF-specific binding activity encodes at least one site for N-linked glycosylation and/or O-linked glycosylation.

8. A pharmaceutical composition comprising the engineered CRF binding agent according to claim 1, and a pharmaceutically acceptable carrier, excipient, or stabilizer.

9. The composition of claim 8 for use in treating a disease or disorder, comprising a therapeutically effective amount of the engineered CRF binding agent according to claim 1, for administration to a human subject in need thereof.

10. The composition for use according to claim 9, wherein the disease or disorder is **characterized by** HPA axis hyperactivity.

11. The composition for use according to claim 9, wherein the disease or disorder is selected from anxiety, depression, Alzheimer's disease, Parkinson's disease, obesity, metabolic syndrome, type 2 diabetes, osteoporosis, cardiovascular disease, alcohol or drug abuse, inflammatory bowel disease (IBD), and irritable bowel syndrome (IBS).

12. The composition for use according to claim 9, wherein said composition provides a circulating serum concentration of the CRF binding agent in the subject of about 1 µg/mL to about 150 µg/mL.

13. An engineered nucleic acid molecule, comprising an expression construct encoding a fusion protein comprising (i) a CRF-BP polypeptide having CRF-specific binding activity and (ii) an Fc forming portion of a mammalian immunoglobulin heavy chain, wherein optionally, the CRF-BP polypeptide comprises at least one site for N-linked glycosylation and/or O-linked glycosylation.

14. A recombinant host cell comprising the engineered nucleic acid molecule according to claim 13.

## Patentansprüche

1. Technisch hergestelltes Corticotropin-Releasing-Faktor(CRF)-Bindemittel, umfassend ein Polypeptid, das CRF-spezifische Bindungsaktivität unter physiologischen Bedingungen aufweist, gekoppelt an eine oder mehrere Halbwertszeit-verlängernde Einheiten oder ein pharmazeutisch verträgliches Salz davon, wobei das Polypeptid ein CRF-Bindungsprotein (CRF-BP) ist, und wobei die eine oder mehreren Halbwertszeit-verlängernden Einheiten einen Fc-bildenden Abschnitt einer schweren Kette eines Säugerimmunoglobulins oder eine Fc-Region eines Antikörpers, optional eine Fc-Region eines menschlichen Antikörpers, umfassen.

2. Technisch hergestelltes CRF-Bindemittel nach Anspruch 1, wobei das Polypeptid technisch hergestellt wird, um eine proteolytische Stelle durch Substitution eines oder mehrerer Aminosäurereste in der proteolytischen Stelle oder durch Deletieren eines oder mehrerer Aminosäurereste in der proteolytischen Stelle zu entfernen, wobei optional der eine oder die mehreren substituierten oder deletierten Aminosäurereste in einer proteolytischen Stelle vorliegen, die die Aminosäuresequenz von SEQ ID NO: 25 oder SEQ ID NO: 26 aufweist.

3. Technisch hergestelltes CRF-Bindemittel nach Anspruch 1, wobei das Polypeptid aus der Gruppe bestehend aus hCRF-BP(25-234), bestehend aus SEQ ID NO: 12, hCRF-BP(25-322), bestehend aus SEQ ID NO: 13, hCRF-BP(25-322), bestehend aus SEQ ID NO: 63, rCRF-BP(25-234), bestehend aus SEQ ID NO: 14 und rCRF-BP(25-3 22), bestehend aus SEQ ID NO: 15 ausgewählt ist.

4. Technisch hergestelltes CRF-Bindemittel nach Anspruch 1, wobei das Polypeptid und die eine oder mehreren Halbwertszeit-verlängernden Einheiten kovalent gekoppelt sind, optional über einen Linker, optional einen Peptidyllinker.

5. Technisch hergestelltes CRF-Bindemittel nach Anspruch 1, umfassend ein hCRF-BP(25-234)-Polypeptid, bestehend aus SEQ ID NO: 12, ein hCRF-BP(25-322)-Polypeptid, bestehend aus SEQ ID NO: 13, oder ein hCRF-BP(25-322)-Polypeptid, bestehend aus SEQ ID NO: 63, gekoppelt über einen Peptidyllinker an einen Fc-bildenden Abschnitt einer schweren Kette eines menschlichen Immunglobulins.

6. Technisch hergestelltes CRF-Bindemittel nach Anspruch 1, umfassend ein erstes Element, das an ein zweites Element gekoppelt ist, wobei das erste Element ein hCRF-BP(25-234)-Polypeptid, bestehend aus SEQ ID NO: 12, ein hCRF-BP(25-322)-Polypeptid, bestehend aus SEQ ID NO: 13, oder ein hCRF-BP(25-322)-Polypeptid, bestehend aus SEQ ID NO: 63, gekoppelt über einen Peptidlinker an einen Fc-bildenden Abschnitt einer schweren Kette eines menschlichen Immunglobulins, umfasst; und das zweite Element ein hCRF-BP(25-234)-Polypeptid, bestehend aus SEQ ID NO: 12, ein hCRF-BP(25-322)-Polypeptid, bestehend aus SEQ ID NO: 13, oder ein hCRF-BP(25-322)-Polypeptid, bestehend aus SEQ ID NO: 63, gekoppelt über einen Peptidlinker an einen Fc-bildenden Teil einer schweren Kette eines menschlichen Immunglobulins umfasst.

7. Technisch hergestelltes CRF-Bindemittel nach Anspruch 1, wobei das Polypeptid, das CRF-spezifische Bindungsaktivität aufweist, für mindestens eine Stelle für N-verknüpfte Glykosylierung und/oder O-verknüpfte Glykosylierung codiert.

8. Pharmazeutische Zusammensetzung, umfassend das technisch hergestellte CRF-Bindemittel nach Anspruch 1 und einen pharmazeutisch annehmbaren Träger, Hilfsstoff oder Stabilisator.

9. Zusammensetzung nach Anspruch 8 zur Verwendung beim Behandeln einer Krankheit oder Störung, umfassend eine therapeutisch wirksame Menge des technisch hergestellten CRF-Bindemittels nach Anspruch 1, zur Verabreichung an ein menschliches Subjekt, das dessen bedarf.

10. Zusammensetzung zur Verwendung nach Anspruch 9, wobei die Krankheit oder Störung durch Hyperaktivität der HPA-Achse gekennzeichnet ist.

11. Zusammensetzung zur Verwendung nach Anspruch 9, wobei die Krankheit oder Störung aus Angst, Depression, Alzheimer-Krankheit, Parkinson-Krankheit, Fettleibigkeit, metabolischem Syndrom, Diabetes Typ 2, Osteoporose, kardiovaskulärer Krankheit, Alkohol- oder Drogenmissbrauch, entzündlicher Darmerkrankung (IBD) und Reizdarmsyndrom (IBS) ausgewählt ist.

12. Zusammensetzung zur Verwendung nach Anspruch 9, wobei die Zusammensetzung eine zirkulierende Serumkonzentration des CRF-Bindungsmittels bei dem Subjekt von etwa 1 µg/ml bis etwa 150 µg/ml bereitstellt.

13. Technisch hergestelltes Nukleinsäuremolekül, umfassend ein Expressionskonstrukt, das für ein Fusionsprotein codiert, umfassend (i) ein CRF-BP-Polypeptid, das CRF-spezifische Bindungsaktivität aufweist, und (ii) einen Fc-bildenden Abschnitt einer schweren Kette eines Säugerimmunoglobulins, wobei optional das CRF-BP-Polypeptid mindestens eine Stelle für N-verknüpfte Glykosylierung und/oder O-verknüpfte Glykosylierung umfasst.

14. Rekombinante Wirtszelle, umfassend das technisch hergestellte Nukleinsäuremolekül nach Anspruch 13.

## Revendications

1. Agent de liaison modifié du facteur de libération de corticotropine (CRF), comprenant un polypeptide présentant une activité de liaison spécifique du CRF dans des conditions physiologiques, couplé à un ou plusieurs fragments prolongeant la demi-vie, ou un sel pharmaceutiquement acceptable de ceux-ci, dans lequel le polypeptide est une protéine de liaison du CRF (CRF-BP), et dans lequel le ou les plusieurs fragments prolongeant la demi-vie comprennent une partie formant Fc d'une chaîne lourde d'immunoglobuline de mammifère, ou une région Fc d'un anticorps, éventuellement une région Fc d'un anticorps humain.

2. Agent de liaison modifié du CRF selon la revendication 1, dans lequel le polypeptide est modifié pour éliminer un site protéolytique par substitution d'un ou de plusieurs résidus d'acides aminés dans le site protéolytique, ou par suppression d'un ou plusieurs résidus d'acides aminés dans le site protéolytique, dans lequel, éventuellement le ou les plusieurs résidus d'acides aminés substitués ou supprimés sont dans un site protéolytique présentant la séquence d'acides aminés SEQ ID NO: 25 ou SEQ ID NO: 26.

3. Agent de liaison modifié du CRF selon la revendication 1, dans lequel le polypeptide est choisi dans le groupe constitué de hCRF-BP(25-234) constitué de SEQ ID NO: 12, hCRF-BP(25-322) constitué de SEQ ID NO: 13, hCRF-BP(25-322) constitué de SEQ ID NO: 63, rCRF-BP(25-234) constitué de SEQ ID NO: 14, et rCRF-BP(25-322) constitué de SEQ ID NO: 15.

4. Agent de liaison modifié du CRF selon la revendication 1, dans lequel le polypeptide et le ou les plusieurs fragments prolongeant la demi-vie sont couplés de manière covalente, éventuellement par l'intermédiaire d'un lieur, éventuellement d'un lieur peptidyle.

5. Agent de liaison modifié du CRF selon la revendication 1, comprenant un polypeptide hCRF-BP(25-234) constitué de SEQ ID NO: 12, un polypeptide hCRF-BP(25-322) constitué de SEQ ID NO: 13, ou un polypeptide hCRF-BP(25-322) constitué de SEQ ID NO: 63 couplé par l'intermédiaire d'un lieur peptidyle à une partie formant Fc d'une chaîne lourde d'immunoglobuline humaine.

6. Agent de liaison modifié du CRF selon la revendication 1, comprenant un premier élément couplé à un second élément, dans lequel le premier élément comprend un polypeptide hCRF-BP(25-234) constitué de SEQ ID NO: 12, un polypeptide hCRF-BP(25-322) constitué de SEQ ID NO: 13, ou un polypeptide hCRF-BP(25-322) constitué de SEQ ID NO: 63, couplé par l'intermédiaire d'un lieur peptidique à une partie formant Fc d'une chaîne lourde d'immunoglobuline humaine ; et le second élément comprend un polypeptide hCRF-BP(25-234) constitué de SEQ ID NO: 12, un polypeptide hCRF-BP(25-322) constitué de SEQ ID NO: 13, ou un polypeptide hCRF-BP(25-322) constitué de SEQ ID NO: 63, couplé par l'intermédiaire d'un lieur peptidique à une partie formant Fc d'une chaîne lourde d'immunoglobuline humaine.

7. Agent de liaison modifié du CRF selon la revendication 1, dans lequel le polypeptide présentant une activité de liaison spécifique du CRF code pour au moins un site de glycosylation à liaison N et/ou de glycosylation à liaison O.

8. Composition pharmaceutique comprenant l'agent de liaison modifié du CRF selon la revendication 1, et un support, un excipient ou un stabilisant pharmaceutiquement acceptable.

9. Composition selon la revendication 8 destinée à être utilisée dans le traitement d'une maladie ou d'un trouble, comprenant une quantité thérapeutiquement efficace de l'agent de liaison modifié du CRF selon la revendication 1 à administrer à un sujet humain en ayant besoin.

10. Composition destinée à être utilisée selon la revendication 9, dans laquelle la maladie ou le trouble est caractérisé(e) par une hyperactivité de l'axe HPA.

11. Composition destinée à être utilisée selon la revendication 9, dans laquelle la maladie ou le trouble est choisi(e) parmi l'anxiété, la dépression, la maladie d'Alzheimer, la maladie de Parkinson, l'obésité, le syndrome métabolique, le diabète de type 2, l'ostéoporose, les maladies cardiovasculaires, la dépendance à l'alcool ou aux drogues, les maladies intestinales inflammatoires (IBD), et le syndrome du côlon irritable (IBS).

12. Composition destinée à être utilisée selon la revendication 9, dans laquelle ladite composition permet d'obtenir une concentration sérique circulante de l'agent de liaison du CRF chez le sujet d'environ 1 µg/mL à environ 150 µg/mL.

13. Molécule d'acide nucléique modifiée, comprenant un produit de recombinaison d'expression codant pour une protéine de fusion comprenant (i) un polypeptide CRF-BP présentant une activité de liaison spécifique du CRF et (ii) une partie formant Fc d'une chaîne lourde d'immunoglobuline de mammifère, dans laquelle éventuellement le polypeptide CRF-BP comprend au moins un site de glycosylation à liaison N et/ou de glycosylation à liaison O.

14. Cellule hôte recombinante comprenant la molécule d'acide nucléique modifiée selon la revendication 13.
